# FASCICULE DE BREVET EUROPEEN

(11) **EP 1 802 773 B1**
(45) Date de publication et mention de la délivrance du brevet: **26.08.2009**
(21) Numéro de dépôt: 05810890.3
(22) Date de dépôt: 17.10.2005
(51) Int. Cl.: C12Q 1/68

(54) **PROCÉDÉ POUR LE DIAGNOSTIC D'UNE INTOLÉRANCE À L'ASPIRINE**
VERFAHREN ZUR DIAGNOSTIZIERUNG VON ASPIRINUNVERTRÄGLICHKEIT
METHOD FOR DIAGNOSING ASPIRIN INTOLERANCE

(30) Priorité: 19.10.2004 FR 0452366
(43) Date de publication de la demande: 04.07.2007
(73) Titulaire: bioMérieux S.A., 69280 Marcy l'Etoile (FR)
(72) Inventeur: PACHOT, Alexandre, F-01400 SULIGNAT (FR); PACHECO, Yves, F-69390 CHARLY (FR); DEVOUASSOUX, Gilles, F-69500 BRON (FR); VAN GANSE, Eric, F-69350 LA MULATIERE (FR)
(74) Mandataire: Denjean, Frédérique
(86) Numéro de dépôt international: PCT/FR2005/050858
(87) Numéro de publication internationale: WO 2006/042995

(56) Documents cités:
- EP-A- 1 394 274
- WO-A-02/077013
- WO-A-03/072827
- US-B1- 6 506 607
- "Affimetrix GeneChip Human Genome U133 Array Set HG-U133A" GEO, 11 mars 2002 (2002-03-11), XP002328996 cité dans la demande
- PUJOLS L ET AL: "Dynamics of COX-2 in nasal mucosa and nasal polyps from aspirin-tolerant and aspirin-intolerant patients with asthma" JOURNAL OF ALLERGY AND CLINICAL IMMUNOLOGY, MOSBY - YEARLY BOOK, INC, US, vol. 114, no. 4, octobre 2004 (2004-10), pages 814-819, XP004631426 ISSN: 0091-6749
- YING SUN ET AL: "Mechanisms of aspirin-sensitive asthma" ALLERGOLOGY INTERNATIONAL, vol. 53, no. 2, juin 2004 (2004-06), pages 111-119, XP002328997 ISSN: 1323-8930
- COWBURN ANDREW S ET AL: "Overexpression of leukotriene C4 synthase in bronchial biopsies from patients with aspirin-intolerant asthma" JOURNAL OF CLINICAL INVESTIGATION, vol. 101, no. 4, 15 février 1998 (1998-02-15), pages 834-846, XP002328998 ISSN: 0021-9738

## Description

La présente invention concerne l'asthme et plus particulièrement un procédé de diagnostic in vitro d'une intolérance à l'aspirine.

L'asthme est une maladie respiratoire caractérisé principalement par une inflammation des bronches et des épisodes de spasmes au cours desquels les bronches se rétrécissent considérablement. Ces crises se résorbent parfois spontanément alors que, dans d'autres cas, elles doivent être traitées. Il existe différentes pathologies de l'asthme. Si 90 % des asthmatiques développe des crises d'asthme d'origine allergique, 8 à 10 % des asthmatiques développent un asthme du à des mécanismes purement biochimiques, en présentant une intolérance à l'aspirine (AIA) ou à un autre anti-inflammatoire non stéroïdien (AINS). L'aspirine (ou acide salicylique), comme les AINS, sont des inhibiteurs des cyclo-oxygénases. L'intolérance à l'aspirine implique deux voies métaboliques : celle de la synthèse des leukotriènes par l'intermédiaire de la leukotriène C 4 synthase et la 5 lipoxygenase (LIPOX 5) et la voie des prostaglandines par l'intermédiaire des cyclooxygénase (COX). Les patients AIA seraient des asthmatiques qui produisent trop de leukotriènes, élément particulièrement bronchostricteur. C'est en particulier le cas chez des personnes ayant une polypose nasale (présence de polype dans le nez), associée à un asthme grave et à une intolérance à l'aspirine. Cette association est appelée syndrome de Fernand Widal. Les AINS sont formellement contre-indiqués dans ce cas. Ces patients réagissant différemment aux médicaments destinés habituellement aux asthmatiques, il est essentiel de pouvoir diagnostiquer le plus tôt possible si l'asthme que développe un patient est d'origine immunologique ou purement biochimique, et ceci afin de lui proposer un traitement adapté.

A l'heure actuelle, le profil d'un patient asthmatique repose essentiellement sur une évaluation clinique standardisée, une exploration respiratoire fonctionnelle, la réalisation d'un bilan allergologique et éventuellement sur un bilan radiologique sinusal et pulmonaire. L'identification de marqueurs géniques de cette pathologie constituerait donc une avancée importante pour aider le clinicien à stratifier les patients afin qu'il leur propose des traitements thérapeutiques adaptés. L'identification de marqueurs géniques de cette pathologie constituerait donc une avancée importante pour aider le clinicien à stratifier les patients afin qu'il leur propose des traitements thérapeutiques adaptés. Toutefois, aucun test génétique n'est actuellement reconnu d'une façon consensuel par les cliniciens.

La présente invention se propose de résoudre l'ensemble des inconvénients de l'état de la technique en présentant un outil de diagnostic pour déterminer si un patient asthmatique est tolérant ou intolérant à l'aspirine. D'une manière surprenante, les inventeurs ont mis en évidence que l'analyse de l'expression de gènes cibles sélectionnés parmi 25 gènes tel que présenté dans le tableau 1 ci après, est très pertinente pour discriminer des patients asthmatiques intolérants à l'aspirine des autres patients.

**Tableau 1- liste des 25 gènes cibles selon l'invention**

| **SEQ ID N°** | **Nom de gène** | **N° GENBANK** |
|---|---|---|
| **1** | Malstrom syndrome 1 | NM_015120 |
| **2** | annexin A3 = lipocortin 3 | NM_005139 |
| **3** | ATP-binding cassette, sub-family A (ABC1), member 1 | NM_005502 |
| **4** | B-cell CLL/lymphoma 6 (zinc finger protein 51) | NM_001706 (variant 1) |
| **5** | carcinoembryonic antigen-relatedcell adhésion molecule 1 (biliary glycoprotein) | NM_001712 |
| **6** | cell division cycle 42 (GTP binding protein, 25kDa) | NM_001791 |
| **7** | Charot-Leyden crystal protein = Galectin 10 | NM_001828 |
| **8** | claudin 18 | NM_016369 |
| **9** | cofactor required for Sp1transcriptional activation, subunit 2, 150kDa | NM_004229 |
| **10** | Type (calcium dépendent, carbohydrate-recognition domain)lectin, superfamily member 14 (macrophage-derived) | NM_182906 (variant 1) |
| **11** | glutathione S-transferase M4 | NM_000850 (variant 1) |
| **12** | homeodomain interacting protein kinase 3 | NM_005951 |
| **13** | Homo sapiens cDNA clone IMAGE:5218466 | BC030533 |
| **14** | hypothetical protein FLJ35827 | NM_153265 |
| **15** | KIAA0329 gene product | XM_375105 |
| **16** | major histocompatibility complex, class **II,** DP bêta 1 | NM_002121 |
| **17** | MAX dimerization protein 4 | NM_006454 |
| **18** | Metallothionein 1 H | NM_005734 |
| **19** | N-acetylglucosamine-1-phosphodiesteralpha-N-acetylglucosaminidase | NM_016256 |
| **20** | phospholipase A2, group V | NM_000929 |
| **21** | protein tyrosine phosphatase,non-receptor type 22 (lymphoid) | NM_015967 (variant 1) |
| **22** | RNA binding motif protein 25 | XM_027330 |
| **23** | serine (or cysteine) proteinase inhibitor, clade B (ovalbumin), member 2 | NM_002575 |
| **24** | TATA box binding protein (TBP) -associated factor,RNA polymerase **I,** A, 48kDa | NM_139352 (variant 1) |
| **25** | UDP-N-acetyl-alpha-D-galactosamine:polypeptideN-acetylgalactosaminyltransferase 3 (GalNAc-T3) | NM_004482 |

Il existe parfois plusieurs variants pour un même gène cible. Dans la présente invention, tous les variants sont pertinents. A ce titre, il convient notamment de noter qu'il existe deux variants pour le gène cible de SEQ ID N°4, seul le premier variant est présenté dans le tableau ci dessus, mais le deuxième variant,ayant pour numéro d'accession Genbank NM_138931 est tout aussi pertinent au sens de la présente invention. D'une manière comparable, il existe un deuxième variant pour le gène cible de SEQ ID N°10 ayant pour numero d'accession Genbank NM_006344 ; deux autres variants pour le gène cible de SEQ ID N°11 ayant pour numéro d'accession Genbank NM_147148 (variant 2) et NM_147149 (variant 3), un deuxième variant pour le gène cible de SEQ ID N°21 ayant pour numero d'accession Genbank NM_012411 (variant 2); et un deuxième variant pour le gène cible de SEQ ID N°24 ayant pour numero d'accession Genbank Nom_00568 (variant 2).

A cet effet, la présente invention concerne un procédé pour le diagnostic d'une intolérance à l'aspirine à partir d'un échantillon biologique d'un patient caractérisé en ce qu'il comprend les étapes suivantes :
a. on extrait du matériel biologique de l'échanlillon biologique,
b. on met en contact le matériel biologique avec au moins un réactif spécifique choisi parmi les réactifs spécifiques des gènes cibles présentant une séquence nucléique ayant l'une quelconque des SEQ ID N° 1 à 25;
c. on détermine l'expression d'au moins un des dits gènes cibles.
   Au sens de la présente invention, on entend par échantillon biologique, tout échantillon prélevé chez un patient, et susceptible de contenir un matériel biologique tel que défini ci après. Cet échantillon biologique peut être notamment un échantillon de sang, de sérum, de salive, tissu, de cellules circulantes du patient. On dispose de cet échantillon biologique par tout type de prélèvement connu de l'homme du métier. Selon un mode préféré de réalisation de l'invention, l'échantillon biologique prélevé chez le patient est un échantillon sanguin.

Lors de l'étape a) du procédé selon l'invention, on extrait le matériel biologique de l'échantillon biologique par tous les protocoles d'extraction et de purification d'acides nucléiques bien connus de l'homme du métier. Au sens de la présente invention, on entend par matériel biologique, tout matériel permettant de détecter l'expression d'un gène cible. Le matériel biologique peut comprendre notamment des protéines, ou des acides nucléiques tels que notamment les acides desoxyribonucléiques (ADN) ou les acides ribonucléiques (ARN). L'acide nucléique peut être notamment un ARN (acide ribonucléique). Selon un mode préféré de réalisation de l'invention, le matériel biologique extrait lors de l'étape a) comprend des acides nucléiques, préférentiellement, des ARN, et encore plus préférentiellement des ARN totaux. Les ARN totaux comprennent les ARN de transfert, les ARN messagers (ARNm), tel que les ARNm transcrits du gène cible, mais également transcrit de tout autre gène et les ARN ribosomaux. Ce matériel biologique comprend du matériel spécifique d'un gène cible, tel que notamment les ARNm transcrits du gène cible ou les protéines issues de ces ARNm mais peut comprendre également du matériel non spécifique d'un gène cible, tel que notamment les ARNm transcrits d'un gène autre que le gène cible, les ARNt, les ARNr issus d'autres gènes que le gène cible.

A titre indicatif, l'extraction d'acides nucléique peut être réalisée par :
- une étape de lyse des cellules présentes dans l'échantillon biologique, afin de libérer les acides nucléiques contenus dans les cellules du patient. A titre d'exemple, on peut utiliser les méthodes de lyse telles que décrites dans les demandes de brevet:
   o WO 00/05338 sur la lyse mixte magnétique et mécanique,
   o WO 99/53304 sur la lyse électrique,
   o WO 99/15321 sur la lyse mécanique.
      L'homme du métier pourra utiliser d'autres méthodes de lyse bien connues, telles que les chocs thermiques ou osmotiques ou les lyses chimiques par des agents chaotropiques tels que les sels de guanidium (US 5,234,809).
- une étape de purification, permettant la séparation entre les acides nucléiques et les autres constituants cellulaires relargués dans l'étape de lyse. Cette étape permet généralement de concentrer les acides nucléiques, et peut être adaptée à la purification d'ADN ou d'ARN. A titre d'exemple, on peut utiliser des particules magnétiques éventuellement revêtues d'oligonucléotides, par adsorption ou covalence (voir à ce sujet les brevets US 4,672,040 et US 5,750,338), et ainsi purifier les acides nucléiques qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet: WO-A-97/45202 et WO-A-99/35500. Un autre exemple intéressant de méthode de purification des acides nucléiques est l'utilisation de silice soit sous forme de colonne, soit sous forme de particules inertes (Boom R. et al., J. Clin. Microbiol., 1990, n°28(3), p. 495-503) ou magnétiques (Merck: MagPrep^{®} Silica, Promega: MagneSil^{™} Paramagnetic particles). D'autres méthodes très répandues reposent sur des résines échangeuses d'ions en colonne ou en format particulaire paramagnétique (Whatman: DEAE-Magarose) (Levison PR et al., J. Chromatography, 1998, p. 337-344). Une autre méthode très pertinente mais non exclusive pour l'invention est celle de l'adsorption sur support d'oxyde métallique (société Xtrana: matrice Xtra-Bind^{™}).
   Lorsque l'on souhaite extraire spécifiquement l'ADN d'un échantillon biologique, on peut notamment réaliser une extraction par du phénol, du chloroforme et de l'alcool pour éliminer les protéines et précipiter l'ADN avec de l'éthanol 100%. L'ADN peut alors être culoté par centrifugation, lavé et remis en solution .
   Lorsque l'on souhaite extraire spécifiquement les ARN d'un échantillon biologique, on peut notamment réaliser une extraction par du phénol, du chloroforme et de l'alcool pour éliminer les protéines et précipiter les ARN avec de l'éthanol 100%. Les ARN peuvent alors être culotés par centrifugation, lavés et remis en solution.

Lors de l'étape b), et au sens de la présente invention, on entend par réactif spécifique, un réactif qui, lorsqu'il est mis en contact avec du matériel biologique tel que défini précédemment, se lie avec le matériel spécifique dudit gène cible. A titre indicatif, lorsque le réactif spécifique et le matériel biologique sont d'origine nucléique, la mise en contact du réactif spécifique et du matériel biologique permet l'hybridation du réactif spécifique avec le matériel spécifique du gène cible. Par hybridation, on entend le processus au cours duquel, dans des conditions appropriées, deux fragments nucléotidiques se lient avec des liaisons hydrogènes stables et spécifiques pour former un complexe double brin. Ces liaisons hydrogènes se forment entre les bases complémentaires Adénine (A) et thymine (T) (ou uracile (U)) (on parle de liaison AT) ou entre les bases complémentaires Guanine (G) et cytosine (C) (on parle de liaison G-C). L'hybridation de deux fragments nucléotidiques peut être totale (on parle alors de fragments nucléotidiques ou de séquences complémentaires), c'est à dire que le complexe double brin obtenu lors de cette hybridation comprend uniquement des liaisons AT et des liaisons C-G. Cette hybridation peut être partielle (on parle alors de fragments nucléotidiques ou de séquences suffisamment complémentaires), c'est à dire que le complexe double brin obtenu comprend des liaisons A-T et des liaisons C-G permettant de former le complexe double brin, mais également des bases non liées à une base complémentaire. L'hybridation entre deux fragments nucléotidiques dépend des conditions opératoires qui sont utilisées, et notamment de la stringence. La stringence est définie notamment en fonction de la composition en bases des deux fragments nucléotidiques, ainsi que par le degré de mésappariement entre deux fragments nucléotidiques. La stringence peut également être fonction des paramètres de la réaction, tels que la concentration et le type d'espèces ioniques présentes dans la solution d'hybridation, la nature et la concentration d'agents dénaturants et/ou la température d'hybridation. Toutes ces données sont bien connues et les conditions appropriées peuvent être déterminées par l'homme du métier. En général, selon la longueur des fragments nucléotidiques que l'on souhaite hybrider, la température d'hybridation est comprise entre environ 20 et 70°C, en particulier entre 35 et 65°C dans une solution saline à une concentration d'environ 0,5 à 1 M. Une séquence, ou fragment nucléotidique, ou oligonucléotide, ou polynucléotide, est un enchaînement de motifs nucléotidiques assemblés entre eux par des liaisons ester phosphoriques, caractérisé par la séquence informationnelle des acides nucléiques naturels, susceptibles de s'hybrider à un fragment nucléotidique, l'enchaînement pouvant contenir des monomères de structures différentes et être obtenu à partir d'une molécule d'acide nucléique naturelle et/ou par recombinaison génétique et/ou par synthèse chimique. Un motif est dérivé d'un monomère qui peut être un nucléotide naturel d'acide nucléique dont les éléments constitutifs sont un sucre, un groupement phosphate et une base azotée ; dans l'ADN le sucre est le désoxy-2-ribose, dans l'ARN le sucre est le ribose ; selon qu'il s'agisse de l'ADN ou l'ARN, la base azotée est choisie parmi l'adénine, la guanine, l'uracile, la cytosine, la thymine ; ou bien le monomère est un nucléotide modifié dans l'un au moins des trois éléments constitutifs ; à titre d'exemple, la modification peut intervenir soit au niveau des bases, avec des bases modifiées telles que l'inosine, la méthyl-5désoxycytidine, la désoxyuridine, la diméthy!amino-5désoxyuridine, la diamino-2,6-purine, la bromo-5désoxyuridine ou toute autre base modifiée capable d'hybridation, soit au niveau du sucre, par exemple le remplacement d'au moins un désoxyribose par un polyamide (P.E. Nielsen et al, Science, 254, 1497-1500 (1991), soit encore au niveau du groupement phosphate, par exemple son remplacement par des esters notamment choisis parmi les diphosphates, alkyl- et aryl-phosphonates et phosphorothioates.

Selon un mode particulier de réalisation de l'invention, le réactif spécifique comprend au moins une amorce d'amplification. Au sens de la présente invention, on entend par amorce d'amplification, un fragment nucléotidique comprenant de 5 à 100 motifs nucléiques, préférentiellement de 15 à 30 motifs nucléiques permettant l'initiation d'une polymérisation enzymatique, telle que notamment une réaction d'amplification enzymatique. Par réaction d'amplification enzymatique, on entend un processus générant de multiples copies d'un fragment nucléotidique par l'action d'au moins une enzyme. De telles réactions d'amplification sont bien connues de l'homme du métier et on peut citer notamment les techniques suivantes :
- PCR (Polymerase Chain Reaction), telle que décrite dans les brevets US 4,683,195, US 4,683,202 et US 4,800,159,
- LCR (Ligase Chain Reaction), exposée par exemple dans la demande de brevet EP 0 201 184,
- RCR (Repair Chain Reaction), décrite dans la demande de brevet WO 90/01069,
- 3SR (Self Sustained Sequence Replication) avec la demande de brevet WO 90/06995,
- NASBA (Nucleic Acid Sequence-Based Amplification) avec la demande de brevet WO 91/02818, et
- TMA (Transcription Mediated Amplification) avec le brevet US 5,399,491.

Lorsque l'amplification enzymatique est une PCR, le réactif spécifique comprend au moins 2 amorces d'amplification, spécifiques d'un gène cible, permettant l'amplification du matériel spécifique du gène cible. Le matériel spécifique du gène cible comprend alors préférentiellement un ADN complémentaire obtenu par transcription inverse d'ARN messager issu du gène cible (on parle alors d'ADNc spécifique du gène cible) ou un ARN complémentaire obtenu par transcription des ADNc spécifiques d'un gène cible (on parle alors d'ARNc spécifique du gène cible). Lorsque l'amplification enzymatique est une PCR réalisée après une réaction de transcription reverse, on parle de RT-PCR.

Selon un autre mode préféré de réalisation de l'invention, le réactif spécifique de l'étape b) comprend au moins une sonde d'hybridation.

Par sonde d'hybridation, on entend un fragment nucléotidique comprenant au moins 5 motifs nucléotidiques, tel que de 5 à 100 motifs nucléiques, notamment de 10 à 35 motifs nucléiques, possédant une spécificité d'hybridation dans des conditions déterminées pour former un complexe d'hybridation avec le matériel spécifique d'un gène cible. Dans la présente invention, le matériel spécifique du gène cible peut être une séquence nucléotidique comprise dans un ARN messager issu du gène cible (on parle alors d'ARNm spécifique du gène cible), une séquence nucléotidique comprise dans un ADN complémentaire obtenu par transcription inverse dudit ARN messager (on parle alors d'ADNc spécifique du gène cible), ou encore une séquence nucléotidique comprise dans un ARN complémentaire obtenu par transcription dudit ADNc tel que décrit précédemment (on parlera alors d'ARNc spécifique du gène cible). La sonde d'hybridation peut comprendre un marqueur permettant sa détection. Par détection on entend soit une détection directe par une méthode physique, soit une détection indirecte par une méthode de détection à l'aide d'un marqueur. De nombreuses méthodes de détection existent pour la détection des acides nucléiques. [Voir par exemple Kricka et al., Clinical Chemistry, 1999, n° 45(4), p.453-458 ou Keller G.H. et al., DNA Probes, 2nd Ed., Stockton Press, 1993, sections 5 et 6, p.173-249]. Par marqueur, on entend un traceur capable d'engendrer un signal que l'on peut détecter. Une liste non limitative de ces traceurs comprend les enzymes qui produisent un signal détectable par exemple par colorimétrie, fluorescence ou luminescence, comme la peroxydase de raifort, la phosphatase alcaline, la beta galactosidase, la glucose-6-phosphate déshydrogénase; les chromophores comme les composés fluorescents, luminescents ou colorants ; les groupements à densité électronique détectables par microscopie électronique ou par leurs propriétés électriques comme la conductivité, par les méthodes d'ampérométrie ou de voltamétrie, ou par des mesures d'impédance ; les groupements détectables par des méthodes optiques comme la diffraction, la résonance plasmon de surface, la variation d'angle de contact ou par des méthodes physiques comme la spectroscopie de force atomique, l'effet tunnel, etc. ; les molécules radioactives comme ³²P, ³⁵S ou ¹²⁵I.

Au sens de la présente invention, la sonde d'hybridation peut être une sonde dite de détection. Dans ce cas, la sonde dite de détection est marquée au moyen d'un marqueur tel que défini précédemment. La sonde de détection peut être notamment une sonde de détection « molecular beacons » telle que décrite par Tyagi & Kramer (Nature biotech, 1996, 14 :303-308). Ces "molecular beacons" deviennent fluorescentes lors de l'hybridation. Elles possèdent une structure de type tige-boucle et contiennent un fluorophore et un groupe "quencher". La fixation de la séquence de boucle spécifique avec sa séquence complémentaire d'acide nucléique cible provoque un déroulement de la tige et l'émission d'un signal fluorescent lors de l'excitation à la longueur d'onde qui convient.

Pour la détection de la réaction d'hybridation, on peut utiliser des séquences cibles marquées, directement (notamment par l'incorporation d'un marqueur au sein de la séquence cible) ou indirectement (notamment par l'utilisation d'une sonde de détection telle que définie précédemment) la séquence cible. On peut notamment réaliser avant l'étape d'hybridation une étape de marquage et/ou de clivage de la séquence cible, par exemple en utilisant un désoxyribonucléotide triphosphate marqué lors de la réaction d'amplification enzymatique. Le clivage peut être réalisé notamment par l'action de l'imidazole et de chlorure de manganèse. La séquence cible peut aussi être marqué après l'étape d'amplification, par exemple en hybridant une sonde de détection selon la technique d'hybridation sandwich décrite dans le document WO 91/19812. Un autre mode particulier préférentiel de marquage d'acides nucléiques est décrit dans la demande FR2 780 059.

Selon un mode préféré de réalisation de l'invention, la sonde de détection comprend un flurophore et un quencher. Selon un mode encore plus préféré de réalisation de l'invention, la sonde d'hybridation comprend un flurophore FAM (6-carboxy-fluoresccin) ou ROX (6-carboxy-X-rhodamine) en son extrémité 5' et un quencher (Dabsyl) en son extrémité 3'.

La sonde d'hybridation peut être également une sonde dite de capture. Dans ce cas, la sonde dite de capture est immobilisée ou immobilisable sur un support solide par tout moyen approprié, c'est-à-dire directement ou indirectement, par exemple par covalence ou adsorption. Comme support solide, on peut utiliser des matériaux de synthèse ou des matériaux naturels, éventuellement modifiés chimiquement, notamment les polysaccharides tels que les matériaux à base de cellulose, par exemple du papier, des dérivés de cellulose tels que l'acétate de cellulose et la nitrocellulose ou le dextrane, des polymères, des copolymères, notamment à base de monomères du type styrène, des fibres naturelles telles que le coton, et des fibres synthétiques telles que le nylon ; des matériaux minéraux tels que la silice, le quartz, des verres, des céramiques ; des latex ; des particules magnétiques ; des dérivés métalliques, des gels etc. Le support solide peut être sous la forme d'une plaque de microtitration, d'une membrane comme décrit dans la demande WO-A-94/12670, d'une particule. On peut également immobiliser sur le support plusieurs sondes de capture différentes, chacune étant spécifique d'un gène cible. En particulier, on peut utiliser comme support une biopuce sur laquelle peuvent être immobilisées un grand nombre de sondes. Par biopuce, on entend un support solide de dimension réduite où est fixée une multitude de sondes de capture à des positions prédéterminées. Le concept de biopuce, ou puce à ADN, date du début des années 90. Il repose sur une technologie pluridisciplinaire intégrant la micro-électronique, la chimie des acides nucléiques, l'analyse d'images et l'informatique. Le principe de fonctionnement repose sur un fondement de la biologie nnoléculaire: le phénomène d'hybridation, c'est-à-dire l'appariement par complémentarité des bases de deux séquences d'ADN et/ou d'ARN. La méthode des biopuces repose sur l'emploi de sondes de capture fixées sur un support solide sur lesquelles on fait agir un échantillon de fragments nucléotidiques cibles marqués directement ou indirectement avec des fluorochromes. Les sondes de capture sont positionnées de manière spécifique sur le support ou puce et chaque hybridation donne une information particulière, en relation avec le fragment nucléotidique cible. Les informations obtenues sont cumulatives, et permettent par exemple de quantifier le niveau d'expression d'un gène ou de plusieurs gènes cibles. Pour analyser l'expression d'un gène cible, on peut alors réaliser un support comprenant une multitude de sondes, qui correspondent à tout ou partie du gène cible, qui est transcrit en ARNm. Au sens de la présente invention, on entend par support de basse densité, un support comprenant moins de 50 sondes. Au sens de la présente invention, on entend par support de moyenne densité, un support comprenant de 50 sondes à 10 000 sondes. Au sens de la présente invention, on entend par support de haute densité, un support comprenant plus de 10 000 sondes.

On hybride alors par exemple les ADNc ou les ARNc spécifiques d'un gène cible que l'on souhaite analyser sur des sondes de capture spécifique. Après hybridation, le support ou puce est lavé(e), et les complexes ADNc ou ARNc marquées / sondes de capture sont révélés par un ligand de forte affinité lié par exemple à un marqueur de type fluorochrome. La fluorescence est lue par exemple par un scanner et l'analyse de la fluorescence est traitée par informatique. On peut citer à titre indicatif, les puces à ADN mises au point par la société Affymetrix ("Accessing Genetic Information with High-Density DNA arrays", M. Chee et al., Science, 1996, 274, 610-614. "Light-generated oligonucleotide arrays for rapide DNA sequence analysis", A. Caviani Pease et al., Proc. Natl. Acad. Sci. USA, 1994, 91, 5022-5026), pour les diagnostics moléculaires. Dans cette technologie, les sondes de capture sont généralement de tailles réduites, autour de 25 nucléotides. D'autres exemples de biopuces sont donnés dans les publications de G. Ramsay, Nature Biotechnology, 1998, n° 16, p. 40-44 ; F. Ginot, Human Mutation, 1997, n° 10, p. 1-10; J. Cheng et al, Molecular diagnosis, 1996, n°1(3), p.183-200 ; T. Livache et al, Nucleic Acids Research, 1994, n° 22(15), p. 2915-2921 ; J. Cheng et al, Nature Biotechnology, 1998, n° 16, p. 541-546 ou dans les brevets US-A-4,981,783, US-A-5,700,637, US-A-5,445,934, US-A-5,744,305 et US-A-5,807,522. La caractéristique principale du support solide doit être de conserver les caractéristiques d'hybridation des sondes de capture sur les fragments nucléotidiques cibles tout en générant un bruit de fond minimum pour la méthode de détection.

Pour l'immobilisation des sondes sur le support, on distingue trois grands types de fabrication.

Il y a, tout d'abord, une première technique qui consiste en un dépôt de sondes présynthétisées. La fixation des sondes se fait par transfert direct, au moyen de micropipettes, de micro-pointes ou par un dispositif de type jet d'encre. Cette technique permet la fixation de sondes de taille allant de quelques bases (5 à 10) jusqu'à des tailles relativement importantes de 60 bases (impression) à quelques centaines de bases (micro-déposition) :

L'impression est une adaptation du procédé utilisé par les imprimantes à jet d'encre.
Elle repose sur la propulsion de très petites sphères de fluide (volume <1 nl) et à un rythme pouvant atteindre 4000 gouttes/secondes. L'impression, n'implique aucun contact entre le système libérant le fluide et la surface sur laquelle il est déposé.

La micro-déposition consiste à fixer des sondes longues de quelques dizaines à plusieurs centaines de bases à la surface d'une lampe de verre. Ces sondes sont généralement extraites de bases de données et se présentent sous forme de produits amplifiés et purifiés. Cette technique permet de réaliser des puces dénommées microarrays portant environ dix mille spots, dit zones de reconnaissance, d'ADN sur une surface d'un peu moins de 4 cm2. Il ne faut toutefois pas oublier l'emploi de membranes de Nylon, dites « macroarrays », qui portent des produits amplifiés, généralement par PCR, avec un diamètre de 0,5 à 1 mm et dont la densité maximale est de 25 spots/cm2. Cette technique très flexible est utilisée par de nombreux laboratoires. Dans la présente invention, cette dernière technique est considérée comme faisant partie des biopuces. On peut toutefois déposer en fond de plaque de microtitration un certain volume d'échantillon dans chaque puits, comme c'est le cas dans les demandes de brevet WO-A-00/71750 et FR 00/14896, ou déposer au fond d'une même boîte de Pétri un certain nombre de gouttes séparées les unes des autres, selon une autre demande de brevet FR00/14691.

La deuxième technique de fixation des sondes sur le support ou puce est appelée la synthèse in situ. Cette technique aboutit à l'élaboration de sondes courtes directement à la surface de la puce. Elle repose sur la synthèse d'oligonucléotides in situ (voir notamment les demandes de brevet WO 89/10977 et WO 90/03382), et est fondée sur le procédé des synthétiseurs d'oligonucléotides. Elle consiste à déplacer une chambre de réactions, où se déroule la réaction d'élongation d'oligonucléotides, le long de la surface de verre.

Enfin, la troisième technique est appelée la photolithographie, qui est un procédé à l'origine des biopuces développées par Affymetrix. Il s'agit également d'une synthèse in situ.

La photolithographie est dérivée des techniques des microprocesseurs. La surface de la puce est modifiée par la fixation de groupements chimiques photolabiles pouvant être activés par la lumière. Une fois illuminés, ces groupes sont susceptibles de réagir avec l'extrémité 3' d'un oligonucléotide. En protégeant cette surface par des masques de formes définies, on peut illuminer et donc activer sélectivement des zones de la puce où l'on souhaite fixer l'un ou l'autre des quatre nucléotides. L'utilisation successive de masques différents permet d'alterner des cycles de protection/réaction et donc de réaliser les sondes d'oligonucléotides sur des spots d'environ quelques dizaines de micromètre carré (µm2). Cette résolution permet de créer jusqu'à plusieurs centaines de milliers de spots sur une surface de quelques centimètres carré (cm2). La photolithographie présente des avantages : massivement parallèle, elle permet de créer une puce de N-mères en seulement 4 x N cycles. Toutes ces techniques sont utilisables avec la présente invention. Selon un mode préféré de réalisation de l'invention, le au moins un réactif spécifique de l'étape b) définie précédemment comprend au moins une sonde d'hybridation, qui est préférentiellement immobilisée sur un support. Ce support est préférentiellement un support de basse , haute ou moyenne densité tel que définie précédemment.

Ces étapes d'hybridation sur support comprenant une multitude de sondes peuvent être précédées d'une étape de réaction d'amplification enzymatique, telle que définie précédemment, pour augmenter la quantité de matériel génétique cible.

Lors de l'étape c) la détermination de l'expression d'un gène cible peut être réalisée par tous les protocoles connus de l'homme du métier.

D'une manière générale, l'expression d'un gène cible peut être analysée par la détection des ARNm (ARN messagers) qui sont transcrits du gène cible à un instant donné ou par la détection des protéines issues de ces ARNm.

L'invention concerne préférentiellement la détermination de l'expression d'un gène cible par la détection des ARNm issus de ce gène cible selon tous les protocoles bien connus de l'homme du métier. Selon un mode particulier de réalisation de l'invention, on détermine simultanément l'expression de plusieurs gènes cibles, par la détection de plusieurs ARNm différents, chaque ARNm étant issus d'un gène cible.

Lorsque le réactif spécifique comprend au moins une amorce d'amplification, on peut, lors de l'étape c) du procédé selon l'invention, déterminer l'expression d'un gène cible de la manière suivante:
1) après avoir extrait comme matériel biologique, les ARN totaux (comprenant les ARN de transfert (ARNt), les ARN ribosomaux (ARNr) et les ARN messagers (ARNm)) d'un échantillon biologique tel que présenté précédemment, on réalise une étape de transcription reverse afin d'obtenir les ADN complémentaires (ou ADNc) desdits ARN. A titre indicatif, cette réaction de transcription reverse peut être réalisée à l'aide d'une enzyme reverse transcriptase qui permet d'obtenir, à partir d'un fragment d'ARN, un fragment d'ADN complémentaire. On peut utiliser notamment l'enzyme reverse transcriptase provenant de l'AMV (Avian Myoblastosis Virus) ou de MMLV (Moloney Murine Leukaemia Virus). Lorsque l'on souhaite plus particulièrement obtenir uniquement les ADNc des ARNm, on réalise cette étape de transcription reverse en présence de fragments nucléotidiques comprenant uniquement des bases thymine (polyT), qui s'hybrident par complémentarité sur la séquence polyA des ARNm afin de former un complexe polyT-polyA qui sert alors de point de départ à la réaction de transcription reverse réalisée par l'enzyme reverse transcriptase. On obtient alors des ADNc complémentaires des ARNm issus d'un gène cible (ADNc spécifique du gène cible) et des ADNc complémentaires des ARNm issus d'autres gènes que le gène cible (ADNc non spécifique du gène cible).
2) on met en contact la ou les amorces d'amplification spécifiques d'un gène cible avec les ADNc spécifique du gène cible et les ADNc non spécifique du gène cible. La ou les amorces d'amplification spécifiques d'un gène cible s'hybrident avec les ADNc spécifique du gène cible et on amplifie spécifiquement une région prédéterminée, de longueur connue, des ADNc provenant des ARNm issus du gène cible. Les ADNc non spécifiques du gène cible ne sont pas amplifiés, alors qu'on obtient alors une grande quantité d'ADNc spécifiques du gène cible. Au sens de la présente invention, on parle indifféremment d' « ADNc spécifiques du gène cible » ou d' « ADNc provenant des ARNm issus du gène cible ». Cette étape peut être réalisée notamment par une réaction d'amplification de type PCR ou par toute autre technique d'amplification telle que définie précédemment. En PCR, on peut également amplifier simultanément plusieurs ADNc différents, chacun étant spécifique de différents gènes cibles par l'utilisation de plusieurs couples d'amorces d'amplification différents, chacune étant spécifique d'un gène cible: on parle alors d'amplification en multiplex.
3) on détermine l'expression du gène cible en détectant et quantifiant les ADNc spécifiques du gène cible obtenus lors de l'étape 2) ci dessus. Cette détection peut être réalisée après migration par électrophorèse des ADNc spécifiques du gène cible en fonction de leur taille. Le gel et le milieu de migration peuvent comprendre du bromure d'éthydium afin de permettre la détection directe des ADNc spécifiques du gène cible lorsque le gel est placé, après un temps de migration donné, sur une table lumineuse à rayons UV (ultra violet) par l'émission d'un signal lumineux. Ce signal est d'autant plus lumineux que la quantité des ADNc spécifiques du gène cible est importante. Ces techniques d'électrophorèse sont bien connues de l'homme du métier. Les ADNc spécifiques du gène cible peuvent également être détectés et quantifiés par l'utilisation d'une gamme de quantification obtenue par une réaction d'amplification conduite jusqu'à saturation. Afin de tenir compte de la variabilité d'efficacité enzymatique qui peut être observée lors des différentes étapes (transcription reverse, PCR...), on peut normaliser l'expression d'un gène cible de différents groupes de patients, par la détermination simultanée de l'expression d'un gène dit de ménage, dont l'expression est similaire chez les différents groupes de patients. En réalisant un rapport entre l'expression du gène cible et l'expression du gène de ménage, c'est à dire en réalisant un rapport entre la quantité d'ADNc spécifiques du gène cible, et la quantité d'ADNc spécifiques du gène de ménage, on corrige ainsi toute variabilité entre les différentes expérimentations. L'homme du métier pourra se référer notamment aux publications suivantes : Bustin SA, J Mol Endocrinol , 2002, 29 : 23-39 ; Giulietti A Methods, 2001, 25 : 386-401.

Lorsque le réactif spécifique comprend au moins une sonde d'hybridation, on peut déterminer l'expression d'un gène cible de la manière suivante:
1) après avoir extrait, comme matériel biologique, les ARN totaux d'un échantillon biologique tel que présenté précédemment, on réalise une étape de transcription reverse, telle que décrite précédemment afin des ADNc complémentaires des ARNm issus d'un gène cible (ADNc spécifique du gène cible) et des ADNc complémentaires des ARNm issus d'autres gènes que le gène cible (ADNc non spécifique du gène cible).
2) on met en contact tous les ADNc avec un support, sur lequel sont immobilisées des sondes de capture spécifiques du gène cible dont on souhaite analyser l'expression, afin de réaliser une réaction d'hybridation entre les ADNc spécifiques du gène cible et les sondes de capture, les ADNc non spécifiques du gène cible ne s'hybridant pas sur les sondes de capture. La réaction d'hybridation peut être réalisée sur un support solide qui inclut tous les matériaux tels qu'indiqués précédemment. Selon un mode préféré de réalisation, la sonde d'hybridation est immobilisée sur un support. Préférentiellement, le support est un support de basse , haute ou moyenne densité tel que définie précédemment. La réaction d'hybridation peut être précédée d'une étape d'amplification enzymatique des ADNc spécifiques du gène cible telle que décrite précédemment pour obtenir une grande quantité d'ADNc spécifiques du gène cible et augmenter la probabilité qu'un ADNc spécifiques d'un gène cible s'hybride sur une sonde de capture spécifique du gène cible. La réaction d'hybridation peut également être précédée d'une étape de marquage et/ou de clivage des ADNc spécifiques du gène cible telle que décrite précédemment, par exemple en utilisant un désoxyribonucléotide triphosphate marqué pour la réaction d'amplification. Le clivage peut être réalisé notamment par l'action de l'imidazole et de chlorure de manganèse. L'ADNc spécifique du gène cible peut aussi être marqué après l'étape d'amplification, par exemple en hybridant une sonde marquée selon la technique d'hybridation sandwich décrite dans le document WO-A-91/19812. D'autres modes particuliers préférentiels de marquage et/ou clivage d'acides nucléiques sont décrit dans les demandes WO 99/65926, WO 01/44507, WO 01/44506, WO 02/090584, WO 02/090319.
3) on réalise ensuite une étape de détection de la réaction d'hybridation. La détection peut être réalisée par la mise en contact du support sur lequel sont hybridés les sondes de capture spécifiques du gène cible avec les ADNc spécifiques du gène cible avec une sonde dite de détection, marquée par un marqueur, et on détecte le signal émis par le marqueur. Lorsque l'ADNc spécifique du gène cible a été préalablement marqué par un marqueur, on détecte directement le signal émis par le marqueur.

Lorsque le au moins un réactif spécifique mis en contact l'étape b) du procédé selon l'invention comprend au moins une sonde d'hybridation, on peut également déterminer l'expression d'un gène cible de la manière suivante:
1) après avoir extrait, comme matériel biologique, les ARN totaux d'un échantillon biologique tel que présenté précédemment, on réalise une étape de transcription reverse, telle que décrite précédemment afin d'obtenir les ADNc des ARNm du matériel biologique. On réalise ensuite la polymérisation de l'ARN complémentaire du ADNc par l'utilisation d'une enzyme polymerase de type T7 polymérase qui fonctionne sous la dépendance d'un promoteur et qui permet d'obtenir, à partir d'une matrice d'ADN, l'ARN complémentaire. On obtient alors les ARNc des ADNc des ARNm spécifiques du gène cible (on parle alors d'ARNc spécifique du gène cible) et les ARNc des ADNc des ARNm non spécifiques du gène cible.
2) on met en contact tous les ARNc avec un support, sur lequel sont immobilisées des sondes de capture spécifiques du gène cible dont on souhaite analyser l'expression, afin de réaliser une réaction d'hybridation entre les ARNc spécifiques du gène cible et les sondes de capture, les ARNc non spécifiques du gène cible ne s'hy bridant pas sur les sondes de capture. Lorsque l'on souhaite analyser simultanément l'expression de plusieurs gènes cibles, on peut immobiliser sur le support plusieurs sondes de capture différentes, chacune étant spécifique d'un gène cible. La réaction d'hybridation peut également être précédée d'une étape de marquage et/ou de clivage des ARNc spécifiques du gène cible telles que décrites précédemment.
3) on réalise ensuite une étape de détection de la réaction d'hybridation. La détection peut être réalisée par la mise en contact du support sur lequel sont hybridées les sondes de capture spécifiques du gène cible avec l'ARNc spécifique du gène cible avec une sonde dite de détection, marquée par un marqueur, et on détecte le signal émis par le marqueur. Lorsque l'ARNc spécifiques du gène cible a été préalablement marqué par un marqueur, on détecte directement le signal émis par le marqueur. L'utilisation d'ARNc est particulièrement avantageux lorsqu'on utilise un support de type biopuce sur lequel est hybridé un grand nombre de sondes.

Selon un mode particulier de réalisation de l'invention, les étapes B et C sont effectuées en même temps. Ce mode préféré peut être notamment mise en oeuvre par « NASBA en temps réel » qui regroupe en une étape unique la technique d'amplification NASBA et la détection en temps réel qui fait appel à des "molecular beacons". La réaction NASBA intervient dans le tube, produisant de l'ARN simple brin avec lequel les "molecular beacons" spécifiques peuvent s'hybrider simultanément pour donner un signal fluorescent. La formation des nouvelles molécules d'ARN est mesurée en temps réel par contrôle continu du signal dans un lecteur fluorescent. Contrairement à une amplification par RT-PCR, l'amplification en NASBA peut se faire en présence d'ADN dans l'échantillon. Il n'est donc pas nécessaire de vérifier que l'ADN a bien été complètement éliminé lors de l'extraction des ARN.

L'analyse de l'expression d'un gène cible choisi parmi l'une quelconque des SEQ ID N°1 à 25 permet alors de disposer d'un outil pour le diagnostic d'une intolérance à l'asthme. On peut par exemple analyser l'expression d'un gène cible chez un patient dont on ne connaît pas sa réaction à l'aspirine, et comparer avec des valeurs d'expression moyenne connues du gène cible de patients asthmatiques intolérants à l'aspirine (AIA) et des valeurs d'expression moyenne connues du gène cible de patients asthmatiques tolérants à l'aspirine (ATA). Ceci permet de déterminer si le patient est intolérant à l'aspirine, afin de lui proposer un traitement adapté.

Plus particulièrement, les inventeurs ont mis en évidence que l'analyse simultanée de l'expression d'un panel de 25 gènes tels que définis précédemment, ou de 17 gènes cibles présentant une séquence nucléique ayant l'une quelconque des SEQ ID N° 1 à 6; 8 ; 11 à 12 ; 15 à 19 ; 22 à 23 et 25 ou de 19 gènes présentant une séquence nucléique ayant l'une quelconque des SEQ ID N° 1 à 10 ; 13 à 15 ; 17 à 21 ; 24 était très pertinente pour discriminer des patients AIA et des patients ATA.

A ce titre, l'invention concerne également un procédé tel que défini précédemment caractérisé en ce que lors de l'étape b) on met en contact le matériel biologique avec au moins 25 réactifs spécifiques choisis parmi les réactifs spécifiques des gènes cibles présentant une séquence nucléique ayant l'une quelconque des SEQ ID N° 1 à 25 et on détermine, lors de l'étape c, l'expression d'au moins 25 desdits gènes cibles.

A ce titre, l'invention concerne également un procédé tel que défini précédemment caractérisé en ce que lors de l'étape b) on met en contact le matériel biologique avec au moins 17 réactifs spécifiques choisis parmi les réactifs spécifiques des gènes cibles présentant une séquence nucléique ayant l'une quelconque des SEQ ID N° 1 à 6 ; 8 ; 11 à 12 ; 15 à 19 ; 22 à 23 et 25 et on détermine, lors de l'étape c, l'expression d'au moins 17 desdits gènes cibles.

L'invention concerne également un procédé tel que défini précédemment caractérisé en ce que lors de l'étape b) on met en contact le matériel biologique avec au moins 19 réactifs spécifiques choisis parmi les réactifs spécifiques des gènes cibles présentant une séquence nucléique ayant l'une quelconque des SEQ ID N° 1 à 10 ; 13 à 15 ; 17 à 21 ; 24 et on détermine, lors de l'étape c, l'expression d'au moins 19 desdits gènes cibles.

Les inventeurs ont également démontré que ce panel de gènes pouvait être réduit à un panel très restreint, limité à 11 gènes. A ce titre, l'invention concerne un procédé tel que défini précédemment caractérisé en ce que lors de l'étape b) on met en contact le matériel biologique avec au moins 11 réactifs spécifiques choisis parmi les réactifs spécifiques des gènes cibles présentant une séquence nucléique ayant l'une quelconque des SEQ ID N° 1 à 6 ; 8 ; 15 ; 17 à 19 et on détermine, lors de l'étape c, l'expression d'au moins 10 desdits gènes cibles.

L'utilisation de panel de gènes restreint est particulièrement adapté pour obtenir un outil de pronostic. En effet, l'analyse de l'expression d'une dizaine de gènes ne nécessite pas la fabrication à façon de supports de haute densité, et peut être mise en oeuvre directement par des techniques de PCR ou de NASBA, ou support basse densité, ce qui présente un atout économique important et une mise en oeuvre simplifiée.

L'invention concerne également un support comprenant au moins une sonde d'hybridation spécifique d'au moins un gène cible présentant une séquence nucléique ayant l'une quelconque des SEQ ID N° 1 à 25.

L'invention concerne également une support comprenant au moins 25 sondes d'hybridation choisis parmi les sondes spécifiques des gènes cibles présentant une séquence nucléique ayant l'une quelconque des SEQ ID N° 1 à 25.

L'invention concerne également une support comprenant au moins 17 sondes d'hybridation choisis parmi les sondes spécifiques des gènes cibles présentant une séquence nucléique ayant l'une quelconque des SEQ ID N° 1 à 6 ; 8 ; 11 à 12 ; 15 à 19 ; 22 à 23 et 25.

L'invention concerne également un support comprenant au moins 19 sondes d'hybridation choisis parmi les sondes spécifiques des gènes cibles présentant une séquence nucléique ayant l'une quelconque des SEQ ID N° 1 à 10 ; 13 à 15 ; 17 à 21 ; 24.

L'invention concerne également un support comprenant au moins 11 sondes d'hybridation choisis parmi les sondes spécifiques des gènes cibles présentant une séquence nucléique ayant l'une quelconque des SEQ ID N° 1 à 6 ; 8 ; 15 ; 17 à 19.

L'invention concerne également l'utilisation d'un support tel que défini précédemment pour le diagnostic d'une intolérance à l'aspirine.

L'invention concerne également un kit de diagnostic d'une intolérance à l'aspirine comprenant un support tel que défini précédemment.

Les figures ci-jointes sont données à titre d'exemple explicatif et n'ont aucun caractère limitatif. Elles permettront de mieux comprendre l'invention.

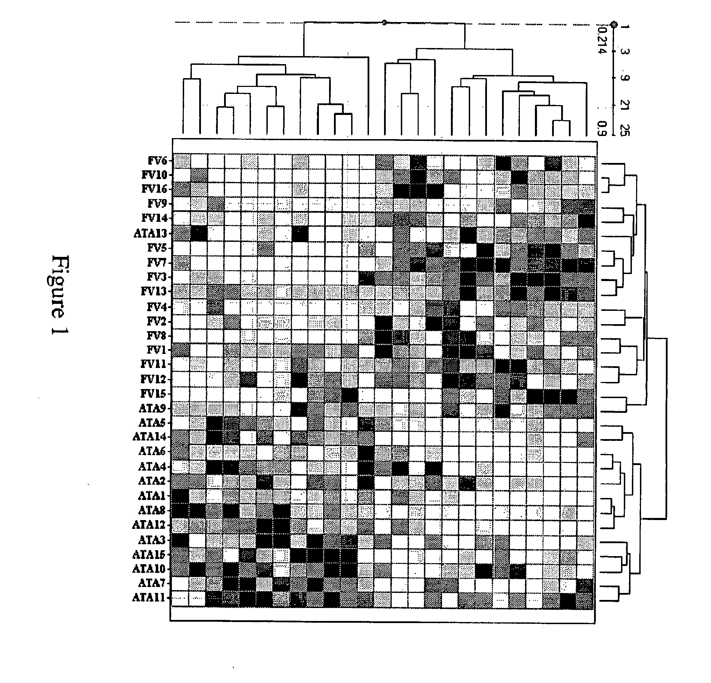

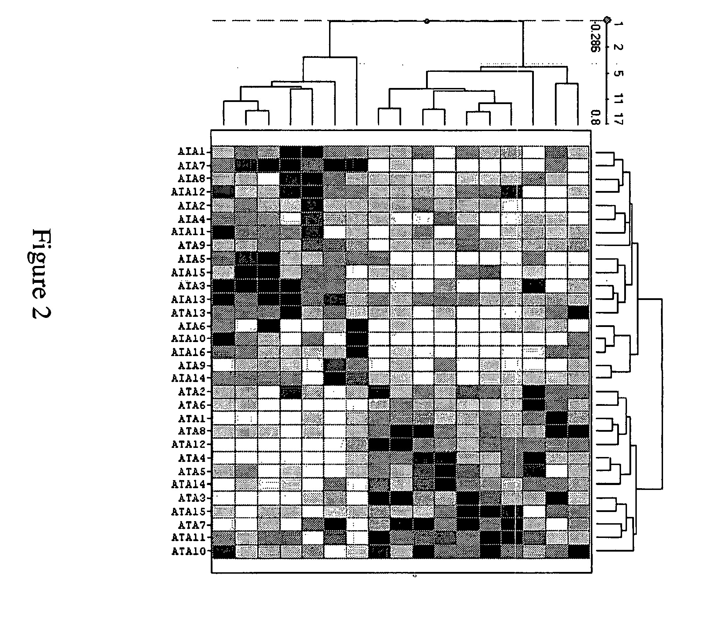

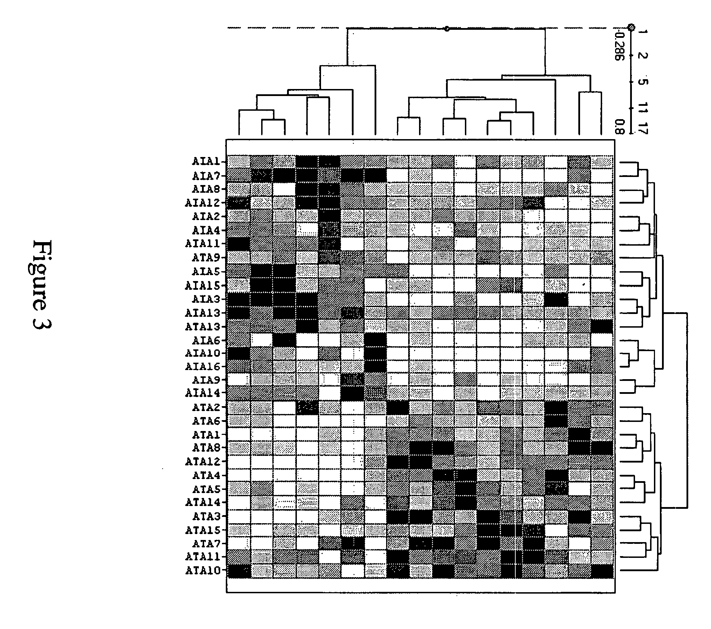

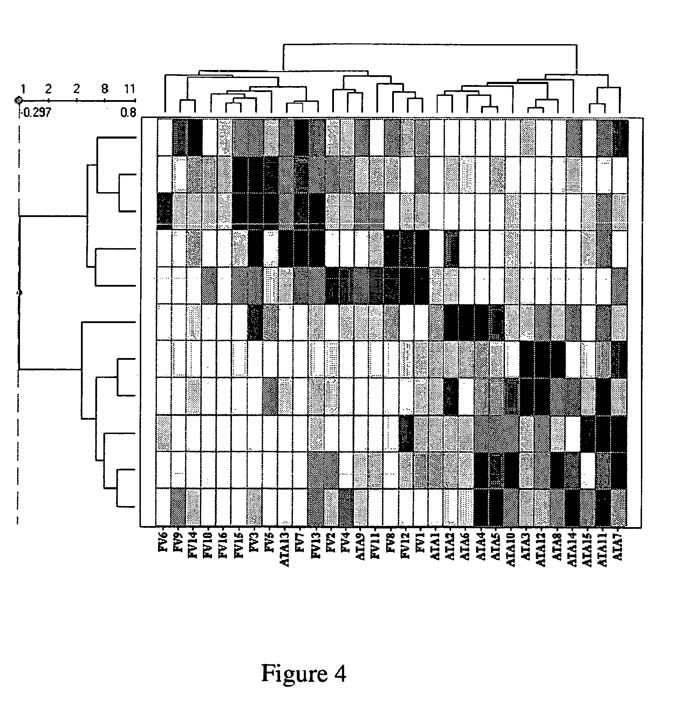

Les figures 1 à 4 représentent une l'analyse de clustering hiérarchique de 31 échantillons de sang obtenu à partir de 15 patients AIA (appelé également FV) et 15 patients ATA en utilisant l'expression de 25 (figure 1), 19 (figure 2) ou 17 (figure 3) gènes identifiés par l'analyse algorithmique. La figure 4 correspond au clustering hiérarchique obtenu avec les 11 gènes communs entre la liste de 19 et de 17 gènes. La fonction de clustering hiérarchique du logiciel Spofire organise les patients AIA et ATA en colonnes, et les gènes en lignes de manière obtenir en position adjacente les patients ou les gènes présentant des profils d'expression comparables. Le coefficient de corrélation de Pearson a été utilisé comme indice de similarité pour les gènes et les patients. Par la suite, la méthode de clustering des moyennes non pondérées UPGMA d'une part, et, la valeur moyenne de tous les échantillons d'autre part, ont respectivement permis d'organiser les patients et les gènes. Les résultats correspondent au niveau de fluorescence Affymetrix normalisé par le logiciel « Affy ». Afin de tenir compte des différences constitutives d'expression entres les gènes, les niveaux d'expression de chaque gène ont été normalisés en calculant une variable centré réduite. Le blanc représente les faibles niveaux d'expression, le gris les nivaux intermédiaires et le noir le niveaux forts. La hauteur des branches du dendodograrnme indique l'indice de similarité entres les profils d'expression.

Les exemples suivants sont donnés à titre illustratif et n'ont aucun caractère limitatif. Ils permettront de mieux comprendre l'invention.

### Exemple 1 : Recherche d'un profil d'expression pour le diagnostic de l'asthme avec intolérance à l'aspirine

### Caractéristiques des échantillons biologiques :

31 échantillons de sang, obtenus auprès du service de Pneumologie du Centre Hospitalier Lyon Sud, France, ont été utilisés dans cette étude. Les patients ont été inclus de manière consécutive lors d'une consultation ou d'une hospitalisation motivée par la prise en charge de leur asthme quel que soit le niveau de stabilisation de leur asthme (asthme stable ou en période d'exacerbation). La cohorte était constituée de 15 patients asthmatiques intolérants à l'aspirine (AIA) et 15 patients asthmatiques tolérants à l'aspirine (ATA). Le diagnostic d'intolérance à l'aspirine a reposé sur la notion d'une anamnèse positive et l'examen clinique (notamment, recherche de polypes nasaux évocateurs d'un syndrome de Femand Widal). Aucun test de provocation n'a été réalisé dans le cadre de l'étude. Tous les patients présentaient un asthme persistant léger, modéré ou sévère (stades 2 à 4 de la classification GINA) associé ou non associé à une intolérance à l'aspirine.
La moyenne d'âge des patients (AIA : 48.3 ± 16.7 ; ATA :48.3 ± 14.8) ainsi que le sex ratio homme/femme (AIA : 1.14 ; ATA : 0.88) étaient similaires dans les deux groupes. De plus, 42.9% des patients AIA et 38.5% des patients ATA présentaient des signes d'atopie. Le compte des polynuclaires éosinophiles au moment de l'analyse de génomique fonctionnelle étaient similaire entres les deux groupes (AIA : 3.5 ± 4.2 10⁸/I; ATA : 2.5 ± 2.4 10⁸/I). Enfin, 40% des patients AIA et 60% des patients ATA présentaient une corticothérapie continue.

### Extraction du matériel biologique (ARN totaux) de l'échantillon biologique :

Les prélèvements ont été collectés directement dans des tubes PAXGene^{™} Blood RNA (PreAnalytix, Frankin Lakes, USA). Après l'étape de prélèvement de l'échantillon sanguin et afin d'obtenir une lyse totale des cellules, les tubes ont été laissés à température ambiante pendant 4 h puis conservés à -20°C jusqu'à l'extraction du matériel biologique. Plus précisément, dans ce protocole, les ARN totaux ont été extraits à l'aide des kits PAXGene Blood RNA® (PreAnalytix) en respectant les recommandations du fabriquant. Brièvement, les tubes ont été centrifugés (10 min, 3000 g) afin d'obtenir un culot d'acides nucléiques. Ce culot a été lavé et repris dans un tampon contenant de la protéinase K nécessaire à la digestion des protéines (10 min à 55°C). Une nouvelle centrifugation (5 min 19 000g) a été effectuée pour éliminer les débris cellulaires et de l'éthanol a été ajouté afin d'optimiser les conditions de fixation des acides nucléiques. Les ARN totaux ont été spécifiquement fixés sur les colonnes PAXgene RNA spin column et, avant l'élution de ceux-ci, une digestion de l'ADN contaminant a été effectuée à l'aide du RNAse free DNAse set (Qiagen Ltd, Crawley, UK). La qualité des ARN totaux a été analysée par le bio analyseur AGILENT 2100 (Agilent Technologies, Waldbronn, Germany). Les ARN totaux comprennent les ARN de transfert, les ARN messagers (ARNm) et les ARN ribosomaux.

*Synthèse d'ADNc, obtention des ARNc, marquage des ARNc et quantification:* Afin d'analyser l'expression des gènes cibles selon l'invention, les ADN complémentaires (ADNc) des ARNm contenus dans les ARN totaux tels que purifiés ci dessus, ont été obtenus à partir de 5 µg d'ARN totaux par l'utilisalion de 400 unités de l'enzyme de transcription reverse SuperScriptII (Invitrogen) et 100 pmol d'amorce poly-T contenant le promoteur de la T7 promotor (T7-oligo(dT)24-primer, Proligo, Paris, France). Les ADNc ainsi obtenus ont ensuite été extraits avec du phénol/chloroforme, et précipités par de l'acétate d'ammonium et de l'éthanol, et remis en solution dans 24 µl d'eau DEPC. Un volume de 20 µl de cette solution purifiée d'ADNc a fait l'objet ensuite d'une transcription *in vitro* par l'utilisation d'une ARN polymérase T7 qui reconnaît spécifiquement le promoteur de la T7 polymérase tel que mentionné ci dessus. Cette transcription permet d'obtenir l'ARNc de l'ADNc. Cette transcription a été réalisée par l'utilisation d'un kit Bioarray High Yield RNA Transcript Labeling Kit (Enzo Diagnostics, Farmingdale, NY), qui permet non seulement d'obtenir l'ARNc mais également l'incorporation de bases cytidine et uridine biotinylées lors de la synthèse de l'ARNc.

Les ARNc purifiés ont ensuite été quantifiés par spectrophotométrie, et la solution d'ARNc a été ajustée à une concentration de 1 µg/µl d'ARNc. L'étape de clivage de ces ARNc a ensuite été réalisée à 94°C pendant 35 min, par l'utilisation d'un tampon de fragmentation (40 mM de Tris acétate, pH 8,1, 100 mM d'acétate de potassium, 30 mM d'acétate de magnesium) afin de provoquer l'hydrolyse des ARNc et obtenir des fragments de 35 à 200 bp. Le succès d'une telle fragmentation a été vérifié par une électrophorèse sur gel d'agarose 1,5%.

### Mise en évidence d'un Profil d'expression différentiel entre les patients AIA et ATA :

L'expression d'environ 14 500 gènes a été analysée et comparée entre les patients AIA et ATA. Pour cela, 20 µg d'ARNc fragmentés issus de chaque échantillon ont été ajoutés à un tampon d'hybridation (Affymetrix) et 200 µl de cette solution ont été mis en contact pendant 16 h à 45°C sur une puce d'expression (Human Genome U133A GeneChip® (Affymetrix), qui comporte 22 283 groupes de sondes représentant environs 14500 gènes selon le protocole d'Affymetrix tel que décrit sur le site internet d'Affymetrix. Afin d'enregistrer les meilleures performances d'hybridation et de lavage, des ARN qualifiés de « contrôle » biotinylés (bioB, bioC, bioD et cre) et des oligonucléotides (oligo B2) ont également été inclus dans le tampon d'hybridation. Après l'étape d'hybridation, la solution d'ARNc biotinylée et hybridée sur la puce, a été révélée par l'utilisation d'une solution de streptavidine-phycoerythrine et le signal a été amplifié par l'utilisation d'anticorps anti-streptavidine. L'hybridation a été réalisée dans une étuve d'hybridation « GeneChip Hybridisation oven» (Affymetrix), et le protocole Euk GE-WS2V4 du protocole d'Affymetrix a été suivi. Les étapes de lavage et de révélation ont été réalisées sur une station «Fluidics Station 450» (Affymetrix). Chaque puce U133A a ensuite été analysée sur un scanner Agilent G2500A GeneArray Scanner à une résolution de 3 microns afin de repérer les zones hybridées sur la puce. Ce scanner permet la détection du signal émis par les molécules fluorescentes après excitation par un laser argon en utilisant la technique du microscope à épifluorescence. On obtient ainsi pour chaque position, un signal proportionnel à la quantité de ARNc fixés. Le signal a ensuite été analysé par le logiciel Microarray Suite 5.0 software (MAS5.0, Affymetrix).
Afin de prévenir les variations obtenues par l'utilisation de différentes puces, il a été réalisé une approche de normalisation globale utilisant le logiciel MAS5.0 (Affymetrix), qui, grâce à un algorithme statistique, permet de définir si un gène était exprimé ou non. Afin de pouvoir comparer les puces entres elles, les données brutes (fichier « .CELL ») ont été traitées par une étape de normalisation quantile à l'aide de l'implémentation « Affy » du logiciel « R » (Gautier, L. et al., Bioinformatics (2004), p.307-315).Chaque gène représenté sur la puce

U 133A était couvert par 11 couples de sondes de 25 oligonucléotides. Par couple de sondes, on entend une première sonde qui s'hybridait parfaitement (on parle alors de sondes PM ou perfect match) avec un des ARNc issus d'un gène cible, et une deuxième sonde, identique à la première sonde à l'exception d'un mésappariement (on parle alors de sonde MM ou mismatched) au centre de la sonde. Chaque sonde MM servait à estimer le bruit de fond correspondant à une hybridation entre deux fragments nucléotidiques de séquence non complémentaire. (Affymetrix technical note "Statistical Algorithms Reference Guide"; Lipshutz, et al (1999) Nat. Genet. 1 Suppl., 20-24). Les 31 échantillons de l'étude montraient une moyenne de 37 % de gènes exprimés.

L'analyse des données d'expression a été réalisée par le logiciel Microsoft Excel, le logiciel Spotfire Decision Site for Functionnal Genomics V7.1 (Spotfire AB, Gothenburg, Sweden), ainsi qu'un algorithme statistique : l'Algorithme Génétique (Gautier, L. et al., Bioinformatics (2004), p.307-315 ; Ooi, C.H. and Tan, P. Bioinformatics (2003), p.37-44).A partir des 22 283 groupes de sondes, représentant environ 14 500 gènes, de la puce, les inventeurs ont dûment sélectionné les gènes pertinents qui permettaient de différencier les patients AIA des patients ATA.

Pour cela, une première étape a consisté à exclure les gènes présentant un niveau d'expression comparable entre tous les groupes de patients. Quatre étapes ont été effectuées :
- les gènes non exprimés chez l'ensemble des patients ont été exclus (logiciel MAS5.0).
- les gènes dont la médiane de fluorescence était inférieure à 30 dans les deux groupes ont été exclus
- les gènes qui n'étaient pas exprimés chez au moins 30 % des patients dans l'un des deux groupes ont été exclus
- les gènes pour lesquels le rapport des médianes d'expression entre les patients AIA et ATA étaient compris entre 0,77 et 1,3 ont été exclus.

Suite à l'application de ces filtres, un groupe de 1383 groupe de sonde a été sélectionné et a servi de base de travail pour une analyse multi-paramétrique avec l'Algorithme Génétique.

*Résultats obtenus:* une liste de 25 gènes a été identifiés. L'augmentation ou la diminution d'expression de chacun de ces gènes, observée chez les patients AIA par rapport aux patients ATA est indiquée dans le tableau 2. Tableau 2 - Liste de 25 gènes exprimés différentiellement chez les patients AIA et ATA

| **SEQ ID N°** | **Nom de gène** | **Nom abrégé** | **Expression chez AIA versus ATA** |
|---|---|---|---|
| **1** | Alstrom syndrome 1 | ALMS1 | augmenté |
| **2** | annexin A3 = lipocortin 3 | ANXA3 | diminué |
| **3** | ATP-binding cassette, sub-family A (ABC1), member 1 | ABCA1 | diminué* |
| **4** | B-cell CLL/lymphoma 6 (zinc finger protein 51) | BCL6 | diminué* |
| **5** | carcinoembryonic antigen-relatedcell adhesion molecule 1 (biliary glycoprotein) | CEACAM1 | diminué * |
| **6** | cell division cycle 42 (GTP binding protein, 25kDa) | CDC42 | augmenté * |
| **7** | Charot-Leyden crystal protein = Galectin 10 | CLC | augmenté |
| **8** | claudin 18 | CLDN18 | diminué* |
| **9** | cofactor required for Sp1 transcriptional activation, subunit 2, 150kDa | CRSP2 | augmenté |
| **10** | C-type (calcium dépendent, carbohydrate recognition domain)lectin, superfamily member 14 (macrophage-derived) | CLECSF14 | augmenté |
| **11** | glutathione S-transferase M 4 | GSTM 4 | augmenté * |
| **12** | homeodomain interacting protein kinase 3 | HIPK3 | diminué* |
| **13** | Homo sapiens cDNA clone IMAGE:5218466 | | augmenté |
| **14** | hypothetical protein FLJ35827 | FLJ35827 | augmenté |
| **15** | KIAA0329 gene product | KIAA0329 | diminué* |
| **16** | major histocompatibility complex, class II, DP bêta 1 | HLA-DPB1 | augmenté |
| **17** | MAX dimerization protein 4 | MXD4 | augmenté * |
| **18** | Metallothionein 1H | MT1H | augmenté * |
| **19** | N-acetylglucosamine-1-phosphodiesteralpha-N-acetylglucosaminidase | NAGPA | augmenté * |
| **20** | phospholipase A2, group V | PLA2G5 | augmenté |
| **21** | protein tyrosine phosphatasgnon-receptor type 22 (lymphoid) | PTPN22 | diminué |
| **22** | RNA binding motif protein 25 | RBM25 | diminué |
| **23** | serine (or cysteine) proteinase inhibitor, clade B (ovalbumin), member 2 | SERPINB2 | diminué |
| **24** | TATA box binding protein (TBP) -associated factor,RNA polymerase I, A, 48kDa | TAF1A | diminué |
| **25** | UDP-N-acetyl-alpha-D-galactosamine:polypeptideN-acetylgalactosaminyltransferase 3 (GalNAc-T3) | GALNT3 | diminué* |

| | | | |
|---|---|---|---|
| L'indication d'une * indique une différence statistiquement différente entre les deux groupes (test T avec correction de Benjamini et Hochberg), indiquant que ces 15 gènes pris isolément sont très pertinents dans le diagnostic d'une intolérance à l'aspirine. | | | |

### Validation par RT-PCR quantitative

Afin de confirmer ces résultats à l'aide d'une autre technique de biologie moléculaire, certains gènes ont été dosé par RT-PCR quantitative. Brièvement, une réaction de transcription reverse (RT) a été réalisée dans un volume final de 20 µl. L'ARN total (1 µg) a été mélangé avec 1 µl de polyT à 50 µM et 1 µl de dNTP mix (ThermoScript^{™} RT-PCR system, Invitrogen), puis incubé 5 min à 65°C. Après refroidissement dans la glace, la solution a été mélangée avec 4 µl de 5x cDNA synthesis buffer, 1 µl de RNAse out (40 U/µl), 1 µl d'eau traitée au DEPC et 1 µl de Thermoscript RT (15 U/µl), tous ces produits étant issus du TherrnoScript^{™} RT-PCR system (Invitrogen). La transcription reverse a été effectuée pendant 1 h à 50°C puis stoppée par une incubation de 5 min à 85°C. Pour finir, chaque solution de cDNA a été diluée au 1/10 dans de l'eau DEPC.

Pour chacun des gènes d'intérêt, un standard a été préparé par une amplification PCR (polymerase chain reaction) conduite jusqu'à saturation. Les amplicons obtenus ont été purifiés (PCR purification kit, Qiagen Ltd) et la présence d'un amplicon unique a été vérifié par électrophorèse sur gel d'agarose et marquage au bromure d'éthidium.

Le standard du gène « de ménage » Peptidylpropyl isomerase B (PP1B) codant pour la cyclophilin B ont été obtenus chez Search-LC (Heidelberg, Allemagne).

### Analyse de l'expression des ARNm par PCR en temps réel

Les ARNm des gènes cibles de SEQ ID N°2 : Annexin A3 ; SEQ ID N°5 : carcinoembryonic antigen-related cell adhesion molecule 1 (CEACAMI), SEQ ID N°4: BCL6 et SEQ ID N°7 : Galectin 10 (CLC) ont été quantifiés par PCR quantitative en temps réel en utilisant le LightCycler^{™} (Roche). Les réactions PCR ont été effectuées à l'aide du Fast-Start^{™} DNA Master SYBR Green I real-time PCR kit (Roche Molecular Biochemicals). Chaque PCR a été effectuée dans un volume final de 20 µl contenant 1 µl de LC-Fast Start Reaction Mix SYBR Green I, 1 µl de LC-Fast Start DNA Master SYBR Green I/Enzyme (incluant la Taq DNA polymerase, le tampon de réaction et un mélange de deoxynucleotides triphosphate), du MgCb (3 mM concentration finale), les amorces sens et anti-sens (0.5 µM concentration finale), et 10 µl de solution de cDNA. Après une étape de dénaturation de 10 min à 95°C, l'amplification a été effectuée à l'aide de 40 cycles d'un protocole de "touch-down" PCR (10 s à 95°C, 10 s d'hybridation" à 68-58°C, suivi d'une extension de 16 s à 72°C). A la fin de chaque cycle, la fluorescence émise par le SYBR Green a été mesurée.

Pour confirmer la spécificité de l'amplification, les produits PCR ont systématiquement fait l'objet d'une analyse de courbe de fusion (LightCycler^{™} - Roche). Pour cela, les produits PCR ont été traités par une température croissante de 58 à 98°C avec une augmentation de 0,1°C/s. Pour chaque produit PCR, un seul pic a été obtenu lors de l'analyse de la courbe, caractérisé par une température de fusion spécifique.

Les combinaisons d'amorces nécessaires à la quantification du gène de ménage PPIB ont été obtenu chez Search-LC (Heidelberg, Allemagne). Les couples d'amorces utilisés pour doser les gènes d'intérêts, la séquence Genbank utilisée comme référence et la position des amplicons sont décrits dans le tableau ci dessous.

| **Gene** | **Primer sens 5'→'3** | **Primer anti-sens 5'→3'** | **amplicon** |
|---|---|---|---|
| ANNEXIN 3 (SEQ ID N°2) | SEQ ID N°26 : CTTTAGCCCATCAGTGGATGC | SEQ ID N°27 : GAGAGATCACCCITCAAGTCATC | 97-276 |
| BCL6(SEQID N° 4) | SEQ ID N°28 : GTGCTTATCCACACTGGTGAG | SEQ ID N°29 : AGGTTACACTTCTCACAATGG | 2188-2321 |
| GALECTIN 10 (SEQ ID N° 7) | SEQ ID N°30: ATATGCCCTTTCAGGATGGCC | SEQ ID N°31: CTTCACAGCCTCAGGCTTGAT | 315-442 |
| CEACAMI (SEQ ID N° 5) | SEQ ID N°32: TTGTGATTGGAGTAGTGGCCC | SEQ ID N°33 GTCATTGGAGAGGTCCTGAGT | 1370-1521 |
| PPIB | Search LC (Heidelberg. Allemagne) | Search LC (Heidelberg. Allemagne) | 105-338 |

La quantité d'ARNm cible relative à la quantité d'ARNm du gène de ménage PPIB a été analysée par la technique de quantification relative avec le LightCycler Relative Quantification Software (Roche Molecular Biochemicals). La "Second Derivative Maximum Method" du logiciel LightCycler^{™} (Roche) a été utilisée pour déterminer automatiquement le Crossing Point (Cp) pour chaque échantillon. La valeur du Cp a été définie comme le nombre de cycles pour lequel la fluorescence était significativement différente du bruit de fond.

Cinq dilutions en séries au 1/10 ont été réalisées en quadruplicate avec chaque standard afin de générer une courbe d'étalonnage exprimant le Cp en fonction du logarithme du nombre de copies. Les dilutions de standard ont été optimisées afin que la courbe d'étalonnage couvre le niveau d'expression attendu pour le gène cible et le gène-de ménage. Les courbes standards relatives décrivant l'efficacité de PCR pour le gène cible et le gène de ménage ont été générées et utilisées pour réaliser une quantification avec le LightCycler Relative Quantification Software (Roche Molecular Biochemicals).

Les résultats obtenus pour le dosage des ARNm des gènes cibles de SEQ ID N°2 : Annexin A3 ; SEQ ID N°5 : carcinoembryonic antigen-related cell adhesion molecule 1 (CEACAM1), SEQ ID N°4 : BCL6 et SEQ ID N°7 : Galectin 10 (CLC)) en Rf-PCR quantitative sont présentés dans le tableau 5 ci dessous. Les résultats correspondent à 26 échantillons (14 AIA et 12 ATA). La corrélation des résultats obtenus d'une part avec la biopuce et d'autre part avec la technique en RT-PCR quantitative a été établie grâce au test de corrélation de Spearman.

**Tableau 2 - Comparaison des niveaux d'expression de 4 gènes entre Affymetrix et RT-PCR quantitative.**

| **Nom du gène abrégé** | **médiane Affymetrix** **AIA** | **médiane Affymetrix** **ATA** | **médiane RT-** **PCR AIA** | **médiane** **RT-PCR ATA** | **Spearman coefficient corrélation: r** | **Spearman coefficient correlation: p** |
|---|---|---|---|---|---|---|
| ANXA3 | 130.21 | 257.35 | 0.002365 | 0.00667 | 0.86 | < 0.0001 |
| CEACAM1 | 177.64 | 266.56 | 0.00156 | 0.00328 | 0,82 | < 0.0001 |
| CLC | 1465.9 | 914.79 | 0.0381 | 0.02735 | 0.78 | < 0,0001 |
| BCL6 | 1333.46 | 2333.59 | 0.00782 | 0,01715 | 0.75 | < 0,0001 |

Pour les 4 gènes analysés, une corrélation significative (r>0.7, p<0.0001) a été observée entres les résultats Affymetrix et ceux de RT-PCR quantitative, confirmant la pertinence des gènes selon l'invention.

### Analyse de l'expression de panel de gènes

Les inventeurs ont également mis en évidence que l'analyse simultané de l'expression de plusieurs gènes était très pertinente pour discriminer des patients ATA et AIA.

Les inventeurs ont ainsi mis en évidence que l'analyse simultanée de l'expression des 25 gènes décrit précédemment était très pertinente pour discriminer les deux groupes de patients asthmatiques.

Les résultats sont présentés dans la figure 1. Cette liste permettait de clusteriser 100% des échantillons de patient AIA dans un groupe et 86.7% des échantillons de patients ATA dans un autre groupe.

De plus, les inventeurs ont mis en évidence que l'analyse de l'expression d'une liste de 19 gènes (tableau 3), compris parmi les 25 gènes décrit précédemment était très pertinente pour discriminer les deux groupes de patients asthmatiques.

**Tableau 3 - Liste de 19 gènes exprimés différentiellement chez les patients AIA et ATA**

| **SEQ ID N°** | **Nom de gène** | **Nom abrégé** | **Expression chez AIA versus ATA** |
|---|---|---|---|
| 1 | Alstrom syndrome 1 | ALMS1 | Augmenté * |
| 2 | annexin A3 = lipocortin 3 | ANXA3 | Diminué * |
| 3 | ATP-binding cassette, sub-family A (ABC1), member 1 | ABCA1 | Diminué * |
| 4 | B-cell CLL/lymphoma 6 (zinc finger protein 51) | BCL6 | Diminué. * |
| 5 | carcinoembryonic antigen-relatedcell adhesion molecule 1 (biliary glycoprotein) | CEACAM1 | Diminué * |
| 6 | cell division cycle 42 (GTP binding protein, 25kDa) | CDC42 | Augmenté * |
| 7 | Charot-Leyden crystal protein = Galectin 10 | CLC | augmenté |
| 8 | claudin 18 | CLDN18 | Diminué * |
| 9 | cofactor required for Sp1transcriptional activation, subunit 2, 150kDa | CRSP2 | augmenté |
| 10 | C-type (calcium dependent, carbohydrate recognition domain)lectin, superfamily member 14 (macrophage-derived) | CLECSF14 | augmenté |
| 13 | Homo sapiens cDNA clone IMAGE:5218466 | | augmenté |
| 14 | hypothetical protein FLJ35827 | FLJ35827 | augmenté |
| 15 | KIAA0329 gene product | KIAA0329 | Diminué * |
| 17 | MAX dimerization protein 4 | MXD4 | Augmenté * |
| 18 | Metallothionein 1H | MT1H | Augmenté * |
| 19 | N-acetylglucosamine-1-phosphodiesteralpha-N-acetylglucosaminidase | NAGPA | Augmenté * |
| 20 | phospholipase A2, group V | PLA2G5 | augmenté |
| 21 | protein tyrosine phosphatase,non-receptor type 22 (lymphoid) | PTPN22 | diminué |
| 24 | TATA box binding protein (TBP) -associated factor,RNA polymerase I, A, 48kDa | TAF1A | diminué |

Les résultats sont présentés dans la figure 2. Cette liste permettait de clusteriser 100% des échantillons de patient AIA dans un groupe et 86.7% des échantillons de patients ATA dans un autre groupe.

Les inventeurs ont également mis en évidence que l'analyse de l'expression d'une liste plus petit, comprenant 17 gènes (tableau 4), compris parmi les 25 gènes décrit précédemment était également très pertinente pour discriminer les deux groupes de patients asthmatiques.

**Tableau 4 - Liste de 17 gènes exprimés différentiellement chez les patients AIA et ATA**

| **SEQ ID N°** | **Nom du gène** | **Nom abrégé** | **Expression chez AIA versus ATA** |
|---|---|---|---|
| **1** | Alstrom syndrome 1 | ALMS1 | Augmenté * |
| **2** | annexin A3 = lipocortin 3 | ANXA3 | Diminué * |
| **3** | ATP-binding cassette, sub-family A (ABC1 ), member 1 | ABCA1 | Diminué * |
| **4** | B-cell CLL/lymphoma 6 (zinc finger protein 51) | BCL6 | Diminué * |
| **5** | carcinoembryonic antigen-relatedcell adhesion molecule 1 (biliary glycoprotein) | CEACAM1 | Diminué * |
| **6** | cell division cycle 42 (GTP binding protein, 25kDa) | CDC42 | Augmenté * |
| **8** | claudin 18 | CLDN18 | Diminué * |
| **11** | glutathione S-transferase M 4 | GSTM24 | Augmenté * |
| **12** | homeodomain interacting protein kinase 3 | HIPK3 | Diminué * |
| **15** | KIAA0329 gène product | KIAA0329 | Diminue * |
| **16** | major histocompatibility complex, class II, DP beta 1 | HLA-DPB1 | Augmenté * |
| **17** | MAX dimerization protein 4 | MXD4 | Augmenté * |
| **18** | Metallothionein 1 H | MT1H | Augmenté * |
| **19** | N-acetylglucosamine-1-phosphodiesteralpha-N-acetylglucosaminidase | NAGPA | Augmenté * |
| **22** | RNA binding motif protein 25 | RBM25 | diminué |
| **23** | serine (or cysteine) proteinase inhibitor, clade B (ovalbumin), member 2 | SERPINB2 | diminué |
| **25** | UDP-N-acetyl-alpha-D-galactosamine:polypeptideN-acetylgalactosaminyltransferase 3 (GalNAc-T3) | GALNT3 | Diminué * |

Les résultats sont présentés dans la figure 3. D'une manière comparable à la liste de 19 gènes, cette liste de 17 gènes permettait de clusteriser 100% des échantillons de patient AIA dans un groupe et 86.7% des échantillons de patients ATA dans un autre groupe.

Les inventeurs ont également mis en évidence que l'analyse de l'expression d'une liste très réduite, comprenant seulement 11 gènes (tableau 5), compris parmi les 25 gènes décrit précédemment était également très pertinente pour discriminer les deux groupes de patients asthmatiques.

**Tableau 5 - Liste des 11 gènes communs à la liste de 19 et de 17 gènes**

| **SEQ ID N°** | **Nom de gène** | **Nom abrégé** | **Expression chez AIA versus ATA.** |
|---|---|---|---|
| **1** | Alstrom syndrome 1 | ALMS1 | Augmenté * |
| **2** | annexin A3 - lipocortin 3 | ANXA3 | Diminué * |
| **3** | ATP-binding cassette, sub-family A (ABC1), member 1 | ABCA1 | Diminué * |
| **4** | B-cell CLL/lymphoma 6 (zinc finger protein 51) | BCL6 | Diminué * |
| **5** | carcinoembryonic antigen-relatedcell adhesion molecule 1 (biliary glycoprotein) | CEACAM1 | Diminué * |
| **6** | cell division cycle 42 (GTP binding protein, 25kDa) | CDC42 | Augmenté * |
| **8** | claudin 18 | CLDN18 | Diminué * |
| **15** | KIAA0329 gene product | KIAA0329 | Diminué * |
| **17** | MAX dimerization protein 4 | MXD4 | Augmenté * |
| **18** | Metallothionein 1H | MT1H | Augmenté * |
| **19** | N-acetylgtucosamine-1-phosphodiesteralpha- N-acetylglucosaminidase | NAGPA | Augmenté * |

Les résultats sont présentés dans la figure 4, respectivement. Cette liste permettait de clusteriser 100% des échantillons de patient AIA dans un groupe et 86.7% des échantillons de patients ATA dans un autre groupe.

En conclusion, quelle que soit la liste de gènes utilisées, les patients asthmatiques atteint d'une intolérance à l'aspirine étaient systématiquement discriminés, il est alors possible de leur proposer un traitement adapté, et surtout d'éviter de leur prescrire un traitement comprenant à base d'AINS qui pourrait avoir des conséquences dramatiques. De plus, l'utilisation de panel de gènes restreint est particulièrement adapté pour obtenir un outil de diagnostic. En effet, l'analyse de l'expression d'une dizaine de gènes ne nécessite pas la fabrication à façon de supports hautes densités, et peut être mise en oeuvre directement par des techniques de PCR ou de NASBA ou de supports basses densité, ce qui présente un atout économique important et une mise en oeuvre simplifiée.

## Revendications

1. Procédé pour le diagnostic d'une intolérance à l'aspirine à partir d'un échantillon biologique d'un patient asthmatique **caractérisé en ce qu'**il comprend les étapes suivantes :
a. on extrait du matériel biologique de l'échantillon biologique,
b. on met en contact le matériel biologique avec au moins 11 réactifs spécifiques choisis parmi les réactifs spécifiques des gènes cibles présentant une séquence nucléique ayant l'une quelconque des SEQ ID N° 1 à 6 ; 8 ; 15 ; 17 à 19;
c. on détermine l'expression d'au moins 10 desdits gènes cibles.

2. Procédé pour le diagnostic d'une intolérance à l'aspirine selon la revendication 1 **caractérisé en ce que** l'échantillon biologique prélevé chez le patient est un échantillon sanguin.

3. Procédé selon la revendication 1 ou 2 **caractérisé en ce que** le matériel biologique extrait lors de l'étape a) comprend des acides nucléiques.

4. Procédé selon la revendication 3 **caractérisé en ce que** le au moins un réactif spécifique de l'étape b) comprend au moins une sonde d'hybridation

5. Procédé selon la revendication 4 **caractérisé en ce que** la au moins une sonde d'hybridation est immobilisée sur un support.

6. Procédé selon l'une quelconque des revendications 1 à 5 **caractérisé en ce que** lors de l'étape b) on met en contact le matériel biologique avec au moins 25 réactifs spécifiques choisis parmi les réactifs spécifiques des gènes cibles présentant une séquence nucléique ayant l'une quelconque des SEQ ID N° 1 à 25 et on détermine, lors de l'étape c, l'expression d'au moins 25 desdits gènes cibles.

7. Procédé selon l'une quelconque des revendications 1 à 5 **caractérisé en ce que** lors de l'étape b) on met en contact le matériel biologique avec au moins 17 réactifs spécifiques choisis parmi les réactifs spécifiques des gènes cibles présentant une séquence nucléique ayant l'une quelconque des SEQ ID N° 1 à 6 ; 8 ; 11 à 12 ; 15 à 19 ; 22 à 23 et 25 et on détermine, lors de l'étape c, l'expression d'au moins 17 desdits gènes cibles.

8. Procédé selon l'une quelconque des revendications 1 à 5 **caractérisé en ce que** lors de l'étape b) on met en contact le matériel biologique avec au moins 19 réactifs spécifiques choisis parmi les réactifs spécifiques des gènes cibles présentant une séquence nucléique ayant l'une quelconque des SEQ ID N° 1 à 10 ; 13 à 15 ; 17 à 21 ; 24 et on détermine, lors de l'étape c, l'expression d'au moins 19 desdits gènes cibles.

## Claims

1. Method for diagnosing aspirin intolerance using a biological sample from an asthmatic patient, **characterized in that** it comprises the following steps:
a. biological material is extracted from the biological sample;
b. the biological material is brought into contact with at least 11 specific reagents chosen from the reagents specific for the target genes exhibiting a nucleic sequence having any one of SEQ ID Nos 1 to 6; 8; 15; and 17 to 19;
c. the expression of at least 10 of said target genes is determined.

2. Method for diagnosing aspirin intolerance according to Claim 1, **characterized in that** the biological sample taken from the patient is a blood sample.

3. Method according to Claim 1 or 2, **characterized in that** the biological material extracted during step a) comprises nucleic acid.

4. Method according to Claim 3, **characterized in that** the at least one specific reagent of step b) comprises at least one hybridization probe.

5. Method according to Claim 4, **characterized in that** the at least one hybridization probe is immobilized on a support.

6. Method according to any one of Claims 1 to 5, **characterized in that**, during step b), the biological material is brought into contact with at least 25 specific reagents chosen from the reagents specific for the target genes exhibiting a nucleic sequence having any one of SEQ ID Nos 1 to 25, and, during step c), the expression of at least 25 of said target genes is determined.

7. Method according to any one of Claims 1 to 5, **characterized in that**, during step b), the biological material is brought into contact with at least 17 specific reagents chosen from the reagents specific for the target genes exhibiting a nucleic sequence having any one of SEQ ID Nos 1 to 6; 8; 11 to 12; 15 to 19; 22 to 23 and 25, and, during step c), the expression of at least 17 of said target genes is determined.

8. Method according to any one of Claims 1 to 5, **characterized in that**, during step b), the biological material is brought into contact with at least 19 specific reagents chosen from the reagents specific for the target genes exhibiting a nucleic sequence having any one of SEQ ID Nos 1 to 10; 13 to 15; 17 to 21; and 24, and, during step c), the expression of at least 19 of said target genes is determined.

## Patentansprüche

1. Verfahren zur Diagnostizierung von Aspirinunverträglichkeit unter Verwendung einer biologischen Probe eines Asthmatikers, **dadurch gekennzeichnet, dass** es die folgenden Schritte umfasst:
a. Entziehen von biologischem Material aus der biologischen Probe,
b. In Kontakt bringen des biologischen Materials mit mindestens 11 spezifischen Reaktanten, ausgewählt aus der Reihe der für Zielgene mit einer Nukleinsäuresequenz gemäß einer der Sequenzen SEQ ID Nr. 1 bis 6, 8, 15, 17 bis 19, spezifischen Reaktanten
c. Bestimmung der Expression von mindestens 10 dieser Zielgene.

2. Verfahren zur Diagnostizierung von Aspirinunverträglichkeit nach Anspruch 1, **dadurch gekennzeichnet, dass** es sich bei der von dem Patienten entnommenen biologischen Probe um eine Blutprobe handelt.

3. Verfahren nach Anspruch 1 oder 2, **dadurch gekennzeichnet, dass** das in Schritt a) entzogene biologische Material Nukleinsäuren umfasst.

4. Verfahren nach Anspruch 3, **dadurch gekennzeichnet, dass** der mindestens eine spezifische Reaktant von Schritt b) mindestens eine Hybridisierungssonde umfasst.

5. Verfahren nach Anspruch 4, **dadurch gekennzeichnet, dass** die mindestens eine Hybridisierungssonde auf einem Träger immobilisiert ist.

6. Verfahren nach einem der Ansprüche 1 bis 5,
**dadurch gekennzeichnet, dass** man in Schritt b) das biologische Material mit mindestens 25 spezifischen Reaktanten, ausgewählt aus der Reihe der für Zielgene mit einer Nukleinsäuresequenz gemäß einer beliebigen Sequenz SEQ ID Nr. 1 bis 25 spezifischen Reaktanten in Kontakt bringt und dass man im Schritt c die Expression von mindestens 25 dieser Zielgene bestimmt.

7. Verfahren nach einem der Ansprüche 1 bis 5,
**dadurch gekennzeichnet, dass** man in Schritt b) das biologische Material mit mindestens 17 spezifischen Reaktanten, ausgewählt aus der Reihe der für Zielgene mit einer Nukleinsäuresequenz gemäß einer beliebigen Sequenz SEQ ID Nr. 1 bis 6, 8, 11 bis 12, 15 bis 19, 22 bis 23 und 25 spezifischen Reaktanten in Kontakt bringt und dass man im Schritt c die Expression von mindestens 17 dieser Zielgene bestimmt.

8. Verfahren nach einem der Ansprüche 1 bis 5,
**dadurch gekennzeichnet, dass** man in Schritt b) das biologische Material mit mindestens 19 spezifischen Reaktanten, ausgewählt aus der Reihe der für Zielgene mit einer Nukleinsäuresequenz gemäß einer beliebigen Sequenz SEQ ID Nr. 1 bis 10, 13 bis 15, 17 bis 21, 24 spezifischen Reaktanten in Kontakt bringt und dass man im Schritt c die Expression von mindestens 19 dieser Zielgene bestimmt.


### SEQUENCE LISTING

<110> bioMerieux SA
<120> Procédé pour le diagnostic d'une intolérance à l'aspirine
<130> Unknown
<160> 33
<170> Patent In version 3.3
<210> 1
   <211> 12922
   <212> DNA
   <213> Homo sapiens
<400> 1 
aggcgggcgg cactgcgcct aagctgggcc acaaccgcca gtcagggctc tccccttccc 60
ctccctcccc ccctcctcct cctcctctgc cgcccagagc gagacaccaa catggagccc 120
gaggatctgc catggccggg cgagctggag gaggaggagg aggaggagga ggaggaggag 180
gaggaagagg aggaggctgc agcggcggcg gcggcgaacg tggacgacgt agtggtcgtg 240
gaggaggtgg aggaagaggc ggggcgggag ttggactccg actctcacta cgggccccag 300
catctggaaa gtatagacga cgaggaggac gaggaggcca aggcctggct gcaggcgcac 360
cccggcagga ttttgcctcc gctgtcgccc ccgcagcacc gctactcgga gggcgagcgg 420
acctccctgg agaagattgt tccattgacc tgtcatgtat ggcaacagat agtatatcaa 480
ggcaatagta gaacacaaat ttctgatact aatgtggtct gtttggaaac aacagctcag 540
cggggttctg gggatgatca gaaaacagaa tcttggcatt gtcttcctca agaaatggac 600
tcttcccaaa ccttggatac atcccagact aggtttaatg tgagaacgga agatactgaa 660
gtgacagact tcccctctct ggaggagggc atattgacgc aatcagaaaa tcaagtaaag 720
gaacccaaca gagatctctt ctgttctcca ctgctagtca tacaagatag ctttgcttct 780
cctgatttgc ctttgctgac ctgtttgaca caagaccaag aatttgcgcc tgattcttta 840
tttcatcaaa gtgaactaag ttttgcacct ctgaggggaa ttcctgataa gtctgaagat 900
actgaatggt cttctcgacc atcggaagtt agtgaagctt tattccaggc tactgcagaa 960
gtagcttcag acttagcaag cagtcgcttt agtgtatctc agcacccgct tataggcagc 1020
acagctgttg ggtctcagtg ccctttttta ccttctgaac aagggaataa tgaagagact 1080
atttcgtctg ttgatgaact gaaattcccc aaagactgtg atcgttatga tgatctttgt 1140
tcatatatgt catggaagac acgaaaagat acacagtggc ctgaaaacaa tttagctgat 1200
aaagatcaag tttcagttgc aacttcattt gacataactg atgaaaacat agctactaaa 1260
agaagtgacc attttgatgc tgctcgttca tatgggcagt attggacaca ggaagattca 1320
tctaagcagg cagaaacata tttaaccaag ggcctgcagg ggaaggttga gtctgacgtc 1380
attactctgg atggcctaaa tgaaaatgct gttgtatgca gtgaaagagt tgctgaacta 1440
caaagaaagc caacaagaga gtcggaatat cactcttcag atctcagaat gttgaggatg 1500
tctcctgaca ctgtgccaaa ggctcctaaa catttaaaag caggagacac ttctaaagga 1560
ggcatagcta aagttactca atccaacttg aagtcaggca tcactaccac tcctgttgat 1620
tcagacattg gatctcattt atccttgtcc cttgaggacc tgtctcagtt ggctgtaagt 1680
 
tctctagaaa ctactactgg tcaacacact gatactctca accaaaagac attagcagat 1740
actcatctaa ctgaagagac tctgaaagtc acagctattc ctgaaccagc tgaccagaag 1800
actgcaacac caacagtact ctctagttcc cactcacata gggggaagcc cagcattttc 1860
taccagcagg gcttgccaga cagtcatcta actgaagagg ctttgaaagt ttcagctgct 1920
cctggactag ctgaccagac aactggcatg tcaactctaa cctctacttc ctactcacat 1980
agagagaagc ctggtacttt ttaccaacaa gagttaccag agagtaactt aaccgaagag 2040
cctttggaag tttcagctgc tcctggccca gtggagcaga agacgggaat acctacagta 2100
tcctctacat cccactcaca tgtagaggac ctcctctttt tctatcgaca gaccttgcca 2160
gatggtcatc taactgatca ggctctgaaa gtctcagctg tgtctggacc agctgaccag 2220
aagactggga cagcaacagt actctctact ccccactcac atagagagaa gcctggtatt 2280
ttttaccaac aagagttcgc agacagtcat caaactgaag agactcttac taaagtttca 2340
gccactcctg gaccagctga ccagaagact gagataccag cagtacagtc tagttcttac 2400
tcacaaagag aaaagcctag tattttgtac ccacaggact tagcagacag tcatctacct 2460
gaagagggtc tgaaagtttc agctgttgct ggaccagctg accagaagac tggcctacca 2520
acagtaccct ctagtgcata ctcacacaga gagaagctcc ttgttttcta ccaacaggcc 2580
ttgctggaca gccatctacc cgaagaggct ctgaaagttt cagctgtttc tggaccagct 2640
gacggaaaga ctgggacacc agctgtaacc tctacttcct ctgcgtcctc ttcacttgga 2700
gaaaagccca gtgctttcta tcagcagacc ttacccaata gtcatctaac tgaagaggct 2760
ctgaaagtat caattgttcc tggaccaggt gatcagaaga ctgggatacc ctcagcacca 2820
tctagtttct actcacacag agagaagccc attatttttt cccagcagac cctgccagac 2880
tttcttttcc ctgaagaagc tctgaaggtt tcagctgttt ctgtattggc tgcccagaag 2940
actgggacac caacagtgtc ctctaattct cactcacata gcgagaaatc tagtgttttc 3000
taccagcaag agttgccaga cagtgatcta cctagagaat ctctgaaaat gtctgctatt 3060
cctggactga ctgaccagaa gactgtccca acaccaacag taccttcagg ttccttctca 3120
catagagaga agcccagtat tttctatcaa caggagtggc cagatagtta tgcaactgaa 3180
aaggctctga aagtttcaac tggccctgga ccagctgacc agaagactga gataccagca 3240
gtacagtcta gttcttaccc acagagggag aagcctagtg ttttgtaccc acaggtgtta 3300
tcagacagtc atctacctga agagagtctg aaagtttcag ccttccctgg accagctgac 3360
cagatgactg acacaccagc agtaccgtct actttctact cacaaagaga gaagcctggt 3420
attttctacc aacagacctt gccagagagt catctgccta aagaggctct gaaaatttca 3480
gtagctcctg gactagcaga ccagaagact ggcacaccaa ctgtaacctc aacttcctac 3540
tcacaacata gagaaaagcc cagcattttc caccagcagg ccttgccagg tactcatata 3600
cctgaagagg ctcagaaagt ttcagctgtt actggaccag gtaaccagaa gacttggata 3660
ccaagagtac tttctacctt ctactcacaa agagagaaac ctggtatttt ctatcaacag 3720
accttgccag gtagtcacat acctgaagag gcacagaaag tttcacctgt tcttggacca 3780
gctgaccaga agactgggac accaactcca acctctgctt cttactcaca cacagagaag 3840
 
cctggtattt tctaccaaca ggtcttgcca gataatcatc caactgaaga ggctctgaaa 3900
atttcagttg cctctgaacc agttgaccag acaactggca caccagctgt aacctctact 3960
tcctactcac aatatagaga gaagcccagc attttctacc aacagtcgtt gccaagtagt 4020
catctaactg aagaggctaa gaatgtttca gcggttcctg gaccagctga ccagaagact 4080
gtgataccaa ttttaccctc tactttctac tcacacacag agaagcctgg tgttttctac 4140
caacaggtct tgccacatag tcatccaact gaagaggctc tgaaaatttc agttgcctct 4200
gaaccagttg accagacaac tggcacacca actgtaacct ctacttctta ctcacaacat 4260
acagagaagc cgagtatttt ctaccaacag tcgttgccag gtagtcatct aactgaagag 4320
gctaagaacg tttcagcggt tcctggacca ggtgaccgga agactgggat accaacttta 4380
ccctctactt tctactcaca cacagagaag cctggtagtt tctaccaaca ggtcttgcca 4440
catagtcatc tacctgaaga ggctttggaa gtttcagttg ctcctggacc agttgaccag 4500
acgattggca caccaactgt aacctcccct tccagctcat ttggagagaa gcccattgtt 4560
atctacaaac aggcctttcc agagggtcat ctacctgaag agtctctgaa agtttcagtt 4620
gctcctggac cagttggcca gacaactggc gcaccaacta taacctctcc ttcctactca 4680
caacatagag caaagtctgg cagtttctac caactggcat tgctaggtag tcaaatacct 4740
gaagaggctc tcagagtttc ttctgctcct ggaccagctg accagacaac tggcatacca 4800
accataacct ctacttccta ctcatttgga gagaagccga ttgttaacta caaacaggcc 4860
tttccagatg gtcatctacc tgaagaggct ctgaaagttt ccattgtttc tggacctact 4920
gaaaaaaaga ctgacatacc agcaggacct ttaggttcca gtgcacttgg agagaagccc 4980
attactttct accggcaggc tctgctagac agtcctctaa ataaagaggt tgtgaaagtt 5040
tcagctgctc ctggaccagc tgaccagaag actgagacat taccagtaca ttctactagc 5100
tactcaaata gggggaagcc tgtcattttc taccagcaga ccctatcaga cagtcattta 5160
cctgaagaag ctctgaaagt tccacctgtt cctggaccag atgcccagaa gactgagaca 5220
ccatcagtat cctctagttt atactcatat agagagaagc ccattgtctt ctaccaacag 5280
gccctgccag acagtgagct aactcaagaa gctctgaaag tttcagctgt tcctcaacca 5340
gctgaccaga agactgggtt atctactgta acttcctctt tctattcaca tacagagaag 5400
cctaatattt cttaccagca agagttgcca gatagtcatc taactgaaga ggctctgaaa 5460
gtttcaaatg ttcctggacc agctgaccag aagactgggg tatcaacagt aacctctact 5520
tcctactcac acagagagaa gcccattgtt tcctaccagc gagagttgcc gcattttact 5580
gaagcaggtt tgaaaatttt aagagttcct ggaccagctg accagaagac tggaataaac 5640
atcctgccct ctaattccta cccacagaga gagcactctg tcatttctta tgagcaggag 5700
ttgccagatc ttactgaagt aactttgaaa gcaatagggg ttcctgggcc tgctgaccag 5760
aagactggga tacaaatagc atcctctagt tcctactcaa atagagagaa ggccagtatt 5820
tttcatcagc aggagttgcc agatgttact gaagaagctt taaatgtttt tgttgttcct 5880
ggacaaggtg accggaagac tgagatacca acagtacctt taagttacta ctcacgtaga 5940
gagaagccca gtgttatctc tcaacaggag ttgccagaca gtcatctcac agaagaggct 6000
ctgaaagttt cacctgtttc tataccagca gagcagaaga ctgggatacc aataggactg 6060
 
tctagttcct actcacattc acataaagag aaactcaaga tttcaactgt gcatatacca 6120
gatgaccaga aaactgagtt tccagcagct acccttagtt cctactcaca aatagagaag 6180
cccaagattt caactgtgat tggaccaaat gaccagaaga ctccatccca gacagctttt 6240
catagttcct attctcaaac agtaaagccc aatattttat ttcaacagca gttgccagat 6300
agagatcaaa gtaaaggtat tctaaagatt tcagctgtcc ctgaactaac tgatgtgaat 6360
actggaaaac cagtatctct ctctagttct tattttcaca gagagaaatc gaatattttc 6420
agtccacagg aattgccagg tagtcatgta actgaagatg tgctgaaggt ttcaacaatt 6480
cctggaccag ctggccagaa aacagtatta ccaacagctc ttcctagttc cttttcacat 6540
cgagagaaac cagatatttt ctatcaaaag gatttgccag atagacatct aactgaagat 6600
gctctaaaga tctcaagtgc tcttgggcaa gctgatcaaa ttaccggatt acaaacagtt 6660
ccctctggta cttactcaca tggtgagaat cacaagcttg tttcagaaca tgtccaaagg 6720
ctaatagata atttgaattc ttctgactcc agtgttagct caaataatgt gcttttaaat 6780
tctcaggctg atgacagagt tgtaataaat aaaccagaat ctgcaggttt tagagatgtt 6840
ggctctgaag aaatccagga tgcagaaaat agtgctaaaa ctcttaagga aattcggaca 6900
cttttgatgg aggcagaaaa tatggcactg aaacgatgca attttcctgc tccccttgcc 6960
cgtttcagag atattagtga tatttcattt atacaatcta agaaggtggt ttgcttcaaa 7020
gaaccctctt ccacgggtgt atctaatggt gatttgcttc acagacagcc attcacagag 7080
gaaagcccaa gcagcaggtg catacagaag gatattggca cacagacgaa tttgaaatgc 7140
cggagaggca ttgaaaattg ggagtttatt agttcaacta cagttagaag tcctctacag 7200
gaagcagaga gcaaagtcag tatggcatta gaagaaactc ttaggcaata tcaagcagcc 7260
aaatctgtaa tgaggtctga acctgaaggg tgtagtggaa ccattgggaa taaaattatt 7320
atccctatga tgactgtcat aaaaagtgat tcaagtagtg atgccagtga tggaaatggt 7380
tcctgctcgt gggacagtaa tttaccagag tctttggaat cagtttctga tgttcttcta 7440
aacttctttc catatgtttc acccaagaca agtataacag atagcaggga ggaagagggt 7500
gtgtcagaga gtgaggatgg tggtggtagc agtgtagatt cactggctgc acatgtgaaa 7560
aaccttctgc aatgtgaatc ctcactgaat catgctaaag aaatactcag aaatgcagag 7620
gaagaggaaa gccgggtacg agcacatgcc tggaatatga agttcaattt agcacatgat 7680
tgtggatact ccatttcaga attaaatgaa gatgacagga ggaaagtaga agagatcaag 7740
gcagagttat ttggtcatgg aagaacaact gacttgtcca agggtttaca gagtccacgg 7800
ggaatgggat gcaagccaga agctgtatgt aatcacatta ttattgagag ccatgaaaag 7860
ggatgtttcc ggactctaac ttctgaacat ccacaactag atagacaccc ttgtgctttc 7920
agatctgctg gaccctcaga aatgaccaga ggacggcaga acccatcatc atgcagagcc 7980
aagcatgtca acctttctgc atccttagac cagaacaact cccatttcaa agtttggaat 8040
tccttgcagt taaaaagtca ttccccattt cagaacttta tacctgatga attcaaaatc 8100
agcaaaggtc ttcgaatgcc attcgatgaa aagatggacc cttggctgtc agaattagta 8160
gaacctgctt ttgtgccacc taaagaagtg gattttcatt cttcatcaca aatgccgtcc 8220
 
ccagaaccca tgaaaaagtt tactacctcc atcacttttt catctcaccg acattctaaa 8280
tgcatttcca attcctctgt tgttaaggtt ggtgttactg aaggtagcca gtgtactgga 8340
gcatctgtgg gggtatttaa ttctcatttc actgaagaac aaaatcctcc cagagatctt 8400
aaacagaaaa cctcttcccc ttcatcattt aaaatgcata gtaattcaca agataaagaa 8460
gtgactattt tagcagaagg tagaaggcaa agccaaaaat tacctgttga ttttgagcgt 8520
tcttttcaag aagaaaaacc cttagaaaga tcagatttta caggcagtca ttctgagccc 8580
agtaccaggg caaattgtag caatttcaag gaaattcaga tttctgataa ccataccctt 8640
attagcatgg gcagaccaag ttccacccta ggagtaaaca gatcgagttc cagactagga 8700
gtaaaagaga agaatgtaac tataactcca gatcttcctt cttgcatttt tcttgaacaa 8760
cgagagctct ttgaacaaag caaagcccca cgtgcagatg accatgtgag gaaacaccat 8820
tctccctctc ctcaacatca ggattatgta gctccagacc ttccttcttg catttttctt 8880
gaacaacgag aactctttga acagtgcaaa gccccatatg tagatcatca aatgagagaa 8940
aaccattctc cccttcctca aggtcaggat tctatagctt cagaccttcc gtctcccatt 9000
tctcttgaac aatgccaaag caaagcgcca ggtgtagatg accaaatgaa taaacaccat 9060
tttccccttc ctcaaggtca ggattgtgta gtggaaaaga ataatcaaca taagcctaaa 9120
tcacacattt ctaatataaa tgttgaagcc aagttcaata ctgtggtctc ccagtcagcc 9180
ccaaatcact gtacattagc agcatctgca tctactcctc cttcaaatag aaaagcactt 9240
tcttgtgttc atataactct ttgtcccaag acttcttcca agttggatag tggaacttta 9300
gatgaaagat tccattcatt ggatgctgct tctaaagcga ggatgaatag tgagtttaac 9360
tttgacttac atactgtatc ttcgagatca ctggaaccaa cctccaaatt attgaccagt 9420
aaacctgtag cacaggatca agaatcttta ggttttctag gacctaaatc ttcactggat 9480
ttccaagtcg tacagccttc tcttccagac agtaacacta ttactcagga cttgaaaacc 9540
ataccttctc agaatagcca gatagtaacc tccaggcaaa tacaagtgaa catttcagat 9600
ttcgaaggac attccaatcc agaggggacc ccagtatttg cagatcgatt accagagaag 9660
atgaagaccc cactttctgc tttctctgaa aaattgtcat ctgatgcagt cactcagata 9720
acaacagaaa gtccagaaaa gaccctattt tcatctgaga tttttattaa tgctgaagat 9780
cgtggacatg aaattataga gcctggtaac cagaagctac gcaaagctcc tgtcaagttt 9840
gcctcatcat cttcagtcca acaggttact ttttctcgcg gcacagatgg ccagccttta 9900
ttattgccat ataagccttc tggtagtacc aagatgtatt atgttccaca attaagacaa 9960
attcctccat ctccggattc caaatcagat accaccgttg aaagctccca ttcaggatcc 10020
aatgatgcca ttgctccaga cttcccagct caggtgctag gcacaagaga tgatgacctc 10080
tcagccactg ttaacattaa acataaagaa ggaatctaca gtaagagggt agtgactaag 10140
gcatccttgc cagtgggaga aaaacccttg cagaatgaaa atgcagatgc ctcagttcaa 10200
gtgctaatca ctggggatga gaacctctca gacaaaaaac agcaagagat tcacagtaca 10260
agggcagtga ctgaggctgc ccaggctaaa gaaaaagaat ctttgcagaa agatactgca 10320
gattccagtg ctgctgctgc tgcagagcac tcagctcaag taggagaccc agaaatgaag 10380
aacttgccag acactaaagc cattacacag aaagaggaga tccataggaa gaagacagtt 10440
 
cccgaggaag cctggccaaa caataaagaa tccctacaga tcaatattga agagtccgaa 10500
tgtcattcag aatttgaaaa tactacccgt tctgtcttca ggtcagcaaa gttttacatt 10560
catcatcccg tacacctacc aagtgatcaa gatatttgcc atgaatcttt gggaaagagt 10620
gttttcatga gacattcttg gaaagatttc tttcagcatc atccagacaa acatagagaa 10680
cacatgtgtc ttcctcttcc ttatcaaaac atggacaaga ctaagacaga ttataccaga 10740
ataaagagcc tcagcatcaa tgtgaatttg ggaaacaaag aagtgatgga tactactaaa 10800
agtcaagtta gagattatcc aaaacataat ggacaaatta gtgatccaca aagggatcag 10860
aaggtcaccc cagagcaaac aactcagcac actgtgagtt tgaatgaact gtggaacaag 10920
tatcgggagc gacagaggca acagagacag cctgagttgg gtgacaggaa agaactgtcc 10980
ttggtggacc gacttgatcg tttggctaaa attcttcaga atccaatcac acattctctc 11040
caggtctcag aaagtacaca tgatgatagc agaggggaac gaagtgtgaa ggaatggagt 11100
ggtagacaac agcagagaaa taagcttcag aaaaagaagc ggtttaaaag cctagagaaa 11160
agccataaaa atacaggcga gcttaaaaaa agcaaggtgc tttctcatca tcgagctggg 11220
aggtctaatc aaattaaaat tgaacagatt aaatttgata aatatattct gagtaaacag 11280
ccaggtttta attatataag caacacttct tcggattgtc ggccctcaga ggagagtgag 11340
ctgctcacag atactaccac caacatcctt tccggcacca cttctactgt cgaatcagat 11400
atattgaccc aaacagatag agaggtggct ctgcacgaaa ggagtagctc tgtttccact 11460
attgacactg cccggctgat tcaagctttt ggccatgaaa gagtatgctt gtcacccaga 11520
cgaattaaat tatatagcag catcaccaac caacagagga gataccttga gaagcggagc 11580
aaacacagca agaaagtgct gaatacaggt catcccctag tgacttctga gcacaccaga 11640
aggagacaca tccaggtagc aaaccatgtg atttcttctg actctatttc ctcttctgcc 11700
agtagtttcc tgagctcaaa ctctactttt tgcaacaagc agaatgtaca catgttaaac 11760
aagggcatac aagcaggtaa cttggagatt gtgaacggtg ccaaaaaaca cactcgagat 11820
gttgggataa ctttcccaac tccaagttcc agcgaggcta aattggaaga gaacagtgat 11880
gtgacttctt ggtcagaaga aaaacgtgaa gagaaaatgc tctttaccgg ttatcctgag 11940
gacagaaagt taaaaaagaa caagaagaat tcccatgaag gagtttcctg gtttgttcct 12000
gtggaaaatg tggagtctag atcaaagaag gaaaacgtgc ctaacacttg tggccctggc 12060
atctcctggt ttgaaccaat aaccaagacc agaccctgga gggagccact gcgggagcag 12120
aactgtcagg ggcagcacct ggacggtcgg ggctacctgg caggcccagg cagagaggct 12180
ggcagagacc tactgaggcc atttgtgaga gcaacccttc aggaatcgct tcagtttcac 12240
agacctgact tcatctcccg ctctggggag cggataaagc gcctgaagtt aatagtccag 12300
gagaggaagc tgcagagcat gttacagacc gagcgggatg cactattcaa cattgacagg 12360
gaacggcagg gccaccagaa tcgcatgtgc ccgctgccca agagagtctt cctggctatc 12420
cagaagaaca agcctatcag caagaaggaa atgattcaga ggtccaaacg gatttatgag 12480
cagcttccag aagtacagaa aaagagagaa gaagagaaga gaaaatcaga atataagtca 12540
taccggctgc gagcccagct atataaaaag agagtgacca atcaacttct ggggagaaaa 12600
 
gttccctggg actgacacaa gtttattttc ctcagagcct tggaattcta ttttatgaac 12660
ctagagaagc agaatcctta cttttgtgag tctggttgaa taaagcttat tctttgtcca 12720
tgtgtatttt agaaatagta acttctaaag agtctggaac aaagtggtga ttaaaattcc 12780
taatggtttg ggagcaatac tttctgcata gtggccttgt ccaatggcct gtgtgttaca 12840
atgatatgat catttctcaa gaataagtcc ctttttgtat gtgtttttat acttttagaa 12900
aataaaaact ttagattaac tc 12922
 <210> 2
   <211> 1339
   <212> DNA
   <213> Homo sapiens
<400> 2 
gaattccgat tagtgtgatc tcagctcaag gcaaaggtgg gatatcatgg catctatctg 60
ggttggacac cgaggaacag taagagatta tccagacttt agcccatcag tggatgctga 120
agctattcag aaagcaatca gaggaattgg aactgatgag aaaatgctca tcagcattct 180
gactgagagg tcaaatgcac agcggcagct gattgttaag gaatatcaag cagcatatgg 240
aaaggagctg aaagatgact tgaagggtga tctctctggc cactttgagc atctcatggt 300
ggccctagtg actccaccag cagtctttga tgcaaagcag ctaaagaaat ccatgaaggg 360
cgcgggaaca aacgaagatg ccttgattga aatcttaact accaggacaa gcaggcaaat 420
gaaggatatc tctcaagcct attatacagt atacaagaag agtcttggag atgacattag 480
ttccgaaaca tctggtgact tccggaaagc tctgttgact ttggcagatg gcagaagaga 540
tgaaagtctg aaagtggatg agcatctggc caaacaagat gcccagattc tctataaagc 600
tggtgagaac agatggggca cggatgaaga caaattcact gagatcctgt gtttaaggag 660
ctttcctcaa ttaaaactaa catttgatga atacagaaat atcagccaaa aggacattgt 720
ggacagcata aaaggagaat tatctgggca ttttgaagac ttactgttgg ccatagttaa 780
ttgtgtgagg aacacgccgg cctttttagc cgaaagactg catcgagcct tgaagggtat 840
tggaactgat gagtttactc tgaaccgaat aatggtgtcc agatcagaaa ttgacctttt 900
ggacattcga acagagttca agaagcatta tggctattcc ctatattcag caattaaatc 960
ggatacttct ggagactatg aaatcacact cttaaaaatc tgtggtggag atgactgaac 1020
caagaagata atctccaaag gtccacgatg ggctttccca acagctccac cttacttctt 1080
ctcatactat ttaagagaac aagcaaatat aaacagcaac ttgtgttcct aacaggaatt 1140
ttcattgttc tataacaaca acaacaaaag cgattattat tttagagcat ctcatttata 1200
atgtagcagc tcataaatga aattgaaaat ggtattaaag atctgcaact actatccaac 1260
ttatatttct gctttcaaag ttaagaatct ttatagttct actccattaa atataaagca 1320
agataataaa acggaattc 1339
 <210> 3
   <211> 10412
   <212> DNA
   <213> Homo sapiens
<400> 3 gtaattgcga gcgagagtga gtggggccgg gacccgcaga gccgagccga cccttctctc 60 
ccgggctgcg gcagggcagg gcggggagct ccgcgcacca acagagccgg ttctcagggc 120
gctttgctcc ttgttttttc cccggttctg ttttctcccc ttctccggaa ggcttgtcaa 180
ggggtaggag aaagagacgc aaacacaaaa gtggaaaaca gttaatgacc agccacggcg 240
tccctgctgt gagctctggc cgctgccttc cagggctccc gagccacacg ctgggggtgc 300
tggctgaggg aacatggctt gttggcctca gctgaggttg ctgctgtgga agaacctcac 360
tttcagaaga agacaaacat gtcagctgct gctggaagtg gcctggcctc tatttatctt 420
cctgatcctg atctctgttc ggctgagcta cccaccctat gaacaacatg aatgccattt 480
tccaaataaa gccatgccct ctgcaggaac acttccttgg gttcagggga ttatctgtaa 540
tgccaacaac ccctgtttcc gttacccgac tcctggggag gctcccggag ttgttggaaa 600
ctttaacaaa tccattgtgg ctcgcctgtt ctcagatgct cggaggcttc ttttatacag 660
ccagaaagac accagcatga aggacatgcg caaagttctg agaacattac agcagatcaa 720
gaaatccagc tcaaacttga agcttcaaga tttcctggtg gacaatgaaa ccttctctgg 780
gttcctgtat cacaacctct ctctcccaaa gtctactgtg gacaagatgc tgagggctga 840
tgtcattctc cacaaggtat ttttgcaagg ctaccagtta catttgacaa gtctgtgcaa 900
tggatcaaaa tcagaagaga tgattcaact tggtgaccaa gaagtttctg agctttgtgg 960
cctaccaagg gagaaactgg ctgcagcaga gcgagtactt cgttccaaca tggacatcct 1020
gaagccaatc ctgagaacac taaactctac atctcccttc ccgagcaagg agctggctga 1080
agccacaaaa acattgctgc atagtcttgg gactctggcc caggagctgt tcagcatgag 1140
aagctggagt gacatgcgac aggaggtgat gtttctgacc aatgtgaaca gctccagctc 1200
ctccacccaa atctaccagg ctgtgtctcg tattgtctgc gggcatcccg agggaggggg 1260
gctgaagatc aagtctctca actggtatga ggacaacaac tacaaagccc tctttggagg 1320
caatggcact gaggaagatg ctgaaacctt ctatgacaac tctacaactc cttactgcaa 1380
tgatttgatg aagaatttgg agtctagtcc tctttcccgc attatctgga aagctctgaa 1440
gccgctgctc gttgggaaga tcctgtatac acctgacact ccagccacaa ggcaggtcat 1500
ggctgaggtg aacaagacct tccaggaact ggctgtgttc catgatctgg aaggcatgtg 1560
ggaggaactc agccccaaga tctggacctt catggagaac agccaagaaa tggaccttgt 1620
ccggatgctg ttggacagca gggacaatga ccacttttgg gaacagcagt tggatggctt 1680
agattggaca gcccaagaca tcgtggcgtt tttggccaag cacccagagg atgtccagtc 1740
cagtaatggt tctgtgtaca cctggagaga agctttcaac gagactaacc aggcaatccg 1800
gaccatatct cgcttcatgg agtgtgtcaa cctgaacaag ctagaaccca tagcaacaga 1860
agtctggctc atcaacaagt ccatggagct gctggatgag aggaagttct gggctggtat 1920
tgtgttcact ggaattactc caggcagcat tgagctgccc catcatgtca agtacaagat 1980
ccgaatggac attgacaatg tggagaggac aaataaaatc aaggatgggt actgggaccc 2040
tggtcctcga gctgacccct ttgaggacat gcggtacgtc tgggggggct tcgcctactt 2100
gcaggatgtg gtggagcagg caatcatcag ggtgctgacg ggcaccgaga agaaaactgg 2160
tgtctatatg caacagatgc cctatccctg ttacgttgat gacatctttc tgcgggtgat 2220
 
gagccggtca atgcccctct tcatgacgct ggcctggatt tactcagtgg ctgtgatcat 2280
caagggcatc gtgtatgaga aggaggcacg gctgaaagag accatgcgga tcatgggcct 2340
ggacaacagc atcctctggt ttagctggtt cattagtagc ctcattcctc ttcttgtgag 2400
cgctggcctg ctagtggtca tcctgaagtt aggaaacctg ctgccctaca gtgatcccag 2460
cgtggtgttt gtcttcctgt ccgtgtttgc tgtggtgaca atcctgcagt gcttcctgat 2520
tagcacactc ttctccagag ccaacctggc agcagcctgt gggggcatca tctacttcac 2580
gctgtacctg ccctacgtcc tgtgtgtggc atggcaggac tacgtgggct tcacactcaa 2640
gatcttcgct agcctgctgt ctcctgtggc ttttgggttt ggctgtgagt actttgccct 2700
ttttgaggag cagggcattg gagtgcagtg ggacaacctg tttgagagtc ctgtggagga 2760
agatggcttc aatctcacca cttcggtctc catgatgctg tttgacacct tcctctatgg 2820
ggtgatgacc tggtacattg aggctgtctt tccaggccag tacggaattc ccaggccctg 2880
gtattttcct tgcaccaagt cctactggtt tggcgaggaa agtgatgaga agagccaccc 2940
tggttccaac cagaagagaa tatcagaaat ctgcatggag gaggaaccca cccacttgaa 3000
gctgggcgtg tccattcaga acctggtaaa agtctaccga gatgggatga aggtggctgt 3060
cgatggcctg gcactgaatt tttatgaggg ccagatcacc tccttcctgg gccacaatgg 3120
agcggggaag acgaccacca tgtcaatcct gaccgggttg ttccccccga cctcgggcac 3180
cgcctacatc ctgggaaaag acattcgctc tgagatgagc accatccggc agaacctggg 3240
ggtctgtccc cagcataacg tgctgtttga catgctgact gtcgaagaac acatctggtt 3300
ctatgcccgc ttgaaagggc tctctgagaa gcacgtgaag gcggagatgg agcagatggc 3360
cctggatgtt ggtttgccat caagcaagct gaaaagcaaa acaagccagc tgtcaggtgg 3420
aatgcagaga aagctatctg tggccttggc ctttgtcggg ggatctaagg ttgtcattct 3480
ggatgaaccc acagctggtg tggaccctta ctcccgcagg ggaatatggg agctgctgct 3540
gaaataccga caaggccgca ccattattct ctctacacac cacatggatg aagcggacgt 3600
cctgggggac aggattgcca tcatctccca tgggaagctg tgctgtgtgg gctcctccct 3660
gtttctgaag aaccagctgg gaacaggcta ctacctgacc ttggtcaaga aagatgtgga 3720
atcctccctc agttcctgca gaaacagtag tagcactgtg tcatacctga aaaaggagga 3780
cagtgtttct cagagcagtt ctgatgctgg cctgggcagc gaccatgaga gtgacacgct 3840
gaccatcgat gtctctgcta tctccaacct catcaggaag catgtgtctg aagcccggct 3900
ggtggaagac atagggcatg agctgaccta tgtgctgcca tatgaagctg ctaaggaggg 3960
agcctttgtg gaactctttc atgagattga tgaccggctc tcagacctgg gcatttctag 4020
ttatggcatc tcagagacga ccctggaaga aatattcctc aaggtggccg aagagagtgg 4080
ggtggatgct gagacctcag atggtacctt gccagcaaga cgaaacaggc gggccttcgg 4140
ggacaagcag agctgtcttc gcccgttcac tgaagatgat gctgctgatc caaatgattc 4200
tgacatagac ccagaatcca gagagacaga cttgctcagt gggatggatg gcaaagggtc 4260
ctaccaggtg aaaggctgga aacttacaca gcaacagttt gtggcccttt tgtggaagag 4320
actgctaatt gccagacgga gtcggaaagg attttttgct cagattgtct tgccagctgt 4380
gtttgtctgc attgcccttg tgttcagcct gatcgtgcca ccctttggca agtaccccag 4440
 
cctggaactt cagccctgga tgtacaacga acagtacaca tttgtcagca atgatgctcc 4500
tgaggacacg ggaaccctgg aactcttaaa cgccctcacc aaagaccctg gcttcgggac 4560
ccgctgtatg gaaggaaacc caatcccaga cacgccctgc caggcagggg aggaagagtg 4620
gaccactgcc ccagttcccc agaccatcat ggacctcttc cagaatggga actggacaat 4680
gcagaaccct tcacctgcat gccagtgtag cagcgacaaa atcaagaaga tgctgcctgt 4740
gtgtccccca ggggcagggg ggctgcctcc tccacaaaga aaacaaaaca ctgcagatat 4800
ccttcaggac ctgacaggaa gaaacatttc ggattatctg gtgaagacgt atgtgcagat 4860
catagccaaa agcttaaaga acaagatctg ggtgaatgag tttaggtatg gcggcttttc 4920
cctgggtgtc agtaatactc aagcacttcc tccgagtcaa gaagttaatg atgccatcaa 4980
acaaatgaag aaacacctaa agctggccaa ggacagttct gcagatcgat ttctcaacag 5040
cttgggaaga tttatgacag gactggacac caaaaataat gtcaaggtgt ggttcaataa 5100
caagggctgg catgcaatca gctctttcct gaatgtcatc aacaatgcca ttctccgggc 5160
caacctgcaa aagggagaga accctagcca ttatggaatt actgctttca atcatcccct 5220
gaatctcacc aagcagcagc tctcagaggt ggctctgatg accacatcag tggatgtcct 5280
tgtgtccatc tgtgtcatct ttgcaatgtc cttcgtccca gccagctttg tcgtattcct 5340
gatccaggag cgggtcagca aagcaaaaca cctgcagttc atcagtggag tgaagcctgt 5400
catctactgg ctctctaatt ttgtctggga tatgtgcaat tacgttgtcc ctgccacact 5460
ggtcattatc atcttcatct gcttccagca gaagtcctat gtgtcctcca ccaatctgcc 5520
tgtgctagcc cttctacttt tgctgtatgg gtggtcaatc acacctctca tgtacccagc 5580
ctcctttgtg ttcaagatcc ccagcacagc ctatgtggtg ctcaccagcg tgaacctctt 5640
cattggcatt aatggcagcg tggccacctt tgtgctggag ctgttcaccg acaataagct 5700
gaataatatc aatgatatcc tgaagtccgt gttcttgatc ttcccacatt tttgcctggg 5760
acgagggctc atcgacatgg tgaaaaacca ggcaatggct gatgccctgg aaaggtttgg 5820
ggagaatcgc tttgtgtcac cattatcttg ggacttggtg ggacgaaacc tcttcgccat 5880
ggccgtggaa ggggtggtgt tcttcctcat tactgttctg atccagtaca gattcttcat 5940
caggcccaga cctgtaaatg caaagctatc tcctctgaat gatgaagatg aagatgtgag 6000
gcgggaaaga cagagaattc ttgatggtgg aggccagaat gacatcttag aaatcaagga 6060
gttgacgaag atatatagaa ggaagcggaa gcctgctgtt gacaggattt gcgtgggcat 6120
tcctcctggt gagtgctttg ggctcctggg agttaatggg gctggaaaat catcaacttt 6180
caagatgtta acaggagata ccactgttac cagaggagat gctttcctta acaaaaatag 6240
tatcttatca aacatccatg aagtacatca gaacatgggc tactgccctc agtttgatgc 6300
catcacagag ctgttgactg ggagagaaca cgtggagttc tttgcccttt tgagaggagt 6360
cccagagaaa gaagttggca aggttggtga gtgggcgatt cggaaactgg gcctcgtgaa 6420
gtatggagaa aaatatgctg gtaactatag tggaggcaac aaacgcaagc tctctacagc 6480
catggctttg atcggcgggc ctcctgtggt gtttctggat gaacccacca caggcatgga 6540
tcccaaagcc cggcggttct tgtggaattg tgccctaagt gttgtcaagg aggggagatc 6600
 
agtagtgctt acatctcata gtatggaaga atgtgaagct ctttgcacta ggatggcaat 6660
catggtcaat ggaaggttca ggtgccttgg cagtgtccag catctaaaaa ataggtttgg 6720
agatggttat acaatagttg tacgaatagc agggtccaac ccggacctga agcctgtcca 6780
ggatttcttt ggacttgcat ttcctggaag tgttctaaaa gagaaacacc ggaacatgct 6840
acaataccag cttccatctt cattatcttc tctggccagg atattcagca tcctctccca 6900
gagcaaaaag cgactccaca tagaagacta ctctgtttct cagacaacac ttgaccaagt 6960
atttgtgaac tttgccaagg accaaagtga tgatgaccac ttaaaagacc tctcattaca 7020
caaaaaccag acagtagtgg acgttgcagt tctcacatct tttctacagg atgagaaagt 7080
gaaagaaagc tatgtatgaa gaatcctgtt catacggggt ggctgaaagt aaagaggaac 7140
tagactttcc tttgcaccat gtgaagtgtt gtggagaaaa gagccagaag ttgatgtggg 7200
aagaagtaaa ctggatactg tactgatact attcaatgca atgcaattca atgcaatgaa 7260
aacaaaattc cattacaggg gcagtgcctt tgtagcctat gtcttgtatg gctctcaagt 7320
gaaagacttg aatttagttt tttacctata cctatgtgaa actctattat ggaacccaat 7380
ggacatatgg gtttgaactc acactttttt tttttttttt gttcctgtgt attctcattg 7440
gggttgcaac aataattcat caagtaatca tggccagcga ttattgatca aaatcaaaag 7500
gtaatgcaca tcctcattca ctaagccatg ccatgcccag gagactggtt tcccggtgac 7560
acatccattg ctggcaatga gtgtgccaga gttattagtg ccaagttttt cagaaagttt 7620
gaagcaccat ggtgtgtcat gctcactttt gtgaaagctg ctctgctcag agtctatcaa 7680
cattgaatat cagttgacag aatggtgcca tgcgtggcta acatcctgct ttgattccct 7740
ctgataagct gttctggtgg cagtaacatg caacaaaaat gtgggtgtct ccaggcacgg 7800
gaaacttggt tccattgtta tattgtccta tgcttcgagc catgggtcta cagggtcatc 7860
cttatgagac tcttaaatat acttagatcc tggtaagagg caaagaatca acagccaaac 7920
tgctggggct gcaagctgct gaagccaggg catgggatta aagagattgt gcgttcaaac 7980
ctagggaagc ctgtgcccat ttgtcctgac tgtctgctaa catggtacac tgcatctcaa 8040
gatgtttatc tgacacaagt gtattatttc tggcttttg aattaatcta gaaaatgaaa 8100
agatggagtt gtattttgac aaaaatgttt gtacttttta atgttatttg gaattttaag 8160
ttctatcagt gacttctgaa tccttagaat ggcctctttg tagaaccctg tggtatagag 8220
gagtatggcc actgccccac tatttttatt ttcttatgta agtttgcata tcagtcatga 8280
ctagtgccta gaaagcaatg tgatggtcag gatctcatga cattatattt gagtttcttt 8340
cagatcattt aggatactct taatctcact tcatcaatca aatatttttt gagtgtatgc 8400
tgtagctgaa agagtatgta cgtacgtata agactagaga gatattaagt ctcagtacac 8460
ttcctgtgcc atgttattca gctcactggt ttacaaatat aggttgtctt gtggttgtag 8520
gagcccactg taacaatact gggcagcctt tttttttttt tttttaattg caacaatgca 8580
aaagccaaga aagtataagg gtcacaagtc taaacaatga attcttcaac agggaaaaca 8640
gctagcttga aaacttgctg aaaaacacaa cttgtgttta tggcatttag taccttcaaa 8700
taattggctt tgcagatatt ggatacccca ttaaatctga cagtctcaaa tttttcatct 8760
cttcaatcac tagtcaagaa aaatataaaa acaacaaata cttccatatg gagcattttt 8820
 
cagagttttc taacccagtc ttatttttct agtcagtaaa catttgtaaa aatactgttt 8880
cactaatact tactgttaac tgtcttgaga gaaaagaaaa atatgagaga actattgttt 8940
ggggaagttc aagtgatctt tcaatatcat tactaacttc ttccactttt tccagaattt 9000
gaatattaac gctaaaggtg taagacttca gatttcaaat taatctttct atatttttta 9060
aatttacaga atattatata acccactgct gaaaaagaaa aaaatgattg ttttagaagt 9120
taaagtcaat attgatttta aatataagta atgaaggcat atttccaata actagtgata 9180
tggcatcgtt gcattttaca gtatcttcaa aaatacagaa tttatagaat aatttctcct 9240
catttaatat ttttcaaaat caaagttatg gtttcctcat tttactaaaa tcgtattcta 9300
attcttcatt atagtaaatc tatgagcaac tccttacttc ggttcctctg atttcaaggc 9360
catattttaa aaaatcaaaa ggcactgtga actattttga agaaaacaca acattttaat 9420
acagattgaa aggacctctt ctgaagctag aaacaatcta tagttataca tcttcattaa 9480
tactgtgtta ccttttaaaa tagtaatttt ttacattttc ctgtgtaaac ctaattgtgg 9540
tagaaatttt taccaactct atactcaatc aagcaaaatt tctgtatatt ccctgtggaa 9600
tgtacctatg tgagtttcag aaattctcaa aatacgtgtt caaaaatttc tgcttttgca 9660
tctttgggac acctcagaaa acttattaac aactgtgaat atgagaaata cagaagaaaa 9720
taataagccc tctatacata aatgcccagc acaattcatt gttaaaaaac aaccaaacct 9780
cacactactg tatttcatta tctgtactga aagcaaatgc tttgtgacta ttaaatgttg 9840
cacatcattc attcactgta tagtaatcat tgactaaagc catttgtctg tgttttcttc 9900
ttgtggttgt atatatcagg taaaatattt tccaaagagc catgtgtcat gtaatactga 9960
accactttga tattgagaca ttaatttgta cccttgttat tatctactag taataatgta 10020
atactgtaga aatattgctc taattctttt caaaattgtt gcatccccct tagaatgttt 10080
ctatttccat aaggatttag gtatgctatt atcccttctt ataccctaag atgaagctgt 10140
ttttgtgctc tttgttcatc attggccctc attccaagca ctttacgctg tctgtaatgg 10200
gatctatttt tgcactggaa tatctgagaa ttgcaaaact agacaaaagt ttcacaacag 10260
atttctaagt taaatcattt tcattaaaag gaaaaaagaa aaaaaatttt gtatgtcaat 10320
aactttatat gaagtattaa aatgcatatt tctatgttgt aatataatga gtcacaaaat 10380
aaagctgtga cagttctgtt ggtctacaga aa 10412
 <210> 4
   <211> 3537
   <212> DNA
   <213> Homo sapiens
<400> 4 
ggcccctcga gcctcgaacc ggaacctcca aatccgagac gctctgctta tgaggacctc 60
gaaatatgcc ggccagtgaa aaaatcttgt ggctttgagg gcttttggtt ggccaggggc 120
agtaaaaatc tcggagagct gacaccaagt cctcccctgc cacgtagcag tggtaaagtc 180
cgaagctcaa attccgagaa ttgagctctg ttgattctta gaactggggt tcttagaagt 240
ggtgatgcaa gaagtttcta ggaaaggccg gacaccaggt tttgagcaaa attttggact 300
gtgaagcaag gcattggtga agacaaaatg gcctcgccgg ctgacagctg tatccagttc 360
 
acccgccatg ccagtgatgt tcttctcaac cttaatcgtc tccggagtcg agacatcttg 420
actgatgttg tcattgttgt gagccgtgag cagtttagag cccataaaac ggtcctcatg 480
gcctgcagtg gcctgttcta tagcatcttt acagaccagt tgaaatgcaa ccttagtgtg 540
atcaatctag atcctgagat caaccctgag ggattctgca tcctcctgga cttcatgtac 600
acatctcggc tcaatttgcg ggagggcaac atcatggctg tgatggccac ggctatgtac 660
ctgcagatgg agcatgttgt ggacacttgc cggaagttta ttaaggccag tgaagcagag 720
atggtttctg ccatcaagcc tcctcgtgaa gagttcctca acagccggat gctgatgccc 780
caagacatca tggcctatcg gggtcgtgag gtggtggaga acaacctgcc actgaggagc 840
gcccctgggt gtgagagcag agcctttgcc cccagcctgt acagtggcct gtccacaccg 900
ccagcctctt attccatgta cagccacctc cctgtcagca gcctcctctt ctccgatgag 960
gagtttcggg atgtccggat gcctgtggcc aaccccttcc ccaaggagcg ggcactccca 1020
tgtgatagtg ccaggccagt ccctggtgag tacagccggc cgactttgga ggtgtccccc 1080
aatgtgtgcc acagcaatat ctattcaccc aaggaaacaa tcccagaaga ggcacgaagt 1140
gatatgcact acagtgtggc tgagggcctc aaacctgctg ccccctcagc ccgaaatgcc 1200
ccctacttcc cttgtgacaa ggccagcaaa gaagaagaga gaccctcctc ggaagatgag 1260
attgccctgc atttcgagcc ccccaatgca cccctgaacc ggaagggtct ggttagtcca 1320
cagagccccc agaaatctga ctgccagccc aactcgccca cagagtcctg cagcagtaag 1380
aatgcctgca tcctccaggc ttctggctcc cctccagcca agagccccac tgaccccaaa 1440
gcctgcaact ggaagaaata caagttcatc gtgctcaaca gcctcaacca gaatgccaaa 1500
ccagaggggc ctgagcaggc tgagctgggc cgcctttccc cacgagccta cacggcccca 1560
cctgcctgcc agccacccat ggagcctgag aaccttgacc tccagtcccc aaccaagctg 1620
agtgccagcg gggaggactc caccatccca caagccagcc ggctcaataa catcgttaac 1680
aggtccatga cgggctctcc ccgcagcagc agcgagagcc actcaccact ctacatgcac 1740
cccccgaagt gcacgtcctg cggctctcag tccccacagc atgcagagat gtgcctccac 1800
accgctggcc ccacgttccc tgaggagatg ggagagaccc agtctgagta ctcagattct 1860
agctgtgaga acggggcctt cttctgcaat gagtgtgact gccgcttctc tgaggaggcc 1920
tcactcaaga ggcacacgct gcagacccac agtgacaaac cctacaagtg tgaccgctgc 1980
caggcctcct tccgctacaa gggcaacctc gccagccaca agaccgtcca taccggtgag 2040
aaaccctatc gttgcaacat ctgtggggcc cagttcaacc ggccagccaa cctgaaaacc 2100
cacactcgaa ttcactctgg agagaagccc tacaaatgcg aaacctgcgg agccagattt 2160
gtacaggtgg cccacctccg tgcccatgtg cttatccaca ctggtgagaa gccctatccc 2220
tgtgaaatct gtggcacccg tttccggcac cttcagactc tgaagagcca cctgcgaatc 2280
cacacaggag agaaacctta ccattgtgag aagtgtaacc tgcatttccg tcacaaaagc 2340
cagctgcgac ttcacttgcg ccagaagcat ggcgccatca ccaacaccaa ggtgcaatac 2400
cgcgtgtcag ccactgacct gcctccggag ctccccaaag cctgctgaag catggagtgt 2460
tgatgctttc gtctccagcc ccttctcaga atctacccaa aggatactgt aacactttac 2520
 
aatgttcatc ccatgatgta gtgcctcttt catccactag tgcaaatcat agctgggggt 2580

tgggggtggt gggggtcggg gcctggggga ctgggagccg cagcagctcc ccctccccca 2640

ctgccataaa acattaagaa aatcatattg cttcttctcc tatgtgtaag gtgaaccatg 2700

tcagcaaaaa gcaaaatcat tttatatgtc aaagcagggg agtatgcaaa agttctgact 2760

tgactttagt ctgcaaaatg aggaatgtat atgttttgtg ggaacagatg tttcttttgt 2820

atgtaaatgt gcattctttt aaaagacaag acttcagtat gttgtcaaag agagggcttt 2880

aattttttta accaaaggtg aaggaatata tggcagagtt gtaaatatat aaatatatat 2940

atatataaaa taaatatata taaacctaac aaagatatat taaaaatata aaactgcgtt 3000

aaaggctcga ttttgtatct gcaggcagac acggatctga gaatctttat tgagaaagag 3060

cacttaagag aatattttaa gtattgcatc tgtataagta agaaaatatt ttgtctaaaa 3120

tgcctcagtg tatttgtatt tttttgcaag tgaaggttta caatttacaa agtgtgtatt 3180

aaaaaaaaca aaaagaacaa aaaaatctgc agaaggaaaa atgtgtaatt ttgttctagt 3240

tttcagtttg tatatacccg tacaacgtgt cctcacggtg ccttttttca cggaagtttt 3300

caatgatggg cgagcgtgca ccatcccttt ttgaagtgta ggcagacaca gggacttgaa 3360

gttgttacta actaaactct ctttgggaat gtttgtctca tcccattctg cgtcatgctt 3420

gtgttataac tactccggag acagggtttg gctgtgtcta aactgcatta ccgcgttgta 3480

aaatatagct gtacaaatat aagaataaaa tgttgaaaag tcaaactgga aaaaaaa 3537
 <210> 5
   <211> 3464
   <212> DNA
   <213> Homo sapi ens
<400> 5 
cagccgtgct cgaagcgttc ctggagccca agctctcctc cacaggtgaa gacagggcca 60
gcaggagaca ccatggggca cctctcagcc ccacttcaca gagtgcgtgt accctggcag 120
gggcttctgc tcacagcctc acttctaacc ttctggaacc cgcccaccac tgcccagctc 180
actactgaat ccatgccatt caatgttgca gaggggaagg aggttcttct ccttgtccac 240
aatctgcccc agcaactttt tggctacagc tggtacaaag gggaaagagt ggatggcaac 300
cgtcaaattg taggatatgc aataggaact caacaagcta ccccagggcc cgcaaacagc 360
ggtcgagaga caatataccc caatgcatcc ctgctgatcc agaacgtcac ccagaatgac 420
acaggattct acaccctaca agtcataaag tcagatcttg tgaatgaaga agcaactgga 480
cagttccatg tatacccgga gctgcccaag ccctccatct ccagcaacaa ctccaaccct 540
gtggaggaca aggatgctgt ggccttcacc tgtgaacctg agactcagga cacaacctac 600
ctgtggtgga taaacaatca gagcctcccg gtcagtccca ggctgcagct gtccaatggc 660
aacaggaccc tcactctact cagtgtcaca aggaatgaca caggacccta tgagtgtgaa 720
atacagaacc cagtgagtgc gaaccgcagt gacccagtca ccttgaatgt cacctatggc 780
ccggacaccc ccaccatttc cccttcagac acctattacc gtccaggggc aaacctcagc 840
ctctcctgct atgcagcctc taacccacct gcacagtact cctggcttat caatggaaca 900
ttccagcaaa gcacacaaga gctctttatc cctaacatca ctgtgaataa tagtggatcc 960
 
tatacctgcc acgccaataa ctcagtcact ggctgcaaca ggaccacagt caagacgatc 1020
atagtcactg agctaagtcc agtagtagca aagccccaaa tcaaagccag caagaccaca 1080
gtcacaggag ataaggactc tgtgaacctg acctgctcca caaatgacac tggaatctcc 1140
atccgttggt tcttcaaaaa ccagagtctc ccgtcctcgg agaggatgaa gctgtcccag 1200
ggcaacacca ccctcagcat aaaccctgtc aagagggagg atgctgggac gtattggtgt 1260
gaggtcttca acccaatcag taagaaccaa agcgacccca tcatgctgaa cgtaaactat 1320
aatgctctac cacaagaaaa tggcctctca cctggggcca ttgctggcat tgtgattgga 1380
gtagtggccc tggttgctct gatagcagta gccctggcat gttttctgca tttcgggaag 1440
accggcaggg caagcgacca gcgtgatctc acagagcaca aaccctcagt ctccaaccac 1500
actcaggacc actccaatga cccacctaac aagatgaatg aagttactta ttctaccctg 1560
aactttgaag cccagcaacc cacacaacca acttcagcct ccccatccct aacagccaca 1620
gaaataattt attcagaagt aaaaaagcag taatgaaacc tgtcctgctc actgcagtgc 1680
tgatgtattt caagtctctc accctcatca ctaggagatt cctttcccct ctagggtaga 1740
ggggtgggga cagaaacaac tttctcctac tcttccttcc taataggcat ctccaggctg 1800
cctggtcact gcccctctct cagtgtcaat agatgaaagt acattgggag tctgtaggaa 1860
acccaacctt cttgtcattg aaatttggca aagctgactt tgggaaagag ggaccagaac 1920
ttcccctccc ttcccctttt cccaacctgg acttgtttta aacttgcctg ttcagagcac 1980
tcattccttc ccacccccag tcctgtccta tcactctaat tcggatttgc catagccttg 2040
aggttatgtc cttttccatt aagtacatgt gccaggaaac agcgagagag agaaagtaaa 2100
cggcagtaat gcttctccta tttctccaaa gccttgtgtg aactagcaaa gagaagaaaa 2160
ccaaatatat aaccaatagt gaaatgccac aggtttgtcc actgtcaggg ttgtctacct 2220
gtaggatcag ggtctaagca ccttggtgct tagctagaat accacctaat ccttctggca 2280
agcctgtctt cagagaaccc actagaagca actaggaaaa atcacttgcc aaaatccaag 2340
gcaattcctg atggaaaatg caaaagcaca tatatgtttt aatatcttta tgggctctgt 2400
tcaaggcagt gctgagaggg aggggttata gcttcaggag ggaaccagct tctgataaac 2460
acaatctgct aggaacttgg gaaaggaatc agagagctgc ccttcagcga ttatttaaat 2520
tattgttaaa gaatacacaa tttggggtat tgggattttt ctccttttct ctgagacatt 2580
ccaccatttt aatttttgta actgcttatt tatgtgaaaa gggttatttt tacttagctt 2640
agctatgtca gccaatccga ttgccttagg tgaaagaaac caccgaaatc cctcaggtcc 2700
cttggtcagg agcctctcaa gatttttttt gtcagaggct ccaaatagaa aataagaaaa 2760
ggttttcttc attcatggct agagctagat ttaactcagt ttctaggcac ctcagaccaa 2820
tcatcaacta ccattctatt ccatgtttgc acctgtgcat tttctgtttg cccccattca 2880
ctttgtcagg aaaccttggc ctctgctaag gtgtatttgg tccttgagaa gtgggagcac 2940
cctacaggga cactatcact catgctggtg gcattgttta cagctagaaa gctgcactgg 3000
tgctaatgcc ccttgggaaa tggggctgtg aggaggagga ttataactta ggcctagcct 3060
cttttaacag cctctgaaat ttatcttttc ttctatgggg cttataaatg tatcttataa 3120
taaaaaggaa ggacaggagg aagacaggca aatgtacttc tcacccagtc ttctacacag 3180
 
atggaatctc tttggggcta agagaaaggt tttattctat attgcttacc tgatctcatg 3240
ttaggcctaa gaggctttct ccaggaggat tagcttggag ttctctatac tcaggtacct 3300
ctttcagggt tttctaaccc tgacacggac tgtgcatact ttccctcatc catgctgtgc 3360
tgtgttattt aatttttcct ggctaagatc atgtctgaat tatgtatgaa aattattcta 3420
tgtttttata ataaaaataa tatatcagac atcgaaaaaa aaaa 3464
 <210> 6
   <211> 2183
   <212> DNA
   <213> Homo sapiens
<400> 6 
ggcagccgag gagaccccgc gcagtgctgc caacgccccg gtggagaagc tgaggtcatc 60
atcagatttg aaatatttaa agtggataca aaattatttc agcaatgcag acaattaagt 120
gtgttgttgt gggcgatggt gctgttggta aaacatgtct cctgatatcc tacacaacaa 180
acaaatttcc atcggaatat gtaccgactg tttttgacaa ctatgcagtc acagttatga 240
ttggtggaga accatatact cttggacttt ttgatactgc agggcaagag gattatgaca 300
gattacgacc gctgagttat ccacaaacag atgtatttct agtctgtttt tcagtggtct 360
ctccatcttc atttgaaaac gtgaaagaaa agtgggtgcc tgagataact caccactgtc 420
caaagactcc tttcttgctt gttgggactc aaattgatct cagagatgac ccctctacta 480
ttgagaaact tgccaagaac aaacagaagc ctatcactcc agagactgct gaaaagctgg 540
cccgtgacct gaaggctgtc aagtatgtgg agtgttctgc acttacacag aaaggcctaa 600
agaatgtatt tgacgaagca atattggctg ccctggagcc tccagaaccg aagaagagcc 660
gcaggtgtgt gctgctatga acatctctcc agagcccttt ctgcacagct ggtgtcggca 720
tcatactaaa agcaatgttt aaatcaaact aaagattaaa aattaaaatt cgtttttgca 780
ataatgacaa atgccctgca cctacccaca tgcactcgtg tgagacaagg cccataggta 840
tggccccccc cttccccctc ccagtactag ttaattttga gtaattgtat tgtcagaaaa 900
gtgattagta ctattttttt ttgttgtttc aaaaaaaaaa tttttgtgtg tctgtttttt 960
tttttttttt tttttgttgt ttaaaaggaa ggcatgcttg tggatgactc tgtaacagac 1020
taattggaat tgttgaagct gctccctggt tccactctgg agagtaatct gggacatctt 1080
agtgttttgt tttgtttttt tccctcctct tttttttggg ggggagtgtg tgtggggttt 1140
gttttttagt cttgtttttt taattcatta accagtggtt agcccttaag gggaggagga 1200
cggattgatt ccacattcca cttcctagat ctagtttaga aaacatgttc cccatctggt 1260
gctcttagga aggagtatag taaatgcctc atttaataac atactccttt ttgaaagttg 1320
ccttttctct ccacccttga gtagatccag tatttgatga aactcatgaa agtgggtgga 1380
gcccatcttg cccctcctct tttctaggac gcactatatg tgactgtgac tttcaaggac 1440
atttgtttgc catttgctga tttttttggg aagttaattt ctaacttctt tcactgataa 1500
atgaagaaaa gtattgcacc tttgaaatgc accaaatgaa ttgagtttgt aattaaaaaa 1560
atttttttcc ctttcagtca ttgtcttata tgcttagcat agatttgcag ctcagtagta 1620
tatggtgttc ctagaatgca gctgaagacc tgttatgtag aggaaatacg aggggtggtg 1680
 
ctagaagaca gacatctgtg gaatgattca catcctctca agttaggagg atggaggcct 1740
gcttcattaa gaagctgggg gtagggtggg ggtggggaga acacttaaca acatggggac 1800
cagtcagggg aatcccctta tttctgtttt gcatatgagg aaccctagag cagccaggtg 1860
aggctctcta gtttaataaa aatcatggaa agactcttaa tgcagactct tcttaagtgt 1920
taatagggat tttttcagct tattttggtt gcagtttcca atttttaaaa atgttgaggt 1980
aatctttccc accttcccaa acctaattct tgtagatgca ttagtgttga accaatgctt 2040
tctcatgtct caattctttg tatatgcatt cttttcagat gtattaaaca aacaaaaacc 2100
cttcaaaaaa aaaaaaaaaa aaaaaaaaaa aaaaaaaaaa aaaaaaaaaa aaaaaaaaaa 2160
aaaaaaaaaa aaaaaaaaaa aaa 2183
 <210> 7
   <211> 641
   <212> DNA
   <213> Homo sapiens
<400> 7 
atttaaattc tgcagctcag agattcacac agaagtctgg acacaattca gaagagccac 60
ccagaaggag acaacaatgt ccctgctacc cgtgccatac acagaggctg cctctttgtc 120
tactggttct actgtgacaa tcaaagggcg accacttgcc tgtttcttga atgaaccata 180
tctgcaggtg gatttccaca ctgagatgaa ggaggaatca gacattgtct tccatttcca 240
agtgtgcttt ggtcgtcgtg tggtcatgaa cagccgtgag tatggggcct ggaagcagca 300
ggtggaatcc aagaatatgc cctttcagga tggccaagaa tttgaactga gcatctcagt 360
gctgccagat aagtaccagg taatggtcaa tggccaatcc tcttacacct ttgaccatag 420
aatcaagcct gaggctgtga agatggtgca agtgtggaga gatatctccc tgaccaaatt 480
taatgtcagc tatttaaaga gataaccaga cttcatgttg ccaaggaatc cctgtctcta 540
cgtgaacttg ggattccaaa gccagctaac agcatgatct tttctcactt caatccttac 600
tcctgctcat taaaacttaa tcaaacttca aaaaaaaaaa a 641
 <210> 8
   <211> 2157
   <212> DNA
   <213> Homo sapiens
<400> 8 
ggccaggatc atgtccacca ccacatgcca agtggtggcg ttcctcctgt ccatcctggg 60
gctggccggc tgcatcgcgg ccaccgggat ggacatgtgg agcacccagg acctgtacga 120
caaccccgtc acctccgtgt tccagtacga agggctctgg aggagctgcg tgaggcagag 180
ttcaggcttc accgaatgca ggccctattt caccatcctg ggacttccag ccatgctgca 240
ggcagtgcga gccctgatga tcgtaggcat cgtcctgggt gccattggcc tcctggtatc 300
catctttgcc ctgaaatgca tccgcattgg cagcatggag gactctgcca aagccaacat 360
gacactgacc tccgggatca tgttcattgt ctcaggtctt tgtgcaattg ctggagtgtc 420
tgtgtttgcc aacatgctgg tgactaactt ctggatgtcc acagctaaca tgtacaccgg 480
catgggtggg atggtgcaga ctgttcagac caggtacaca tttggtgcgg ctctgttcgt 540
 
gggctgggtc gctggaggcc tcacactaat tgggggtgtg atgatgtgca tcgcctgccg 600
gggcctggca ccagaagaaa ccaactacaa agccgtttct tatcatgcct caggccacag 660
tgttgcctac aagcctggag gcttcaaggc cagcactggc tttgggtcca acaccaaaaa 720
caagaagata tacgatggag gtgcccgcac agaggacgag gtacaatctt atccttccaa 780
gcacgactat gtgtaatgct ctaagacctc tcagcacggg cggaagaaac tcccggagag 840
ctcacccaaa aaacaaggag atcccatcta gatttcttct tgcttttgac tcacagctgg 900
aagttagaaa agcctcgatt tcatctttgg agaggccaaa tggtcttagc ctcagtctct 960
gtctctaaat attccaccat aaaacagctg agttatttat gaattagagg ctatagctca 1020
cattttcaat cctctatttc tttttttaaa tataactttc tactctgatg agagaatgtg 1080
gttttaatct ctctctcaca ttttgatgat ttagacagac tccccctctt cctcctagtc 1140
aataaaccca ttgatgatct atttcccagc ttatccccaa gaaaactttt gaaaggaaag 1200
agtagaccca aagatgttat tttctgctgt ttgaattttg tctccccacc cccaacttgg 1260
ctagtaataa acacttactg aagaagaagc aataagagaa agatatttgt aatctctcca 1320
gcccatgatc tcggttttct tacactgtga tcttaaaagt taccaaacca aagtcatttt 1380
cagtttgagg caaccaaacc tttctactgc tgttgacatc ttcttattac agcaacacca 1440
ttctaggagt ttcctgagct ctccactgga gtcctctttc tgtcgcgggt cagaaattgt 1500
ccctagatga atgagaaaat tatttttttt aatttaagtc ctaaatatag ttaaaataaa 1560
taatgtttta gtaaaatgat acactatctc tgtgaaatag cctcacccct acatgtggat 1620
agaaggaaat gaaaaaataa ttgctttgac attgtctata tggtactttg taaagtcatg 1680
cttaagtaca aattccatga aaagctcact gatcctaatt ctttcccttt gaggtctcta 1740
tggctctgat tgtacatgat agtaagtgta agccatgtaa aaagtaaata atgtctgggc 1800
acagtggctc acgcctgtaa tcctagcact ttgggaggct gaggaggaag gatcacttga 1860
gcccagaagt tcgagactag cctgggcaac atggagaagc cctgtctcta caaaatacag 1920
agagaaaaaa tcagccagtc atggtggcat acacctgtag tcccagcatt ccgggaggct 1980
gaggtgggag gatcacttga gcccagggag gttggggctg cagtgagcca tgatcacacc 2040
actgcactcc agccaggtga catagcgaga tcctgtctaa aaaaataaaa aataaataat 2100
ggaacacagc aagtcctagg aagtaggtta aaactaattc tttaaaaaaa aaaaaaa 2157
 <210> 9
   <211> 7984
   <212> DNA
   <213> Homo sapiens
<400> 9 
cggcccggcg gtaggcggtg gcgactctgc ccgctcccgt ttcggcgcgg tgaccgagcg 60
cccgggaggc tcgaggaccg catcgtgtgc cgttgcgcca agcccggtcc tgcgccgcca 120
tggccccagt gcagctggag aaccaccagc tggtcccgcc cggaggcggc ggcgggggca 180
gcggcggacc cccgtcagcc ccagcccctc ctcccccggg agccgccgtg gcggcggccg 240
ctgcagctgc ggctagcccg ggctaccggc tgagcaccct cattgaattt ctgctgcacc 300
gggcctactc ggagcttatg gtgttgacgg acctactgcc aaggaaatct gatgtggaaa 360
 
ggaaaataga aatagtgcag tttgctagcc ggacacgcca actcttcgtt cgattattag 420
ctttagtgaa atgggctaat aatgctggca aagtggaaaa atgtgcgatg atttcaagct 480
ttttagatca gcaagccatc ctgtttgtgg acactgctga tcgcctggcc tcgttagcta 540
gagatgctct ggtccatgca cgcctgccta gttttgccat cccatatgcc attgatgtac 600
taactactgg atcttaccca cggctgccaa cctgcattag ggataaaatt attcctccag 660
acccaattac caaaattgaa aaacaagcca cactccatca gctgaatcag attcttagac 720
atcggcttgt aaccacagat cttcctcctc agttagcaaa tcttacagtt gcaaatggcc 780
gggtgaagtt tcgtgttgaa ggagaatttg aagccacctt gactgtgatg ggagatgacc 840
ctgatgttcc atggcgtctt ctcaagctag aaattctagt tgaggataag gaaacaggag 900
atgggcgagc tttggttcat agcatgcaaa tcagcttcat ccatcaactg gtgcagtcta 960
ggctctttgc tgatgagaaa cctcttcagg atatgtacaa ctgcctacat tctttctgtt 1020
tatcacttca gttagaagtg ttacattccc aaactctaat gttaatccga gaacggtggg 1080
gagaccttgt gcaggtggaa aggtatcatg ctggaaagtg cctctccctt tcagtttgga 1140
atcaacaggt tcttgggaga aaaactggaa cagcatctgt tcacaaagtt acaattaaaa 1200
ttgatgagaa tgatgtctcc aagcctttac agatttttca cgatcctcct ttgccagctt 1260
ctgattccaa attagtagaa agagccatga agatcgacca cttatcaata gaaaaactcc 1320
tgattgacag tgtccatgca agagctcatc agaagctcca agaactgaag gccattctta 1380
gaggcttcaa tgccaatgaa aactcttcca tagagactgc actcccagct cttgttgttc 1440
ccatcttgga gccctgtggt aattcagagt gtctgcacat ttttgtagat ttacattctg 1500
gaatgtttca attgatgctt tatggacttg accaggccac tctggatgac atggagaagt 1560
ctgtgaatga tgatatgaaa cgaatcatac cctggattca gcaacttaag ttttggcttg 1620
gacaacagcg ttgcaagcag tctataaaac atctgcctac gataagcagt gaaacattgc 1680
agctttccaa ttactcaact catcctattg gaaacctttc taagaataaa ctgttcatta 1740
aacttacccg ccttccacaa tactacattg ttgtggagat gttggaggtt cccaataaac 1800
ccacacaact gtcgtacaag tactacttta tgtctgtgaa tgctgcagat cgtgaagaca 1860
gccctgcaat ggcattgctg ctgcagcagt tcaaggaaaa cattcaggac ttggtttttc 1920
gtacaaaaac cgggaaacag accagaacca atgccaagcg caagttgtct gatgatccat 1980
gtccagtaga atccaagaaa acaaaacgag caggagaaat gtgtgccttc aataaagttt 2040
tagcccactt cgtcgctatg tgtgatacaa atatgccatt tgtaggactt cggttggagt 2100
tgtccaatct ggagattcca catcaaggag tgcaagtgga aggtgatggc ttcagccatg 2160
caattcgctt attaaaaatt cctccctgta agggtataac tgaggaaacc caaaaggctc 2220
tggaccgctc tcttcttgat tgcactttcc gattacaagg tagaaataac cgcacttggg 2280
tagcagagtt agtgtttgca aattgtccac ttaatggcac ttctactagg gagcaaggac 2340
catcccggca cgtttacctg acatatgaaa atctgttgtc tgagcctgtt ggtggtagaa 2400
aggtggttga aatgtttctt aatgactgga atagcattgc acgattatat gagtgtgtgt 2460
tggaatttgc acgttctcta ccagacatac ctgctcatct aaatattttc tcagaagttc 2520
gtgtttataa ttaccgaaaa cttatcttgt gttatggaac caccaaggga agctcaatta 2580
 
gtatccaatg gaattcgatc catcaaaaat tccacatttc tttgggaact gttggcccaa 2640
actcaggttg cagtaactgt cacaatacca ttctccatca gcttcaagaa atgttcaaca 2700
aaacaccaaa tgtggttcag ttattacagg tactgtttga tactcaggct ccattaaatg 2760
ccatcaacaa actccccact gtgccgatgt tgggcttgac ccagagaacc aatactgcct 2820
accagtgctt ctccattctg ccacagtcgt ccacccacat cagactggcc ttcaggaaca 2880
tgtattgcat tgatatatac tgccggagtc gaggtgttgt ggcaatacgg gatggtgcct 2940
atagtctttt tgataacagc aaactagttg aaggtttcta tcctgcacca ggactaaaga 3000
cgttcctgaa tatgtttgtt gacagcaatc aggatgctcg aagaaggtct gtaaatgagg 3060
acgataatcc cccttctcct ataggaggag atatgatgga ttctttaata tcgcagctcc 3120
agccaccacc ccagcaacag ccatttccaa agcagccagg aacatcaggt gcttatcctc 3180
ttacttcacc ccctacatct tatcatagca cagtcaatca gtctccctca atgatgcaca 3240
cacagtctcc aggaaatctg catgctgcca gctcccccag tggggctttg agagccccat 3300
caccagcgtc atttgttcca actcctcccc catcctcgca tggaatctca ataggaccag 3360
gggccagttt tgctagtcca catggaactc ttgaccctag ttccccatac actatggtgt 3420
caccaagtgg acgagcaggg aactggccag gatctcctca agtgtctggc ccctcaccag 3480
cagcacgcat gcctggaatg tcaccagcca acccctcact acattctccg gttccagatg 3540
cttctcattc ccctcgagct ggaacaagtt ctcaaacaat gccaacaaac atgcctccac 3600
ctcgtaaact acctcagcgc tcttgggcgg catccatacc taccatcctc actcacagtg 3660
ccttgaacat tttactgctg ccctctccaa ctccaggcct tgtgcccggc ctggcaggta 3720
gttacctttg ttctccactt gagagattcc ttggatcagt catcatgaga cgacatcttc 3780
aaagaattat tcaacaagaa acgctgcagc tgataaattc taatgaaccc ggagtgatca 3840
tgtttaagac tgatgcactg aaatgcagag tagctcttag tcccaaaacc aaccaaacgc 3900
ttcagctaaa agtgacacct gaaaatgcag gacagtggaa acctgatgaa cttcaagttt 3960
tggagaaatt ctttgaaaca agagttgcag gaccaccatt taaagctaat acgttaatag 4020
ccttcaccaa gctattagga gctcctacac acatcctcag ggattgtgtg cacattatga 4080
agctggagct gttccctgac caagcaacac agctaaaatg gaatgttcag ttttgcctga 4140
cgatccctcc cagcgcgccg ccgattgcac ctcctgggac gcctgctgtg gtgctgaaat 4200
ccaaaatgct atttttcttt caactaactc agaaaacatc ggtccctccc caagaacctg 4260
ttagtattat agttccaatc atttatgaca tggcttcagg tacaacccag caggcagaca 4320
ttcccagaca gcagaactct tctgttgctg ctcccatgat ggtcagcaac attctgaaga 4380
ggtttgcaga gatgaatcca ccacgacaag gtgaatgcac aatatttgca gctgttcgtg 4440
atttaatggc taatcttaca ctgccccctg gtgggcgtcc atagacacta ttgtttttaa 4500
accaggaagg ctgacagatg agacaacaaa aaaaatctga attcagcctt cagtttaaaa 4560
aaggaaaagg actttttttt taaactttaa gagctaaata ttctaaactg gcaaaaattt 4620
tcagggtgca cttctttcat caaaatgatc atccatcttt tgtataatga acttgtatag 4680
tgtgtttaaa tgggacacat ttcaaaggaa ataaatcagt gtccgtttgc cagtaataaa 4740
 
tttaatattc tgttttatac tataaagtta tgaaaatgcc aaacttgttt atttaattgt 4800
tttcttatgt tttgggtgta atagtccttt tttggttttt gttttgtttt ttaaggcagg 4860
tctgtcattt ttgaatactg taaaactgtg ataaacttta tattgaagct gtattttaat 4920
atgaccgctt tgaatcctta catgaaatgt tctgggagtt gttataaaca caccccaagg 4980
aagcaatatt taataaaact aggtcaaaaa caaaacaaaa cagtgacact cacttgtgtg 5040
tatataaact ctagagacat cttaggccct cccttcatct ttaacctggt ggatggagcc 5100
agttattaat ttataataat acatgtctga aatttgacca aaaacatctt ctttatgtca 5160
aggttaatat attttcttca gacattccta acttgactag tggttaacat ttagcacctt 5220
ggttgtcttt caatatatag gatttgggtg aaaaataagg aacttagtct ttgagaaggg 5280
acagtatgtg gttcgtattg atcaaggatc ccaaataatg caatcattct catctgttga 5340
ggatgtggga acttttctgt caggaaaatt tagtttacag aatacttctg catacaacat 5400
taattaggaa acaaagaact ttaatgctgt acctctgcat gaagtttctg tgagctttct 5460
ttatcttgct taacttatcc tcctttcctt ccccaccaaa attgggattg ttctaaacaa 5520
tttcaattta attcattgtc aacaaaacga tagaatgttt tgcttggaag aaacctggtc 5580
cattcccctc attgtgaaga atcccgaatc ctgggcctgt ccagggtcaa atggagtttt 5640
tggtattaat acatctctgc agggtttctt tttggagcac tgtgctgtct ttcctagtca 5700
gtcagggact tttttaagct tcaaagaaca tggtctgatg caaaggtctt ctaccgactt 5760
catggtattt aggaaaaatt tttaagtgat aataccatgc cgagttacct ggtcactgta 5820
atactggttc tccattaaaa tgtagatgct ttgtctaatt ccacttcaag atgatttaat 5880
tgagcttcta taactatcaa ctatcaaatt ctagtttcaa gtatctcact gtacctatat 5940
catatctgtt ctcaactttg tttgattctc cactcatttt caatcagagt tgaagccttc 6000
tttgcctttc ttctttgtct tatttctgcc ttcatctccc acttctcacc caactctgcc 6060
actgttcagg agcagttgac taaactttta tccttccttt aacttaatct ttttatcctt 6120
ctgtttttag ttcttacagt ggtaatcaca tcagtttggc aatctaagat gtctttatca 6180
agggtcagcc cttttctatt tggagctgaa tcttttcttt catgtagaaa tctacatgta 6240
tatacaagaa atgtattttt atctgcagga taatattgtt tcaaataatt ttattaagag 6300
ttaacttttt aggtattttc ccttttaatg tggttttgaa atgttaattg catttttaca 6360
catgatgcat tcagtggaca ctcttttttc ctaattataa ctcatctctg aatgcattgt 6420
gccaatcaag agtaagatag gattcagtgc gcaaaatttg cttttgatgc ttattaagag 6480
taaacccatg aatttgaatt tttatattat gcttggtact tggaattgat atatacctta 6540
ttaaagcttg aaaacattta gcatttgaat agtaagtttc atcacaaatc atgtcttttg 6600
cctcttttgt tacccaggtt cgatcaaggg gactggatgc ccaagtgatt ttctctaata 6660
atcgggttgg gacttaactt taaggtgttt tcaatgtgat tttagtattt acagtaataa 6720
ataaaattta tacaaagttt cagagaacta ccaactctct agttaatttt gatgaagatg 6780
ttttcattaa tagtctggtt tagtattaat ttaaccttga aataagaaat tggtcctgat 6840
ttgccacctt catttttcca tctgctgaaa tgccatttca gaatgtataa aagtactttc 6900
agaattagaa gaaaagtgag agcttcagca gaacgtgtca agtcgctaat aacaggagtc 6960
 
gaaccttatg tttgaaaatt aactgagtga gttaagtgca agcataaact aaaatttata 7020
tgactttgaa tttaagaacc actaataact gtgttctaat taacacagta atctgttcag 7080
ccaaaggata ctaacagcca ttctttggac tgtggttaat ttgataactt caagaagtta 7140
taactttgct accctggatt ctgatgcaat ttcaatataa aaagttgttc ctttcatagt 7200
ttttcccccc aaaagaagtg cctattacat tttaccttaa tgtttattta tcatccaagt 7260
gttcttgata taaaatcagt atacagaaaa ttttaagaga acttacattc aagggacata 7320
gagggaaaat gttaatgtgt atttcattgt ttcttgtgga attttttttt cattcggttt 7380
gagaatggac agtgtgtcta aggatgtgat ttaaatttac ttaggatgga tttagcattg 7440
aggttattaa gacaaagtta gcagaggcca gccagcactg tgctgcacaa ctccaggaac 7500
tccatggtat gctcacaatt ttctgcaagt tgcctccact tccaagtggc cagtgatttg 7560
ttaaaatacc ttgcccaaga atataactga ttgatgaaag ggcatattta ggtcaatata 7620
aaataatagc aagtatttac tgagagctta ctgtgtgcta ggcattttct taatatttca 7680
atgtgtattt aatcctcaca acaacccaat gtggtaggtt attgatatcc tcttcttaaa 7740
aatgagtaaa tggaggccca gagaaatgta atttgcctga gatcaattta aggttgctta 7800
agatcaaact gctaagaagg ggcagtttga gtctgagcta gtgctcctaa acactggaca 7860
tttttgctgt gaaccacagt cgggaaggtt taatgtttag actctttcct cagaggcttt 7920
taactacaca caattttgac atgttttcca agctcagaaa gctcattaaa aacaactaaa 7980
actg 7984
 <210> 10
   <211> 1788
   <212> DNA
   <213> Homo sapiens
<400> 10 
ggtctctgtg tgttctaatc cctgttcatt ctcatttact gtctaaagtt gaggagatgg 60
gatgtcccag atgatagggc tcctgggatt tcagacccaa gaccagcagg actccagtca 120
cctctacccc agctctccag gacacagcgc tcccaactct gagtgacgtc ccacctctgg 180
tccttgcagc acaaccaacg tgggaatcac accctccaga cctcccacag ctccacccca 240
gactgggcgc cggccctgcc tccatttcag ctgtgacaac ctcagagccg tgttggccca 300
agcatgacaa ggacgtatga aaacttccag tacttggaga ataaggtgaa agtccagggg 360
tttaaaaatg ggccacttcc tctccagtcc ctcctgcagc gtctctgctc tgggccctgc 420
catctcctgc tgtccctggg cctcggcctc ctgctgctgg tcatcatctg tgtggttgga 480
ttccaaaatt ccaaatttca gagggacctg gtgaccctga gaacagattt tagcaacttc 540
acctcaaaca ctgtggcgga gatccaggca ctgacttccc agggcagcag cttggaagaa 600
acgatagcat ctctgaaagc tgaggtggag ggtttcaagc aggaacggca ggcaggggta 660
tctgagctcc aggaacacac tacgcagaag gcacacctag gccactgtcc ccactgccca 720
tctgtgtgtg tcccagttca ttctgaaatg ctcctgcgag tccagcagct ggtgcaagac 780
ctgaagaaac tgacctgcca ggtggctact ctcaacaaca atgcctccac tgaagggacc 840
tgctgccctg tcaactgggt ggagcaccaa gacagctgct actggttctc tcactctggg 900
 
atgtcctggg ccgaggctga gaagtactgc cagctgaaga acgcccacct ggtggtcatc 960
aactccaggg aggagcagaa ttttgtccag aaatatctag gctccgcata cacctggatg 1020
ggcctcagtg accctgaagg agcctggaag tgggtggatg gaacagacta tgcgaccggc 1080
ttccagaact ggaagccagg ccagccagac gactggcagg ggcacgggct gggtggaggc 1140
gaggactgtg ctcacttcca tccagacggc aggtggaatg acgacgtctg ccagaggccc 1200
taccactggg tctgcgaggc tggcctgggt cagaccagcc aggagagtca ctgagctgcc 1260
tttggtggga ccacccggcc acagaaatgg cggtgggagg aggactcttc tcacgacctc 1320
ctcgcaagac cgctctggga gagaaataag cactgggaga ttggaagcac tgctaacatt 1380
ttgaattttt ttctctttaa ttttaaaaag atggtatagt gttcttaagc ttttattttt 1440
tttccaactt ttgaaagtca acttcatgaa ggtataattt ttacataata aaaatgcact 1500
catttaaaga gtagagatga ctttgacaaa tatgcatgcc taggtgacta ccactccgat 1560
cgcaatagat aacattgcca tcgcccccac cagtcccctc atgcctctgg gcagtccaac 1620
cacttccctg tttccaggcc agtgatctac ttctttttca ctatttattg gccttgcctc 1680
ttctagagct tctagaactt catataagtg aaatcataca ctctcgtgta tatacttcat 1740
ataagtgaat atatactctc gtgagaactt aaaaaaaaaa aaaaaaaa 1788
 <210> 11
   <211> 1436
   <212> DNA
   <213> Homo sapiens
<400> 11 
ggcgaggccg agcccctcct agtgcttccg gaccttgctc cctgaacact cggaggtggc 60
ggtggatctt actccttcca gccagtgagg atccagcaac ctgctccgtg cctcccgcgc 120
ctgttggttg gaagtgacga ccttgaagat cggccggttg gaagtgacga ccttgaagat 180
cggcgggcgc agcggggccg agggggcggg tctggcgcta ggtccagccc ctgcgtgccg 240
ggaaccccag aggaggtcgc agttcagccc agctgaggcc tgtctgcaga atcgacacca 300
accagcatca tgtccatgac actggggtac tgggacatcc gcgggctggc ccacgccatc 360
cgcctgctcc tggaatacac agactcaagc tacgaggaaa agaagtatac gatgggggac 420
gctcctgact atgacagaag ccagtggctg aatgaaaaat tcaagctggg cctggacttt 480
cccaatctgc cctacttgat tgatggggct cacaagatca cccagagcaa cgccatcctg 540
tgctacattg cccgcaagca caacctgtgt ggggagacag aagaggagaa gattcgtgtg 600
gacattttgg agaaccaggc tatggacgtc tccaatcagc tggccagagt ctgctacagc 660
cctgactttg agaaactgaa gccagaatac ttggaggaac ttcctacaat gatgcagcac 720
ttctcacagt tcctggggaa gaggccatgg tttgttggag acaagatcac ctttgtagat 780
ttcctcgcct atgatgtcct tgacctccac cgtatatttg agcccaactg cttggacgcc 840
ttcccaaatc tgaaggactt catctcccgc tttgagggct tggagaagat ctctgcctac 900
atgaagtcca gccgcttcct cccaaaacct ctgtacacaa gggtggctgt ctggggcaac 960
aagtaatgcc ttgaaggcca ggaggtggga gtgaggagcc catactcagc ctgctgccca 1020
ggctgtgcag cgcagctgga ctctgcatcc cagcacctgc ctcctcgttc ctttctcctg 1080
 
tttattccca tctttacccc caagacttta ttgggcctct tcacttcccc taaacccctg 1140
tcccatgcag gccctttgaa gcctcagcta cccactttcc ttcatgaaca tccccctccc 1200
aacactaccc ttccctgcac taaagccagc ctgaccttcc ttcctgttag tggttgtatc 1260
tgctttgaag ggcctacctg gcccctcgcc tgtggagctc agccctgagc tgtccccgtg 1320
ttgcatgaca gcattgactg gtttacaggc cctgctcctg cagcatggcc cctgccttag 1380
gcctacctga tcaaaataaa gcctcagcca caaaaaaaaa aaaaaaaaaa aaaaaa 1436
 <210> 12
   <211> 3657
   <212> DNA
   <213> Homo sapiens
<400> 12 
taggaaggta tggcctcaca agtcttggtc tacccaccat atgtttatca aactcagtca 60
agtgcctttt gtagtgtgaa gaaactcaaa gtagagccaa gcagttgtgt attccaggaa 120
agaaactatc cacggaccta tgtgaatggt agaaactttg gaaattctca tcctcccact 180
aagggtagtg cttttcagac aaagatacca tttaatagac ctcgaggaca caacttttca 240
ttgcagacaa gtgctgttgt tttgaaaaac actgcaggtg ctacaaaggt catagcagct 300
caggcacagc aagctcacgt gcaggcacct cagattgggg tgtggcgaaa cagattgcat 360
ttcctagaag gcccccagcg atgtggattg aagcgcaaga gtgaggagtt ggataatcat 420
agcagcgcaa tgcagattgt cgatgaattg tccatacttc ctgcaatgtt gcaaaccaac 480
atgggaaatc cagtgacagt tgtgacagct accacaggat caaaacagaa ttgtaccact 540
ggagaaggtg actatcagtt agtacagcat gaagtcttat gctccatgaa aaatacttac 600
gaagtccttg attttcttgg tcgaggcacg tttggccagg tagttaaatg ctggaaaaga 660
gggacaaatg aaattgtagc aatcaaaatt ttgaagaatc atccttctta tgcccgtcaa 720
ggtcaaatag aagtgagcat attagcaagg ctcagtactg aaaatgctga tgaatataac 780
tttgtacgag cttatgaatg ctttcagcac cgtaaccata cttgtttagt ctttgagatg 840
ctggaacaaa acttgtatga ctttctgaaa caaaataaat ttagtcccct gccactaaaa 900
gtgattcggc ccattcttca acaagtggcc actgcactga aaaaattgaa aagtcttggt 960
ttaattcatg ctgatctcaa gccagagaat attatgttgg tggatcctgt tcggcagcct 1020
tacagggtta aagtaataga ctttgggtcg gccagtcatg tatcaaagac tgtttgttca 1080
acatatctac aatctcggta ctacagagct ccagagatta tattggggtt gccattttgt 1140
gaagccatag acatgtggtc attgggatgt gtgattgcag aattatttct tggatggccg 1200
ctctacccag gagccttgga gtatgatcag attcgataca tttctcagac tcaaggtttg 1260
ccaggagaac agttgttaaa tgtgggtact aaatccacaa gatttttttg caaagaaaca 1320
gatatgtctc attctggttg gagattaaag acattggaag agcatgaggc agagacagga 1380
atgaagtcta aagaagccag aaaatacatt ttcaacagtc tggatgatgt agcgcatgtg 1440
aacacagtga tggatttgga aggaagtgat cttttggctg agaaagctga tagaagagaa 1500
tttgttagtc tgttgaagaa aatgttgctg attgatgcag atttaagaat tactccagct 1560
gagaccctga accatccttt tgttaatatg aaacatcttc tagatttccc tcatagcaac 1620
 
catgtaaagt cctgttttca tattatggat atttgtaagt cccacctaaa ttcatgtgac 1680
acaaataatc acaacaaaac ttcactttta agaccagttg cttcaagcag tactgctaca 1740
ctgactgcaa attttactaa aatcggaaca ttaagaagtc aggcattgac cacatctgct 1800
cattcagttg tgcaccatgg aatacctctg caggcaggaa ctgctcagtt tggttgtggt 1860
gatgcttttc agcagacatt gattatctgt cccccagcta ttcaaggtat tcctgcaaca 1920
catggtaaac ccaccagtta ttcaataagg gtagataata cagttccact tgtaactcag 1980
gccccagctg tgcagccact acagatccga ccaggagttc tttctcagac gtggtctggt 2040
agaacacagc agatgctggt gcctgcctgg caacaggtga cacccctggc tcctgctact 2100
actacactaa cttctgagag tgtggctggt tcacacaggc ttggagactg ggggaagatg 2160
atttcatgca gcaatcatta taactcagtg atgccgcagc ctcttctgac caatcagata 2220
actttatctg cccctcagcc agttagtgtg gggattgcac atgttgtctg gcctcagcct 2280
gccactacca agaaaaataa acagtgccag aacagaggta ttttggtaaa actaatggaa 2340
tgggagccag gaagagagga aataaatgct ttcagttgga gtaattcatt acagaatacc 2400
aatatcccac attcagcatt tatttctcca aagataatta atgggaaaga tgtcgaggaa 2460
gtaagttgta tagaaacaca ggacaatcag aactcagaag gagaggcaag aaattgctgt 2520
gaaacatcta tcagacagga ctctgattca tcagtttcag acaaacagcg gcaaaccatc 2580
attattgccg actccccgag tcctgcagtg agtgtcatca ctatcagcag tgacactgat 2640
gaggaagaga cttcccagag acattcactc agagaatgta aaggtagtct agattgtgaa 2700
gcttgccaga gcactttgaa tattgatcgg atgtgttcat taagtagtcc tgatagtact 2760
ctgagtacca gctcctcagg gcagtccagc ccatccccct gcaagagacc gaatagtatg 2820
tcagatgaag agcaagaaag tagttgtgat acggtggatg gctctccgac atctgactct 2880
tccgggcatg acagtccatt tgcagagagc acttttgtgg aggacactca tgaaaacaca 2940
gaattggtat cctctgctga cacagaaacc aagccagctg tctgttctgt tgtggtgcca 3000
ccagtggaac tagaaaatgg cttaaatgcc gatgagcata tggcaaacac agattctata 3060
tgccagccat taataaaagg acgatctgcc cctggaagat taaaccagcc ttctgcagtg 3120
ggtactcgtc agcaaaaatt gacatcagca ttccagcagc agcatttgaa cttcagtcag 3180
gttcagcact ttggatctgg gcatcaagag tggaatggaa actttgggca cagaagacag 3240
caagcttata ttcctactag tgttaccagt aatccattca ctctttctca tggaagtccc 3300
aatcacacag cagtgcatgc ccacctggct ggaaatacac acctcggagg acagcctact 3360
ctacttccat acccatcatc agccaccctc agtagtgctg caccagtggc ccacctgtta 3420
gcctctccgt gtacctcaag acctatgtta cagcatccaa cttataatat ctcccatccc 3480
agtggcatag ttcaccaagt cccagtgggc ttaaatcccc gtctgttacc atccccaacc 3540
attcatcaga ctcagtacaa accaatcttc ccaccacatt cttacattgc agcatcacct 3600
gcatatactg gatttccact gagtccaaca aaactcagcc agtatccata tatgtga 3657
 <210> 13
   <211> 1221
   <212> DNA
   <213> Homo sapiens
<400> 13 
gggctccagg ctgctctgtt gggtgctgct ttgtctcctg ggagcaggcc cagtaaaggc 60
tggagtcact caaactccaa gatatctgat caaaacgaga ggacagcaag tgacactgag 120
ctgctcccct atctctgggc ataggagtgt atcctggtac caacagaccc caggacaggg 180
ccttcagttc ctctttgaat acttcagtga gacacagaga aacaaaggaa acttccctgg 240
tcgattctca gggcgccagt tctctaactc tcgctctgag atgaatgtga gcaccttgga 300
gctgggggac tcggcccttt atctttgcgc cagcagccgg acagggggcg taaagaattc 360
acccctccac tttgggaacg ggaccaggct cactgtgaca gaggacctga acaaggtgtt 420
cccacccgag gtcgctgtgt ttgagccatc agaagcagag atctcccaca cccaaaaggc 480
cacactggtg tgcctggcca caggcttctt ccctgaccac gtggagctga gctggtgggt 540
gaatgggaag gaggtgcaca gtggggtcag cacggacccg cagcccctca aggagcagcc 600
cgccctcaat gactccagat actgcctgag cagccgcctg agggtctcgg ccaccttctg 660
gcagaacccc cgcaaccact tccgctgtca agtccagttc tacgggctct cggagaatga 720
cgagtggacc caggataggg ccaaacccgt cacccagatc gtcagcgccg aggcctgggg 780
tagagcagac tgtggcttta cctcggtgtc ctaccagcaa ggggtcctgt ctgccaccat 840
cctctatgag atcctgctag ggaaggccac cctgtatgct gtgctggtca gcgcccttgt 900
gttgatggcc atggtcaaga gaaaggattt ctgaaggcag ccctggaagt ggagttagga 960
gcttctaacc cgtcatggtt tcaatacaca ttcttctttt gccagcgctt ctgaagagct 1020
gctctcacct ctctgcatcc caatagatat ccccctatgt gcatgcacac ctgcacactc 1080
acggctgaaa tctccctaac ccagggggac cttagcatgc ctaagtgact aaaccaataa 1140
aaatgttctg gtctggcctg aaaaaaaaaa aaaaaaaaaa aaaaaaaaaa aaaaaaaaaa 1200
aaaaaaaaaa aaaaaaaaaa a 1221
 <210> 14
   <211> 2820
   <212> DNA
   <213> Homo sapiens
<400> 14 
cggtgacggc cctgctcggg aggccctcca gtctctgagc cagcggcttc gggtgcagga 60
gcaggagatg gaactggtaa aggcagccct ggcagaagcc cttcgcctgc tgcggctgca 120
ggtgccccct tcctccctgc agggctctgg cacaccagct cctccggggg acagcagtct 180
tgcagccccc ccaggactgc cacccacgtg caccccttcc ttggtgagcc gaggcaccca 240
gacggagaca gaggtggagc tcaagtcatc ccctggaccc cctggcctga gcaatggacc 300
cccagcccct caggggccca gcgaagagcc tagcgggacc caatctgaag gagggggcag 360
cagcagcagt ggtgctggct cccctggccc cccggggatc ctcaggccct tgcagccccc 420
acagcgtgct gacacgccgc gaagaaattc ttcctcctcc tcatccccct cagagcggcc 480
tcggcagaag ctctccagga aggcaatctc ctccgccaac ctgttagtgc ggtccgggag 540
cacagagagc cgtgggggaa aagaccccct ctccagccct gggggccctg gatctcggag 600
gagcaattac aatttggaag gcatctcagt gaagatgttc cttcgagggc gccccattac 660
 
catgtacatc ccgtctggca tccgcagcct tgaggagctg ccgagtggcc caccgccaga 720
gaccctcagc cttgactggg tttatgggta caggggtcgt gactcccgct ctaatctgtt 780
tgtgttgcgc tctggggagg tggtctactt tatcgcctgt gtggtggtgc tgtaccggcc 840
tggaggaggc ccagggggtc ctggaggtgg cggccagaga cattaccggg ggcacacaga 900
ctgcgttcga tgccttgctg ttcaccctga tggcgttcgg gtagcctcgg gacagacagc 960
tggagtggat aaggatggaa agcccctgca gcctgtggtt cacatctggg actcagagac 1020
gctgttgaaa ctgcaggaga ttggactggg ggccttcgag cggggtgttg gggccctggc 1080
cttttcagct gcggatcagg gtgcctttct ttgtgtggtg gataattcca atgagcacat 1140
gctgtcggtg tgggactgca gccggggaat gaagctggct gagatcaaga gtacaaatga 1200
ctcagtcctg gccgttggct tcaaccctcg tgacagcagc tgcatcgtca ccagtgggaa 1260
atctcacgtc cacttctgga attggagtgg tggagtaggg gttcctggga atgggaccct 1320
tacccggaaa cagggtgtct ttgggaaata caagaaaccc aagtttatcc cttgctttgt 1380
gttccttccg gatggagaca ttctcactgg agactcagag gggaacattc tcacctgggg 1440
gcggagccct tcagattcca agaccccagg caggggtggc gccaaagaga cctatgggat 1500
tgtggcccag gctcacgctc atgaaggttc tatcttcgcc ttgtgtctcc ggagggacgg 1560
gacagtgctg agtggtggcg ggcgggaccg ccggctggta cagtgggggc ccgggttggt 1620
ggccctccag gaggctgaga ttcccgagca cttcggggcc gtgcgagcca ttgctgaagg 1680
gcttggctct gagctgctgg tgggaaccac gaagaatgca ttgctgaggg gagacctggc 1740
ccagggcttc tcccctgtaa tccagggcca cactgatgag ctctgggggc tctgcacaca 1800
cccctcccag aaccgcttcc tcacctgcgg ccacgaccgg cagctctgcc tgtgggatgg 1860
ggagagccat gcactggcct ggagcatcga cctcaaggag actggtctct gtgctgactt 1920
ccacccgagt ggggcagttg tggccgtagg actgaacacg gggaggtggt tggttttgga 1980
cacagagacc agagagatcg tgtctgatgt cattgatggc aatgagcagc tctcagtggt 2040
ccggtacagc ccagatgggt tgtacctggc cattggttcc catgacaacg tgatctacat 2100
ctatagtgtt tccagtgatg gtgccaaatc cagccgcttt ggccgctgta tgggtcactc 2160
cagcttcatc actcatcttg actggtccaa ggatgggaat ttcatcatgt ccaattctgg 2220
ggactatgag attctttact gggacgtggc tggaggctgc aagcagctga agaatcgcta 2280
tgagagccga gaccgggaat gggctaccta cacctgtgtg ctgggctttc acgtctacgt 2340
acccgtgcgc tcgtgccaag gcgccgagcc gcatgtacgg gggccacggc agccacgtga 2400
ccagcgtccg attcacgcac gacgactcgc acctcgtctc gctgggcggc aaggacgcca 2460
gcatcttcca gtggcgagtg ctgggcgctg ggggcgcggg gccggcgccc gccacgccct 2520
ctcgaacccc ctccctgtcc cccgcctcct ccctcgacgt ttgatcgctg cctggcggga 2580
ccgactggcc cggcggcgtg gccccgcccc gccctgccct tccctggccc aatcccccac 2640
gactaggggc cgactctttc ctggactgac ttcgagacat tcccgatcgc gcattttcct 2700
ggagggcgcg aacggcgccc ctgcacacac tgtttagacc cgctggctga gccgggcagc 2760
cccagcctag gccttgactc ccgctgcctg ctgaggggca ataaaccaga accaaagtcg 2820
 <210> 15
   <211 >8685
   <212> DNA
   <213> Homo sapiens
<400> 15 
   cccccggcgg agccagctgc tgctcttcgg tgctggcccc ggtgccggcc ccgttgccca 60
   gggaacaggc tcccggcagc ccccgcggcc cggagtccat cccgcctcct ccggcccggc 120
   ggggccgacg agtccggagg ggctgccgcg ggagccccca ggtttcccta gatgacaaat 180
   aaacattcct tttcctgcgt gaagatagtc tgtggaaacc ttggccatgg catcgatatc 240
   agagcctgtt acattcagag agttctgccc gttgtactat ctcctcaatg ccattccgac 300
   aaagatccag aagggtttcc gctctatcgt ggtctatctc acggccctcg acaccaacgg 360
   ggactacatc gcggtgggca gcagcatcgg catgctctat ctgtactgcc ggcacctcaa 420
   ccagatgagg aagtacaact ttgaggggaa gacggaatct atcactgtgg tgaagctgct 480
   gagctgcttt gatgacctgg tggcagcagg cacagcctct ggcagggttg cagtttttca 540
   acttgtatct tcattgccag ggagaaataa acagcttcgg agatttgatg tcactggtat 600
   tcacaaaaat agcattacag ctctggcttg gagccccaat ggaatgaaat tgttctctgg 660
   agatgacaaa ggcaaaattg tttattcttc tctggatcta gaccaggggc tctgtaactc 720
   ccagctggtg ttggaggagc catcttccat tgtgcagctg gattatagcc agaaagtgct 780
   gctggtctct actctgcaaa gaagtctgct cttttacact gaagaaaagt ctgtaaggca 840
   aattggaaca caaccaagga aaagtactgg gaaatttggt gcttgtttta taccaggact 900
   ctgtaagcaa agtgatctaa ccttgtatgc gtcacggccc gggctccggc tatggaaggc 960
   tgatgtccac gggactgttc aagccacgtt tatcttaaaa gatgcttttg ccgggggagt 1020
   caagcctttt gaactgcacc cgcgtctgga atcccccaac agtggaagtt gcagcttacc 1080
   tgagaggcac ctggggcttg tttcatgttt ctttcaagaa ggctgggtgc tgagttggaa 1140
   tgaatatagt atctatctcc tagacacagt caaccaggcc acagttgctg gtttggaagg 1200
   atccggtgat attgtgtctg tttcgtgcac agaaaatgaa atatttttct tgaaaggaga 1260
   taggaacatt ataagaattt caagcaggcc tgaaggatta acatcaacag tgagagatgg 1320
   tctggagatg tctggatgct cagagcgtgt ccacgtgcag caagcggaga agctgccagg 1380
   ggccacagtt tctgagacga ggctcagagg ctcttccatg gccagctccg tggccagcga 1440
   gccaaggagc aggagcagct cgctcaactc caccgacagc ggctccgggc tcctgccccc 1500
   tgggctccag gccacccctg agctgggcaa gggcagccag cccctgtcac agagattcaa 1560
   cgccatcagc tcagaggact ttgaccagga gcttgtcgtg aagcctatca aagtgaaaag 1620
   gaagaagaag aagaagaaga cagaaggtgg aagcaggagc acctgtcaca gctccctgga 1680
   atcgacaccc tgctccgaat ttcctgggga cagtccccag tccttgaaca cagacttgct 1740
   gtcgatgacc tcaagtgtcc tgggcagtag cgtggatcag ttaagtgcag agtctccaga 1800
   ccaggaaagc agcttcaatg gtgaagtgaa cggtgtccca caggaaaata ctgaccccga 1860
   aacgtttaat gtcctggagg tgtcaggatc aatgcctgat tctctggctg aggaagatga 1920
   cattagaact gaaatgccac actgtcacca tgcacatggg cgggagctgc tcaatggagc 1980
   gagggaagat gtgggaggca gtgatgtcac gggactcgga gatgagccgt gtcctgcaga 2040
   tgatggacca aatagcacac agttaccctt ccaagaacag gacagctctc ctggggcgca 2100
   tgatggggaa gacatccaac ccattggccc ccaaagcact ttttgtgaag tccccctcct 2160
   gaactcactc actgtgcctt ccagcctcag ctgggcccca agtgctgaac agtggctgcc 2220
   tgggaccaga gctgatgaag gcagccccgt ggagcccagc caagagcagg acatcctaac 2280
   cagcatggag gcctctggcc acctcagcac aaatctctgg catgctgtca ctgatgatga 2340
   cacaggtcag aaagaaatac ccatttctga acgtgtcttg gggagtgtgg gaggacagct 2400
   gactccggtc tctgccttgg cagccagcac tcacaagccc tggcttgagc agcctccacg 2460
   ggatcagaca ttgacgtcca gcgatgagga ggacatctat gcccacgggc ttccttcttc 2520
   atcctcagag acgagtgtga cagagctcgg acctagttgc tcccagcagg acctgagccg 2580
   gctgggtgca gaggacgccg ggctgctcaa gccagatcag tttgcagaaa gctggatggg 2640
   ctactcgggt cccggctatg gcatcctcag cttggtggtc tccgagaagt atatctggtg 2700
   cctggactac aaaggcggcc tgttctgcag cgcgttgccg ggcgccgggc tgcgctggca 2760
   gaagtttgaa gatgctgtcc agcaggtggc agtctcgccc tcaggagccc ttctctggaa 2820
   gattgaacag aaatctaacc gggcttttgc ttgtgggaaa gtcaccatca aggggaagcg 2880
   gcactggtac gaagccctgc cccaggcagt gtttgtggcc ctgagcgatg acacggcctg 2940
   gatcatcagg accagtgggg acctatactt gcagacaggt ctgagcgtgg atcgcccttg 3000
   tgccagagcc gtaaaggtgg actgtcccta cccgctgtcc cagatcacag cccggaacaa 3060
   tgtggtgtgg gcgctgacag agcagagggc cctcctgtac cgggagggcg tgagcagctt 3120
   ctgtccggaa ggcgagcagt ggaagtgtga cattgtcagc gaaaggcaag ctttagaacc 3180
   cgtctgcata acgctcgggg atcagcagac tctctgggcc ctggacatcc atgggaacct 3240
   gtggttcaga actggcatta tttccaagaa gccccaagga gatgacgacc attggtggca 3300
   agtgagcatc acggactatg tggtgtttga ccagtgcagc ttatttcaga cgataatcca 3360
   tgccactcac tcggtggcca cagcagccca agcccccgta gaaaaggtgg cagataagct 3420
   gcgcatggcg ttttggtccc agcagcttca gtgccagcca agccttctcg gggtcaataa 3480
   cagcggtgtc tggatctcct cgggcaagaa tgaattccac gtcgctaagg gaagtctcat 3540
   aggcacctac tggaatcatg tggttccccg tgggacagct tctgctacaa aatgggcctt 3600
   tgtgttggct tctgcagctc ccacgaagga aggaagcttc ctgtggctgt gccagagcag 3660
   caaggacctg tgcagcgtca gcgcccagag cgcacagtcg cggccctcca cggtgcagct 3720
   gcctcccgaa gccgagatgc gcgcctatgc cgcctgccag gatgcgctgt gggcgctgga 3780
   cagcctcggc caggtgttca tcaggacgct ctccaagagc tgccccacgg gcatgcactg 3840
   gaccaggctg gacctctccc agctaggagc tgtaaaattg acaagcttgg catgtggaaa 3900
   tcagcacatc tgggcctgtg attccagggg tggagtttac ttccgtgtag ggactcagcc 3960
   tctcaatccc agtctcatgc ttccagcctg gataatgatt gagccacctg tccagcccgc 4020
   cggggtcagc ttggtcagcg tccattccag ccccaacgac cagatgctgt gggtgcttga 4080
   cagcaggtgg aacgtgcacg tgcggaccgg gatcaccgag gagatgcctg tggggaccgc 4140
   ctgggagcat gtgccagggt tgcaggcctg ccagctggcg ctgagcacca ggaccgtgtg 4200
   ggcccgctgt ccaaacggag acctcgcccg gcggtacggc gtcacagaca agaaccccgc 4260
   cggggactac tggaagaaaa ttcccggcag cgtgtcgtgt ttcacagtga ctgcgtcaga 4320
   tgagctgtgg gctgtgggcc cgcccggcta cctcctccaa cggctgacaa agacgttcag 4380
   ccactcgcac ggcacccaga agagcagcca ggccgccatg ccccaccctg aggacctgga 4440
   ggacgagtgg gaggtcatct gaaggagccc tggccgagtc acgcggaggg gcccggcgtc 4500
   tgtggcgggc acaggggctt cagagtgact ccctggtgga cgcgctgcct caacacttgt 4560
   ccagacacct ctggccaggt tggacccgca cacttacttt catctatgtt ggtttctgtc 4620
   tcgttccaga acccacagcc tccacccgtg gctggcgtga ttgctgcagc agtggcgcct 4680
   cctagctcag gacagtggcg actgcccggc tgcatgcact ccgattaccc acgtgctgcc 4740
   gtcctggtct catccacaga tagctccagc ttttgttggt gggagtggtc tccggaggcc 4800
   tcccagaacc aagggtagcc gggcagctgg tttggcccag ggcctccttc cacattagta 4860
   gccccagggc cagatggagc caaaggtcag ctctctgcag cgcgggatgt gctcggtgat 4920
   ggctttgtcc catcataggg gggtgtcccc ccagagacaa agctgcagag cacattccat 4980
   gccagacgct ctggccagga agctgaggcc gggcttgaga ggagagcgct ggccatgcca 5040
   ggagagaacc cacgcacatg cacaccacaa cacacaacac acctcacctc acaccacagc 5100
   acacctcacc acaccacacc gcactgcacc atacctcacc acatctcacc acaccacagc 5160
   acacctcacc acacaacaca ccacacccca caccgcactg caccgcaccg caccgcaccg 5220
   tacctcgcca catctcacca caccacacca caccacacct cactgcccac acacggcgca 5280
   ggctgcccgc ctcctggaga gcacacttca gctgaaacag taaagcctga tgggtgcaaa 5340
   tggaacctgg atgtgtgcac gtgtgtccca ggtagggacg gcacaggagg gtgcatgggg 5400
   cgtgggggag ctgagcaagg gtcgctcact tagaaatgtc tttggaatgg tgtttaacta 5460
   atgctgctgg cggacatcct aaaaccagat gcatcctcag aggacgagtc tactaattat 5520
   tgcctttgtt gttgtattac aaatctgcat aaaatacctc atttcaaatc aaatcttaca 5580
   aatttagaag agagatatgt tttccgaaaa cagtggaagc cctttgttcc ttcccgggtt 5640
   tgtcctgagc ctgcactgtc ctcgcctgca gcctcagagg ggcaggcatc cccgcacaga 5700
   cttgactggc agggcggtca cgggacctgc gggctggctc cgagtggcag cccatgcctt 5760
   ctgcggggta tgggttgaca cttgacaggt tgaaaccagt gcctctatgg acggctgctg 5820
   tggccccttc agacaatggg cagtgcccac cccgcccact ggcatctgcg tgtgagggct 5880
   aggccgccct gccacacatc ccgccccctc ccggaggcag cttcaggaca ggacaccagg 5940
   ctggctgctt tttttagcct gcccctggcc caggcccagt ccttggtgtc agggagcccc 6000
   caggccgcag gtggagggtg ataaaatatg ttctctgaca ggacccagcc agccacatag 6060
   gtggaggttt tccatgtcca aatgaggtca agatgccgaa atcccagatc tgacttcaca 6120
   cttccctttt ctagaacctt ttgtaaaagt tggtggcagc agaggcagcc ccaggccggg 6180
   ctgcatctct ctgtgtctgt tgtgccttgc ccggcgcctc acggatggca aagctctcct 6240
   cacccatggg actgtagtgc aattaaaccc gcgtctaggt gatgctttta aagttgtagc 6300
   ttcgtgcttt gtacagtttt ctttctggtt ttaattttta gttgtgcttt gagtcagtgc 6360
   aataaactag actttttcca aacctggccg agtgtggtgc ctgagctgtg agaagtgtcc 6420
   tcagccgaca ctcacgaggg cagtgcaagg gagaacctgc cagcccagcc ccaccacagg 6480
   gagagtgcgt cagcagacct gtcctggcct ggctggtgtt gaaatacaca ccggtttgcc 6540
   caagtggctg ttatggggga atggccgctc cagagctggc tgtaccccat ggtagcctct 6600
   gaggaggacg ctgtggggtg aggtccaggg ctgcctcctc atggagctgt gtgtgagcag 6660
   gcgttggagg ggttcgagcc cctggttctg tatcttcagc cagcaaacga aaccatatcg 6720
   caaaccagag ctgctggaaa ccagcacacg caaaagatga cgcccagcac agcagcagga 6780
   cacaccatgt gccaggaaat gacctcagca agaaacctca ggccgtgtga agagcagcaa 6840
   agctttccag aaggcataaa aggaaacttg aataaaggaa gatatgttcc acattcctgg 6900
   atggaggact acacacatta tggaaagacc tcattctttc caaaggaatt tataggtgta 6960
   gcttgatttc agttcaaatc cccactggga ttgttttgtt ggggcaggca gggaggaata 7020
   atgttagtac ccacaaatgc ccatgactgt gcccaatgtg cttctcacca gagccctgca 7080
   aggggaagtc tcattagccc cttgagactc agagaggtta ggtaacttgc tcaaggtcac 7140
   acagcactgg gactggaagc cagctctgta gaactgcaga cctgtgctgt ttgatgcctc 7200
   ctcactgtcc cgtcactcct gggaagaaga aacgggtgag aactgctaag tgatgacagg 7260
   tggagcccca gcaggggcca ctctattgaa tggcatggcc cagaccctcg gagggccagg 7320
   caacaggcta gcaaggccca cagggaggga gcagaatgga gagggcatct cagatgcgct 7380
   taaggagcgg cgggggcggg gcatgggggg tgtcacaact aatgtggcca ctgcttcatt 7440
   ctctgggcaa aataatatta aatggattaa agaatctaaa accagtggga aaactgtctg 7500
   atttctggat ggcaaaggct ttctaaactc aaaagtgata ggacaagcta cagaggaaaa 7560
   taagtacata gatttgattt ctccaaaata cagaaatgct gctgggcaca gtgatgtgca 7620
   gttatagtcc cagctacttg ggaggccaag gcaggaggat tacttgagcc caggagtttg 7680
   aggctgcaat gaactatgat cacaccactg cactccagcc tcggcgacag agcaagacct 7740
   tgtttctcaa aaataaataa atagaaggaa ataaatgtaa aaatgctgca aattaaaaac 7800
   ctcaaagaag ggaaataatt gcaacaaatg ggaattgatg ttgaatattt accttaaaaa 7860
   cctatacaac aataagagga gtgcgccctt cagcagcatg tatacaaaaa ttgcaacgat 7920
   acagagatta gcatggcccc tgcacaagga tgactcgcaa attcataaag ctttccataa 7980
   atatatttat taaaaaccaa taggaggagc acttcgagtc gagtgtaagg gcccttcaca 8040
   aaagcagaag gaacactggc ccaaaccccc agcaccccgg aagcagaggt gagatgggag 8100
   cagcttggga gccccccatt ggcgccgccc tactggggaa gccggtccgt acgtaggcct 8160
   tgcatctcgc cacctccact tctgtcctca agaacaagtc ctaggtcaag ggaaacagca 8220
   cagcctgttt ataattagga aacacttaaa acagcctggc atttgggaac agccatgtta 8280
   acgtgagcaa attcctctca tgaaatagcc tgcagctatt aagaagcacg tgtgtgcagg 8340
   aagcggaggc gcaggacctc accgcgccag cgtgaagttc ccgggcatgg tcatgaaagc 8400
   gtggttctgt aagaaatcag agggagaaag ggagagaagg gggagggaga caacccaaac 8460
   gttggagggt atttgttgta aacttcaaac ttttggcagg tttgaaattt ttcataactc 8520
   cggggaggag caagaggggt gaaaagaaac aagttctcta cttgtgatca gcagctggtc 8580
   atagtggttg cctggagtat atgccttttt gtatcctttg aatttccagc catgtaaatg 8640
   tattatttat tccaaaaata aagcagattt acattttaaa aattc 8685
 <210> 16
   <211> 1501
   <212> DNA
   <213> Homo sapiens
<400> 16 
agcgagtcct tcttttcctg actgcagctc ttttcatttt gccatccttt tccagctcca 60
tgatggttct gcaggtttct gcggcccccc ggacagtggc tctgacggcg ttactgatgg 120
tgctgctcac atctgtggtc cagggcaggg ccactccaga gaattacctt ttccagggac 180
ggcaggaatg ctacgcgttt aatgggacac agcgcttcct ggagagatac atctacaacc 240
gggaggagtt cgcgcgcttc gacagcgacg tgggggagtt ccgggcggtg acggagctgg 300
ggcggcctgc tgcggagtac tggaacagcc agaaggacat cctggaggag aagcgggcag 360
tgccggacag gatgtgcaga cacaactacg agctgggcgg gcccatgacc ctgcagcgcc 420
gagtccagcc tagggtgaat gtttccccct ccaagaaggg gcccttgcag caccacaacc 480
tgcttgtctg ccacgtgacg gatttctacc caggcagcat tcaagtccga tggttcctga 540
atggacagga ggaaacagct ggggtcgtgt ccaccaacct gatccgtaat ggagactgga 600
ccttccagat cctggtgatg ctggaaatga ccccccagca gggagatgtc tacacctgcc 660
aagtggagca caccagcctg gatagtcctg tcaccgtgga gtggaaggca cagtctgatt 720
ctgcccggag taagacattg acgggagctg ggggcttcgt gctggggctc atcatctgtg 780
gagtgggcat cttcatgcac aggaggagca agaaagttca acgaggatct gcataaacag 840
ggttcctgag ctcactgaaa agactattgt gccttaggaa aagcatttgc tgtgtttcgt 900
tagcatctgg ctccaggaca gaccttcaac ttccaaattg gatactgctg ccaagaagtt 960
gctctgaagt cagtttctat cattctgctc tttgattcaa agcactgttt ctctcactgg 1020
gcctccaacc atgttccctt cttcttagca ccacaaataa tcaaaaccca acatgactgt 1080
ttgttttcct ttaaaaatat gcaccaaatc atctctcatc acttttctct gagggtttta 1140
gtagacagta ggagttaata aagaagttca ttttggttta aacataggaa agaagagaac 1200
catgaaaatg gggatatgtt aactattgta taatggggcc tgttacacat gacactcttc 1260
tgaattgact gtatttcagt gagctgcccc caaatcaagt ttagtgccct catccattta 1320
tgtctcagac cactattctt aactattcaa tggtgagcag actgcaaatc tgcctgatag 1380
gacccatatt cccacagcac taattcaaca tataccttac tgagagcatg ttttatcatt 1440
accattaaga agttaaatga acatcagaat ttaaaatcat aaatataatc taatacactt 1500
t 1501
 <210> 17
   <211> 3773
   <212> DNA
   <213> Homo sapiens
   <400> 17 ccgtcccggg gcggacgggc gcgggcggga ggatggagct gaactccctg ctgatcctgc 60 
tggaggcggc cgagtacctg gagcgcaggg atcgagaggc cgagcacggc tacgcctcgg 120
tgctgccctt cgacggcgac ttcgccaggg agaaaacaaa ggcggccggc ctggtgcgca 180
aggccccgaa caacaggtct tcacacaacg agctagaaaa gcacagacga gccaaactca 240
ggctgtacct tgagcagctc aagcaactgg tgcccctggg ccccgacagc acccgccaca 300
ccacgctgag cctcctgaag cgggccaagg tgcacatcaa gaaactggag gagcaggacc 360
gccgggcact gagcatcaag gagcagctgc agcaggagca tcgtttcctg aagcggcgcc 420
tggagcagct gtcggtgcag agcgtggagc gcgtgcgcac agatagcacg ggctctgctg 480
tctccacgga cgactcagag caagaagtgg acatagaggg catggagttt ggccctggtg 540
agctggacag tgttggcagc agcagtgacg cggacgacca ctacagcctg cagagtggca 600
ccggcggcga cagtggcttc gggccccact gccggcggct gggccgcccc gccctctcgt 660
aggcccgtgc cctctgctcc ttggcctgcc tgcccgccag ccacgcgtgt cagccctcca 720
gttctccttc agttgacgcc agcctctcca caggcccact gctgtgccat tctggaagct 780
ccagctgctg ctgggctgcc tggcactgcc cgcttgccgg tcagggcctg ccgagctgcc 840
tgccccttcc agctgggcag agtcccctgc aaggaggcag ggcccagctt ccacatccgg 900
agccctggtc agcatagccg cccacggtct gttctcagat tcctaatcat tccagaagta 960
ttaaacgtca ttgctgcaaa cctcggcagg tgccgtgtga ggggcttaat gaccaccaca 1020
gggagctcag accccaaccc tggatcccag gagaaaggag tggaccgagg aaggaaggaa 1080
ggcaaggctg tctgtccatc cgtccgtctg tccacctacc tgtcagtcca cataggctcc 1140
tggcgtggac aaggggtctg tgaagggcgg gaactgggtg agcacctggg gcaggtgggt 1200
ggttaaggtc cttcccactt cgcaggtgtc agaacctagg gctgggctct cggggccagg 1260
caggccagcc cagcccacgc cgagctgggc agcgtctgct ctggtaggac tgtcagacgc 1320
acacgcgcac gcacctagac acacccactc atgtacatgc tcacacatgc agacacacct 1380
gggcgtcccg aggtcacatg ttctggggat gatggccttc aggggtcatc tggcaaacag 1440
cccctgggct gtgcctggat ccccctctag ctcctgctca cccacgccca cccagtagtc 1500
ctgcctgtct gcacaggaga ggggcttctc ttcctggctg gggctggggt gaactggagg 1560
ctggttaagt tgcagccgct gggtcctcgg gggcttactc atctcccttt tttaaacaaa 1620
aagcaaaaaa gtaaaatgct gcactgccca gcagcccggt tagggctcct ggagccacct 1680
taggaaaggg cttctcatga gctctgctgc ggcagcttca gctggcagag aggctttccc 1740
agaaaaaaaa aaaaaaccat ttttaaaaag aagaaagcct aaagactctc ggcctaggga 1800
cgtccgtgtg tgccgcctct gtttcctgta ccagattttt gtattctatt ttcctagctg 1860
ttgttgcgtc cttgtttgct gaggggtggg agccacccag cgtctcaggg acctgtccct 1920
ccgtcacgtc gtcaaagtgt gccttgtgtc ttgtgtcagg ccttgccctt cccaccagca 1980
tgtccctcgt ggctcagggt gccccaggcc tgcccagcta gtgctgtcct cccatctcct 2040
gtgggcagcc cctcccggca gccagggctt cctggaggcg atgcagccag gcccctgtgg 2100
gtggcacgga ggggctgtga cctggtcccc agtgttgccc tccccagtgg ctggcagggg 2160
cctgcttgct cactagagag atggattctc acctgtcacc tgactcgagc cccctgcttc 2220
ctggcctagg cgagggttcc aggtttcaga cactggcagc caatgaagac tgtgctcgct 2280
 
gggtggtgca ggcctggcac caggaggctt gcacccgcct ttccttcctg acgtcctctg 2340
tccttggggc tggcccatag cagtgcctgc cgtgcctctg gtacatctgt agccaattcc 2400
catatcatgg ggaaaattcg tgtctatttt cagtcgtacg catagacgcc ccaggatggg 2460
gggcccactg tggcggaagg gggtccctgg aaacaactct ggcacagaac ctgccctgca 2520
ggctgtaggg ggcatggtgc ctggagctga ggggcatccg aacgcgttgc gggtggttgt 2580
gaggaggcct gtctgcatct ccttccggcc ccactggggt ccaggggtgc ccagaaagga 2640
gcttcccctg cctgcctgag tctgtccccc caggcttcat ttcaaacacc gtggcacctc 2700
cgagcaaggc gggccgtgtg taaaagcttg cttccccagc cagcactgca gggccctgag 2760
gtggtcgtgt ccctgccctc aattcttgaa gcaccagctc cctgccccac cctccagtgc 2820
ctgaggcagc taggggcttc tgctctcatc tctgaccagc agaatccacc cggtgaccag 2880
tggtggcccc tcagcccacc ctcccggcag ctcagcctgt ggctcttgag gccgtggttc 2940
ccacgtggac tgggaggcag tctcagccac ccggggtgct gttcagctgc ccctccctgc 3000
catcagcagg tgggtgaggg gttgcccact gggtgggggc ccgtgctagg agtcaccaca 3060
tgctccaacc tcccactgct ccctgtcagg ggcccaggct gccatcactg gaggctgcag 3120
ggaccaagag gccatcaccg tgtctataga gagcagacag aagcagaaca gagcccgggg 3180
ctcctgagcc tctgcgtgtg ccctcccagc ccacaccagt gctctcggcc actgagcacc 3240
cagactcagg cttgggttcc ccagccttat tggaaggcag ctcccgcata ccaggataac 3300
ccccgcaaac cacatagcag acccccgcca tcctcgcaga gtgggagagg ctgcagcaag 3360
gctttgcctc tgcagacccc atcttagtgg cacggtgctt gggcctgtgt ccccgggtgg 3420
tggaaccctg taccggtctg tggccctagg gtccctgctc tgtctgcccc ggcccgtgct 3480
gtccgctggg tgaggcaggc tcccccgtgc cctgcctccc tctgtcaggg aacctgggac 3540
cccctcccca ctgcctgcac agaggaccct gaccctcggc cagcagggtg gccccaggtc 3600
catgttgggc actagggcag gttccgtgcc agagtcgggg gccacacgag ggcctggtgc 3660
cggtgagggg ggcgtgcgct agagggggaa aggggccccc ggccacctgt ccaccgtgtg 3720
ggccgtgctg tgtccttatg tcattgtaat ataaatacag atttttatat ctc 3773
 <210> 18
   <211> 367
   <212> DNA
   <213> Homo sapiens
<400> 18 
   ctccagtctc acctcggctt gcaatggacc ccaactgctc ctgcgaggct ggtggctcct 60
   gcgcctgcgc cggctcctgc aagtgcaaaa agtgcaaatg cacctcctgc aagaagagct 120
   gctgctcctg ttgccccctg ggctgtgcca agtgtgccca gggctgcatc tgcaaagggg 180
   cgtcagagaa gtgcagctgc tgtgcctgat gtcgggacag ccctgctgtc agatgaaaac 240
   agaatgacac gtaaaatccg aggttttttt tttctacaac tccgactcat ttgctacatt 300
   cctttttttc tgtgaaatat gtgaataata attaaacact tagacttgaa aaaaaaaaaa 360
   aaaaaaa 367
 <210> 19
   <211> 2183
   <212> DNA
   <213> Homo sapiens
<400> 19 
atggcgacct ccacgggtcg ctggcttctc ctccggcttg cactattcgg cttcctctgg 60
gaagcgtccg gcggcctcga ctcgggggcc tcccgcgacg acgacttgct actgccctat 120
ccacgcgcgc gcgcgcgcct cccccgggac tgcacacggg tgcgcgccgg caaccgcgag 180
cacgagagtt ggcctccgcc tcccgcgact cccggcgccg gcggtctggc cgtgcgcacc 240
ttcgtgtcgc acttcaggga ccgcgcggtg gccggccacc tgacgcgggc cgttgagccc 300
ctgcgcacct tctcggtgct ggagcccggt ggacccggcg gctgcgcggc gagacgacgc 360
gccaccgtgg aggagacggc gcgggcggcc gactgccgtg tcgcccagaa cggcggcttc 420
ttccgcatga actcgggcga gtgcctgggg aacgtggtga gcgacgagcg gcgggtgagc 480
agctccgggg ggctgcagaa cgcgcagttc gggatccgcc gcgacgggac cctggtcacc 540
gggtacctgt ctgaggagga ggtgctggac actgagaacc catttgtgca gctgctgagt 600
ggggtcgtgt ggctgattcg taatggaagc atctacatca acgagagcca agccacagag 660
tgtgacgaga cacaggagac aggttccttt agcaaatttg tgaatgtgat atcagccagg 720
acggccattg gccacgaccg gaaagggcag ctggtgctct ttcatgcaga cggccatacg 780
gagcagcgtg gcatcaacct gtgggaaatg gcggagttcc tgctgaaaca ggacgtggtc 840
aacgccatca acctggatgg gggtggctct gccacctttg tgctcaacgg gaccttggcc 900
agttacccgt cagatcactg ccaggacaac atgtggcgct gtccccgcca agtgtccacc 960
gtggtgtgtg tgcacgaacc ccgctgccag ccgcctgact gccacggcca cgggacctgc 1020
gtggacgggc actgccaatg caccgggcac ttctggcggg gtcccggctg tgatgagctg 1080
gactgtggcc cctctaactg cagccagcac ggactgtgca cggagaccgg ctgccgctgt 1140
gatgccggat ggaccgggtc caactgcagt gaagagtgtc cccttggctg gcatgggccg 1200
ggctgccaga ggcgttgtaa gtgtgagcac cattgtccct gtgaccccaa gactggcaac 1260
tgcagcgtct ccagagtaaa gcagtgtctc cagccacctg aagccaccct gagggcggga 1320
gaactctcct ttttcaccag gaccgcctgg ctagccctca ccctggcgct ggccttcctc 1380
ctgctgatca gcattgcagc aaacctgtcc ttgctcctgt ccagagcaga gaggaaccgg 1440
cgcctgcatg gggactatgc ataccacccg ctgcaggaga tgaacgggga gcctctggcc 1500
gcagagaagg agcagccagg gggcgcccac aaccccttca aggactgaag cctcaagctg 1560
cccggggtgg cacgtcgcga aagcttgttt ccccacggtc tggcttctgc aggggaaatt 1620
tcaaggccac tggcgtggac catctgggtg tcctcaatgg cccctgtggg gcagccaagt 1680
tcctgatagc acttgtgcct cagcccctca cctggccacc tgccagggca cctgcaaccc 1740
tagcaatacc atgctcgctg gagaggctca gctgcctgct tctcgcctgc ctgtgtctgc 1800
tgccgagaag cccgtgcccc cgggagggct gccgcactgc caaagagtct ccctcctcct 1860
ggggaagggg ctgccaacga accagactca gtgaccacgt catgacagaa cagcacatcc 1920
tggccagcac ccctggctgg agtgggttaa agggacgagt ctgccttcct ggctgtgaca 1980
cgggacccct tttctacaga cctcatcact ggatttgcca actagaattc gatttcctgt 2040
 
cataggaagc tccttggaag aagggatggg gggatgaaat catgtttaca gacctgtttt 2100
gtcatcctgc tgccaagaag ttttttaatc acttgaataa attgatataa taaaaggagc 2160
caccaggtgg tgtgtggatt ctg 2183
 <210> 20
   <211> 1016
   <212> DNA
   <213> Homo sapiens
<400> 20 
atggatacca atgttccgac tggagacggg gagcccgcga gacccgggtc tccagggtct 60
gcccaaggaa gttgctcatg ggagcagacc cctagagcag gatttgaggc caggccaaag 120
agaaccccag agatgaaagg cctcctccca ctggcttggt tcctggcttg tagtgtgcct 180
gctgtgcaag gaggcttgct ggacctaaaa tcaatgatcg agaaggtgac agggaagaac 240
gccctgacaa actacggctt ctacggctgt tactgcggct ggggcggccg aggaaccccc 300
aaggatggca ccgattggtg ctgttgggcg catgaccact gctatgggcg gctggaggag 360
aagggctgca acattcgcac acagtcctac aaatacagat tcgcgtgggg cgtggtcacc 420
tgcgagcccg ggcccttctg ccatgtgaac ctctgtgcct gtgaccggaa gctcgtctac 480
tgcctcaaga gaaacctacg gagctacaac ccacagtacc aatactttcc caacatcctc 540
tgctcctagg cctccccagc gagctcctcc cagaccaaga cttttgttct gtttttctac 600
aacacagagt actgactctg cctggttcct gagagaggct cctaagtcac agacctcagt 660
ctttctcgaa gcttggcgga cccccagggc cacactgtac cctccagcga gtcccaggag 720
agtgactctg gtcataggac ttggtagggt cccagggtcc ctaggcctcc acttctgagg 780
gcagcccctc tggtgccaag agctctcctc caactcaggg ttggctgtgt ctctttttct 840
ctctgaagac agcgtcctgg ctccagttgg aacactttcc tgagatgcac ttacttctca 900
gcttctgcga tcagattatc atcaccacca ccctccagag aattttacgc aagaagagcc 960
aaattgactc tctaaatctg gtgtatgggt attaaataaa attcattctc aaggct 1016
 <210> 21
   <211> 3615
   <212> DNA
   <213> Homo sapiens
<400> 21 
agtggctttt tggaggtgtc tcggccatga cacacatttg acatgccctc cctcaaccta 60
cttatagact atttttcttg ctctgcagca tggaccaaag agaaattctg cagaagttcc 120
tggatgaggc ccaaagcaag aaaattacta aagaggagtt tgccaatgaa tttctgaagc 180
tgaaaaggca atctaccaag tacaaggcag acaaaaccta tcctacaact gtggctgaga 240
agcccaagaa tatcaagaaa aacagatata aggatatttt gccctatgat tatagccggg 300
tagaactatc cctgataacc tctgatgagg attccagcta catcaatgcc aacttcatta 360
agggagttta tggacccaag gcttatattg ccacccaggg tcctttatct acaaccctcc 420
tggacttctg gaggatgatt tgggaatata gtgtccttat cattgttatg gcatgcatgg 480
agtatgaaat gggaaagaaa aagtgtgagc gctactgggc tgagccagga gagatgcagc 540
 
tggaatttgg ccctttctct gtatcctgtg aagctgaaaa aaggaaatct gattatataa 600
tcaggactct aaaagttaag ttcaatagtg aaactcgaac tatctaccag tttcattaca 660
agaattggcc agaccatgat gtaccttcat ctatagaccc tattcttgag ctcatctggg 720
atgtacgttg ttaccaagag gatgacagtg ttcccatatg cattcactgc agtgctggct 780
gtggaaggac tggtgttatt tgtgctattg attatacatg gatgttgcta aaagatggga 840
taattcctga gaacttcagt gttttcagtt tgatccggga aatgcggaca cagaggcctt 900
cattagttca aacgcaggaa caatatgaac tggtctacaa tgctgtatta gaactattta 960
agagacagat ggatgttatc agagataaac attctggaac agagagtcaa gcaaagcatt 1020
gtattcctga gaaaaatcac actctccaag cagactctta ttctcctaat ttaccaaaaa 1080
gtaccacaaa agcagcaaaa atgatgaacc aacaaaggac aaaaatggaa atcaaagaat 1140
cttcttcctt tgactttagg acttctgaaa taagtgcaaa agaagagcta gttttgcacc 1200
ctgctaaatc aagcacttct tttgactttc tggagctaaa ttacagtttt gacaaaaatg 1260
ctgacacaac catgaaatgg cagacaaagg catttccaat agttggggag cctcttcaga 1320
agcatcaaag tttggatttg ggctctcttt tgtttgaggg atgttctaat tctaaacctg 1380
taaatgcagc aggaagatat tttaattcaa aggtgccaat aacacggacc aaatcaactc 1440
cttttgaatt gatacagcag agagaaacca aggaggtgga cagcaaggaa aacttttctt 1500
atttggaatc tcaaccacat gattcttgtt ttgtagagat gcaggctcaa aaagtaatgc 1560
atgtttcttc agcagaactg aattattcac tgccatatga ctctaaacac caaatacgta 1620
atgcctctaa tgtaaagcac catgactcta gtgctcttgg tgtatattct tacatacctt 1680
tagtggaaaa tccttatttt tcatcatggc ctccaagtgg taccagttct aagatgtctc 1740
ttgatttacc tgagaagcaa gatggaactg tttttccttc ttctctgttg ccaacatcct 1800
ctacatccct cttctcttat tacaattcac atgattcttt atcactgaat tctccaacca 1860
atatttcctc actattgaac caggagtcag ctgtactagc aactgctcca aggatagatg 1920
atgaaatccc ccctccactt cctgtatgga cacctgaatc atttattgtg gttgaggaag 1980
ctggagaatt ctcaccaaat gttcccaaat ccttatcctc agctgtgaag gtaaaaattg 2040
gaacatcact ggaatggggt ggaacatctg aaccaaagaa atttgatgac tctgtgatac 2100
ttagaccaag caagagtgta aaactccgaa gtcctaaatc agaactacat caagatcgtt 2160
cttctccccc acctcctctc ccagaaagaa ctctagagtc cttctttctt gccgatgaag 2220
attgtatgca ggcccaatct atagaaacat attctactag ctatcctgac accatggaaa 2280
attcaacatc ttcaaaacag acactgaaga ctcctggaaa aagtttcaca aggagtaaga 2340
gtttgaaaat tttgcgaaac atgaaaaaga gtatctgtaa ttcttgccca ccaaacaagc 2400
ctgcagaatc tgttcagtca aataactcca gctcatttct gaattttggt tttgcaaacc 2460
gtttttcaaa acccaaagga ccaaggaatc caccaccaac ttggaatatt taataaaact 2520
ccagatttat aataatatgg gctgcaagta cacctgcaaa taaaactact agaatactgc 2580
tagttaaaat aagtgctcta tatgcataat atcaaatatg aagatatgct aatgtgttaa 2640
tagcttttaa aagaaaagca aaatgccaat aagtgccagt tttgcatttt catatcattt 2700
gcattgagtt gaaaactgca aataaaagtt tgtcacttga gcttatgtac agaatgctat 2760
 
atgagaaaca cttttagaat ggatttattt ttcatttttg ccagttattt ttattttctt 2820
ttacttttct acataaacat aaacttcaaa aggtttgtaa gatttggatc tcaactaatt 2880
tctacattgc cagaatatac tataaaaagt taaaaaaaaa acttactttg tgggttgcaa 2940
tacaaactgc tcttgacaat gactattccc tgacagttat ttttgcctaa atggagtata 3000
ccttgtaaat cttcccaaat gttgtggaaa actggaatat taagaaaatg agaaattata 3060
tttattagaa taaaatgtgc aaataatgac aattatttga atgtaacaag gaattcaact 3120
gaaatcctga taagttttaa ccaaagtcat taaattacca attctagaaa agtaatcaat 3180
gaaatataat agctatcttt tggtagcaaa agatataaat tgtatatgtt tatacaggat 3240
ctttcagatc atgtgcaatt tttatctaac caatcagaaa tactagttta aaatgaattt 3300
ctatatgaat atggatctgc cataagaaaa tctagttcaa ctctaatttt atgtagtaaa 3360
taaattggca ggtaattgtt tttacaaaga atccacctga cttcccctaa tgcattaaaa 3420
atatttttat ttaaataact ttatttataa cttttagaaa catgtagtat tgtttaaaca 3480
tcatttgttc ttcagtattt ttcatttgga agtccaatag ggcaaattga atgaagtatt 3540
attatctgtc tcttgtagta caatgtatcc aacagacact caataaactt tttggttgtt 3600
aaaaaaaaaa aaaaa 3615
 <210> 22
   <211> 4346
   <212> DNA
   <213> Homo sapiens
<400> 22 
cctccatcag ctcgccgcgc agcgcggctg tatttgcggc ctgtgcgagt aggcgcttgg 60
gcactcagtc tccctggcga gcgacgggca gaaatctcga accagtggag cgcactcgta 120
acctggatcc cagaaggtcg cgaaggcagt accgtttcct cagcggcgga cggagtctta 180
ctctgtcgtt caggttggag tgcagtggcg cgacctcggc tcactgcaac atctgcctcc 240
caggttcaag caattctcct gcctcacctt ccagagtagc tgggattaca gactgctgca 300
gtaagaatgt cttttccacc tcatttgaat cgccctccca tgggaatccc agcactccca 360
ccagggatcc cacccccgca gtttccagga tttcctccac ctgtacctcc agggacccca 420
atgattcctg taccaatgag cattatggct cctgctccaa ctgtcttagt acccactgtg 480
tctatggttg gaaagcattt gggcgcaaga aaggatcatc caggcttaaa ggctaaagaa 540
aatgatgaaa attgtggtcc tactaccact gtttttgttg gcaacatttc cgagaaagct 600
tcagacatgc ttataagaca actcttagct aaatgtggtt tggttttgag ctggaagaga 660
gtacaaggtg cttccggaaa gcttcaagcc ttcggattct gtgagtacaa ggagccagaa 720
tctaccctcc gtgcactcag attattacat gacctgcaaa ttggagagaa aaagctactc 780
gttaaagttg atgcaaagac aaaggcacag ctggatgaat ggaaagcaaa gaagaaagct 840
tctaatggga atgcaaggcc agaaactgtc actaatgacg atgaagaagc cttggatgaa 900
gaaacaaaga ggagagatca gatgattaaa ggggctattg aagttttaat tcgtgaatac 960
tccagtgagc taaatgcccc ctcacaggaa tctgattctc accccaggaa gaagaagaag 1020
gaaaagaagg aggacatttt ccgcagattt ccagtggccc cactgatccc ttatccactc 1080
 
atcactaagg aggatataaa tgctatagaa atggaagaag acaaaagaga cctgatatct 1140
cgagagatca gcaaattcag agacacacat aagaaactgg aagaagagaa aggcaaaaag 1200
gaaaaagaaa gacaggaaat tgagaaagaa cggagagaaa gagagaggga gcgtgaaagg 1260
gaacgagaaa ggcgagaacg ggaacgagaa agggaaagag aacgtgaacg agaaaaggag 1320
aaagaacggg agcgggaacg agaacgggat agggaccgtg accggacaaa agagagagac 1380
cgagatcggg atcgagagag agatcgtgac cgggatagag aaaggagctc agatcgtaat 1440
aaggatcgca gtcgatcaag agaaaaaagc agagatcgtg aaagggaacg agagcgggaa 1500
agagagagag agagagaacg agagcgagaa cgagaacggg agcgagagag agagcgagag 1560
agggaacggg agcgagaaag agaaaaagac aaaaaacggg accgagaaga agatgaagaa 1620
gatgcatacg aacgaagaaa acttgaaaga aaactccgag agaaagaagc tgcttatcaa 1680
gagcgcctta agaattggga aatcagagaa cgaaagaaaa cccgggaata tgagaaagaa 1740
gctgaaagag aagaagaaag aagaagagaa atggccaaag aagctaaacg actaaaagaa 1800
ttcttagaag actatgatga tgatagagat gaccccaaat attacagagg aagtgctctt 1860
cagaaaaggt tgcgtgatag agaaaaggaa atggaagcag atgaacgaga taggaagaga 1920
gagaaggagg agcttgagga aatcaggcag cgccttctgg cagaagggca tccagatcca 1980
gatgcagagc tccagaggat ggaacaagag gctgagaggc gcaggcagcc acaaataaag 2040
caagagccag aatcagaaga ggaggaagaa gaaaagcaag aaaaagaaga aaaacgagaa 2100
gaacccatgg aagaggaaga ggagccagag caaaagcctt gtctgaaacc tactctgagg 2160
cccatcagct ctgctccatc tgtttcctct gccagtggca atgcaacacc taacactcct 2220
ggggatgagt ctccctgtgg tattattatt cctcatgaaa actcaccaga tcaacagcaa 2280
cctgaggagc ataggccaaa aataggacta agtcttaaac tgggtgcttc caatagtcct 2340
ggtcagccta attctgtgaa gagaaagaaa ctacctgtag atagtgtctt taacaaattt 2400
gaggatgaag acagtgatga cgtaccccga aaaaggaaac tggttccctt ggattatggt 2460
gaagatgata aaaatgcaac caaaggcact gtaaacactg aagaaaagcg taaacacatt 2520
aagagtctca ttgagaaaat ccctacagcc aaacctgagc tcttcgctta tcccctggat 2580
tggtctattg tggattctat actgatggaa cgtcgaatta gaccatggat taataagaaa 2640
atcatagaat atataggtga agaagaagct acattagttg attttgtttg ttctaaggtt 2700
atggctcata gttcacccca gagcatttta gatgatgttg ccatggtact tgatgaagaa 2760
gcagaagttt ttatagtcaa aatgtggaga ttattgatat atgaaacaga agccaagaaa 2820
attggtcttg tgaagtaaaa ctttttatat ttagagttcc atttcagatt tcttctttgc 2880
caccctttta aggactttga atttttcttt gtctttgaag acattgtgag atctgtaatt 2940
tttttttttt gtagaaaatg tgaatttttt ggtcctctaa tttgttgttg ccctgtgtac 3000
tcccttggtt gtaaagtcat ctgaatcctt ggttctcttt atactcacca ggtacaaatt 3060
actggtatgt tttataagcc gcagctactg tacacagcct atctgatata atcttgttct 3120
gctgatttgt ttcttgtaaa tattaaaacg actccccaat tattttgcag aattgcactt 3180
aatattgaaa tgtactgtat aggaaccaac atgaacaatt ttaattgaaa acaccagtca 3240
 
taaactatta ccacccccac tctcttttga tcagaaatgg caagcccttg tgaaggcatg 3300
gagtttaaaa ttggaatgca aaaattagca gacaatccat tcctactgta tttctgtatg 3360
aatgtgtttg tgaatgtatg tgtaaaagtc tttcttttcc ctaatttgct ttggtggggt 3420
ccttaaaaca tttcccaact aaagaataga attgtaaagg aaaagtggta ctgttccaac 3480
ctgaaatgtc tgttataatt aggttattag tttcccagag catggtgttc tcgtgtcgtg 3540
agcaatgtgg tttgctaact ggatggggtt ttcttattaa taagatggct gcttcagctt 3600
ctcttttaaa ggaatgtgga tcatagtgat ttttcctttt aattttattg ctcagaaatg 3660
aggcatatcc taaaaatcct ggagagctgt atttaatgca tttttgcact aattggtcct 3720
tagtttaatt ctattgtatc tgtttattta acaaaaaatt catcatacca aaaagtgtaa 3780
gtgaaaaccc cctttaaaac aaaacaaaaa aatgaaataa aattaggcaa attgacagac 3840
agtgagagtt ttacaaacat gataggtatt ctgctcggca atttgtaagt ttacatgtta 3900
tttaaggata aaggtaaatc attcaaggca gttaccaacc actaactatt tgttttcatt 3960
tttgtcttgt agaaggttta tatcttgttt taccttggct cattagtgtt taaaaatgta 4020
ctgatgatgt gcttagagaa attcctgggg ctttcttcgt tgtagatcag aatttcacca 4080
gggagtaaaa ttacctgaaa acgtaagaag ttttaaacag cttttcacac aaattagatg 4140
caactgttcc catgtctgag tacttattta aaagaaaggt aaagattggc ctgttagaaa 4200
aagcataatg tgagctttgg attactggat tttttttttt tttaaacaca cctggagagg 4260
acatttgaaa acactgttct taccctcgaa ccctgatgtg gttccattat gtaaatattt 4320
caaatattaa aaatgtatat atttga 4346
 <210> 23
   <211> 1900
   <212> DNA
   <213> Homo sapiens
<400> 23 
acaactctca gaggagcatt gcccgtcaga cagcaactca gagaataacc agagaacaac 60
cagattgaaa caatggagga tctttgtgtg gcaaacacac tctttgccct caatttattc 120
aagcatctgg caaaagcaag ccccacccag aacctcttcc tctccccatg gagcatctcg 180
tccaccatgg ccatggtcta catgggctcc aggggcagca ccgaagacca gatggccaag 240
gtgcttcagt ttaatgaagt gggagccaat gcagttaccc ccatgactcc agagaacttt 300
accagctgtg ggttcatgca gcagatccag aagggtagtt atcctgatgc gattttgcag 360
gcacaagctg cagataaaat ccattcatcc ttccgctctc tcagctctgc aatcaatgca 420
tccacaggga attatttact ggaaagtgtc aataagctgt ttggtgagaa gtctgcgagc 480
ttccgggaag aatatattcg actctgtcag aaatattact cctcagaacc ccaggcagta 540
gacttcctag aatgtgcaga agaagctaga aaaaagatta attcctgggt caagactcaa 600
accaaaggca aaatcccaaa cttgttacct gaaggttctg tagatgggga taccaggatg 660
gtcctggtga atgctgtcta cttcaaagga aagtggaaaa ctccatttga gaagaaacta 720
aatgggcttt atcctttccg tgtaaactcg gctcagcgca cacctgtaca gatgatgtac 780
ttgcgtgaaa agctaaacat tggatacata gaagacctaa aggctcagat tctagaactc 840
 
ccatatgctg gagatgttag catgttcttg ttgcttccag atgaaattgc cgatgtgtcc 900
actggcttgg agctgctgga aagtgaaata acctatgaca aactcaacaa gtggaccagc 960
aaagacaaaa tggctgaaga tgaagttgag gtatacatac cccagttcaa attagaagag 1020
cattatgaac tcagatccat tctgagaagc atgggcatgg aggacgcctt caacaaggga 1080
cgggccaatt tctcagggat gtcggagagg aatgacctgt ttctttctga agtgttccac 1140
caagccatgg tggatgtgaa tgaggagggc actgaagcag ccgctggcac aggaggtgtt 1200
atgacaggga gaactggaca tggaggccca cagtttgtgg cagatcatcc ttttcttttt 1260
cttattatgc ataagataac caactgcatt ttatttttcg gcagattttc ctcaccctaa 1320
aactaagcgt gctgcttctg caaaagattt ttgtagatga gctgtgtgcc tcagaattgc 1380
tatttcaaat tgccaaaaat ttagagatgt tttctacata tttctgctct tctgaacaac 1440
ttctgctacc cactaaataa aaacacagaa ataattagac aattgtctat tataacatga 1500
caaccctatt aatcatttgg tcttctaaaa tgggatcatg cccatttaga ttttccttac 1560
tatcagttta tttttataac attaactttt actttgttat ttattatttt atataatggt 1620
gagtttttaa attattgctc actgcctatt taatgtagct aataaagtta tagaagcaga 1680
tgatctgtta atttcctatc taataaatgc ctttaattgt tctcataatg aagaataagt 1740
aggtaccctc catgcccttc tgtaataaat atctggaaaa aacattaaac aataggcaaa 1800
tatatgttat gtgcatttct agaaatacat aacacatata tatgtctgta tcttatattc 1860
aattgcaagt atataataaa taaacctgct tccaaacaac 1900
 <210> 24
   <211> 1893
   <212> DNA
   <213> Homo sapiens
   <400> 24 
gccaacgata cgcctgctgc agcaggagga gttacgagcc gggccgcgcg ctgcctaaat 60
acctaaacca ggtttagcgc ctgctcatat aaagctctcc taactcgtct tccggtggga 120
atttcttcac gtgggccgga gtcggagact gagtttagct ttactgagga gctctaaatt 180
taggcgggta tgagtgattt cagtgaagaa ttaaaagggc ctgtgacaga tgatgaagaa 240
gtggaaacat ctgtgctcag tggtgcagga atgcattttc cttggcttca aacatacgta 300
gaaactgtgg ccattggagg gaaaaggagg aaggattttg ctcagacaac aagtgcttgt 360
ttaagtttta tccaagaagc tctgctgaag caccaatggc agcaagctgc agaatacatg 420
tacagttatt ttcagacctt ggaagattca gatagctaca aaaggcaggc tgcacctgag 480
attatttgga agctcggaag tgaaattcta ttttatcatc ccaaaagcaa catggagagt 540
ttcaatactt ttgctaaccg gatgaaaaat attggcgtca tgaattattt aaagatctcc 600
ttacaacatg cattatacct tctgcatcat ggaatgctta aagatgctaa gagaaatctg 660
agtgaggcag agacatggag acatggtgaa aatacgtctt cccgggaaat attaatcaac 720
cttattcagg cctataaagg gcttttacag tattatacct ggtctgaaaa gaagatggaa 780
ttgtcaaagc ttgataagga tgattatgct tacaatgcag tagcccagga tgtgttcaac 840
cacagctgga agacatctgc aaatatttct gcattgatta aaattcctgg agtttgggac 900
 
ccttttgtga agagttatgt agaaatgctg gaattctatg gggatcgaga tggagcccaa 960
gaggtactca ccaattatgc atatgatgaa aagtttccat caaatccaaa tgcccatatc 1020
tacttataca actttctaaa gagacagaag gcaccaagat caaaattgat aagtgtgctt 1080
aagattttgt atcagattgt accatctcat aaattgatgt tggaattcca tacattactt 1140
agaaaatcag aaaaagaaga acaccgtaaa ctggggttgg aggtattatt tggagtctta 1200
gattttgccg gatgcactaa gaatataact gcttggaaat acttggcaaa atatctgaaa 1260
aatatcttaa tgggaaacca ccttgcgtgg gttcaagaag agtggaactc caggaaaaac 1320
tggtggccag gctttcattt cagctacttt tgggcaaaaa gtgattggaa ggaagataca 1380
gctttggcct gtgagaaagc ttttgtggct ggtttactgt taggaaaagg ttgtagatat 1440
ttccggtata ttttaaagca agatcaccaa atcttaggga agaaaattaa gcggatgaag 1500
agatctgtga aaaaatacag tattgtaaat ccaagactct gatactgaat tttagttatt 1560
tcacagttgt agctacacag taagtagctt ggtagatagt tattgaatgt atttatgtag 1620
tgtattaaga agcttatatt actacaaaaa acttattttt atatattttt atatttttgt 1680
attatttata gctagagaaa caatattact gcctttgctc tttgtaacta tgtctgtttt 1740
cttttttgta atgttaaatg ttacatttgt taaggaataa ttcttcaaat gacaaactaa 1800
ttacagaata tagctctaca gcagttattg tttgcaaata ctttgcctct tgctattgtg 1860
taataaactg taacttgtaa aaaaaaaaaa aaa 1893
 <210> 25
   <211> 3874
   <212> DNA
   <213> Homo sapiens
<400> 25 
atggctcacc taaagcgact agtaaaatta cacattaaaa gacattacca taaaaagttc 60
tggaagcttg gtgcagtaat ttttttcttt ataatagttt tggttttaat gcaaagagaa 120
gtaagtgttc aatattccaa agaggaatca aggatggaaa ggaacatgaa aaacaaaaac 180
aagatgttgg atttaatgct agaagctgta aacaatatta aggatgccat gccaaaaatg 240
caaataggag cacctgtcag gcaaaacatt gatgctggtg agagaccttg tttgcaagga 300
tattatacag cagcagaatt gaagcctgtc cttgaccgtc cacctcagga ttcaaatgca 360
cctggtgctt ctggtaaagc attcaagaca accaatttaa gtgttgaaga gcaaaaggaa 420
aaggaacgtg gggaagctaa acactgcttt aatgctttcg caagtgacag gatttctttg 480
caccgagatc ttggaccaga cactcgacct cctgaatgta ttgaacaaaa atttaagcgc 540
tgccctcccc tgcccaccac cagtgtcata atagtttttc ataatgaagc gtggtccacg 600
ttgcttagaa ctgtccacag tgtgctctat tcttcacctg caatactgct gaaggaaatc 660
attttggtgg atgatgctag tgtagatgag tacttacatg ataaactaga tgaatatgta 720
aaacaatttt ctatagtaaa aatagtcaga caaagagaaa gaaaaggtct gatcactgct 780
cggttgctag gagcaacagt cgcaacagct gaaacgctca catttttaga tgctcactgt 840
gagtgtttct atggttggct agaacctctg ttggccagaa tagctgagaa ctacacggct 900
gtcgtaagtc cagatattgc atccatagat ctgaacacgt ttgaattcaa caaaccttct 960
 
cctlatggaa gtaaccataa ccgtggaaat tttgactgga gtctttcatt tggctgggag 1020
tcgcttcctg atcatgagaa gcaaagaagg aaagatgaaa cctacccaat taaaacaccc 1080
acttttgcag gaggactttt ttccatatca aaagaatatt ttgagtatat tggaagctat 1140
gatgaagaaa tggaaatctg gggaggtgaa aatatagaaa tgtctttcag agtatggcaa 1200
tgtggtgggc agttggagat tatgccttgc tctgttgttg gacatgtttt tcgcagcaaa 1260
agccctcata gctttccaaa aggcactcag gtgattgcta gaaaccaagt tcgccttgca 1320
gaagtctgga tggatgaata caaggaaata ttttatagga gaaatacaga tgcagcaaaa 1380
attgttaaac aaaaagcatt tggtgatctt tcaaaaagat ttgaaataaa acaccgtctt 1440
cggtgtaaaa attttacatg gtatctgaac aacatttatc cagaggtgta tgtgccagac 1500
cttaatcctg ttatatctgg atacattaaa agcgttggtc agcctctatg tctggatgtt 1560
ggagaaaaca atcaaggagg caaaccatta attatgtata catgtcatgg acttggggga 1620
aaccagtact ttgaatactc tgctcaacat gaaattcggc acaacatcca gaaggaatta 1680
tgtcttcatg ctgctcaagg tctcgttcag ctgaaggcat gtacctacaa aggtcacaag 1740
acagttgtca ctggagagca gatatgggag atccagaagg atcaacttct atacaatcca 1800
ttcttaaaaa tgtgcctttc agcaaatgga gagcatccaa gtttagtgtc atgcaaccca 1860
tcagatccac tccaaaaatg gatacttagc caaaatgatt aagtgttcct taaaattaag 1920
ttgaaaaagg aaatattctt tctcataaaa ctgtgactag gcatacactg tagtttttga 1980
aaattatgca aaagcagcta aatgtaactt attccaagtg catttttctt atttatatct 2040
ttatgtagca ctactacaga aattctgcaa gtttctgttt caaagcacaa taactagtaa 2100
taccaaagac tatttcaaaa tgtccagatg taggggaaga gatgtttaca gtatgatgaa 2160
aataattttc caagtaaagt gatgtttgtg tgttttgtac acttagggat atatatatat 2220
agctacattc acacactcac aatttaaaat atttccccta gttttttggg gggataggaa 2280
gaaagatttg ttactgtatt tttttaacta cataaaaata gatcaataaa tgtcagcatt 2340
ggcctctgtg tacaaaccaa gagcttttac agatccagaa tttattagtt taaaatgcag 2400
gtgaactttt ttttgcgttt ggtttacttg tctgtcaaat gtttccttaa acatgaaact 2460
gaataaggag aagagtattt ttaacactta aatttcttgg caaattttaa aacatttttt 2520
agtctgtaat acactccact tgaagcactt aagtcttcct taaatgactt ttcttaagta 2580
atgatactgt gtgttttccc aaagcacttt taaaaaaatt tttataaatt actatctgtt 2640
gaaaaggtgt ccttttcctt tcttctagta ttttttttct taccaaaatt cactaatctt 2700
gaatgtttgt gatattaaat ttcaaatgca gaatacttga ctcatttaaa gctaaatttt 2760
gttactgatt caattataat tgtaatggat ttttgacttt gtaatggatt cttttcatca 2820
aaaagcctta ttatttttta tctatgtgga aaacacaata aaaaatcctc aacactattg 2880
taatcatttg gttaagtgct tattcctctt ttgggtaaaa tctgtaattg ataataggtg 2940
ggggaaaatg aattttgtat gctgaatttc taagcgccta ttgtttgtaa aaccatcaga 3000
tatttcttat ggcacaaaaa atgaggaata gcaaaattcc tgtgttcaat atttagaaaa 3060
ttttgtatta atttctgata aagttcctta agcatctgat agaatgatgt tttaaaaaaa 3120
tttgacgctt gcttaggaga tttaccactt tttttttttg tttttcgtca ttttatattt 3180
 
agatctcctg tattcttgtt cccgaagtaa aatacgatcg gtttcatatt ttaaatctgg 3240
cagagcctca gctgtacgaa aaagagcata tactggttat tgaccctatc ttctcattgt 3300
ttgtttgtaa gtttgaattt gtattaaaaa gcctgcattc tgagctggac atggtggctc 3360
agcttctaat cccagcactt tggtaggcaa aggtgggagg atcatttgag ctcaggagtt 3420
ccagaccagc ctgggcaaca tagcaaaatc tcatctctac aaaaagtaaa aattaaaaaa 3480
tgaaattaaa aataaaatta cctaggtgtg gtggcacgca tctgtagttc cagctataca 3540
ggaaggtgag gcagaagcat tgcttgagct tgggagatcg aggctacagt gagctatgat 3600
tacaccactg cacttcagtc tgtgtgactg agcaagactc tttcaaaaaa aaaaaaaagc 3660
ctacattctc cagttgatta tttccaacta atgtgtatta tgtgcctaat tttctatcag 3720
aagttgtatt aagcccgttt tcacactgct gttaaagaca tacctgagac tgggtaattt 3780
ataaagaaaa ataggttcaa tggacccaca ggtccgcgtg gctggggaag cttcacaatc 3840
atggcggaag gtgaaagcat gtcttacgtg gaag 3874
 <210> 26
   <211> 21
   <212> DNA
   <213> Homo sapiens
<400> 26 ctttagccca tcagtggat c 21
<210> 27
   <211> 23
   <212> DNA
   <213> Homo sapiens
<400> 27 gagagatcac ccttcaagtc atc 23
<210> 28
   <211> 21
   <212> DNA
   <213> Homo sapiens
<400> 28 gtgcttatcc acactggtga g 21
<210> 29
   <211> 21
   <212> DNA
   <213> Homo sapiens
<400> 29 aggttacact tctcacaatg g 21
<210> 30
   <211> 21
   <212> DNA
   <213> Homo sapiens
<400> 30 atatgccctt tcaggatggc c 21
<210> 31
   <211> 21
   <212> DNA
   <213> Homo sapiens
<400> 31 cttcacagcc tcaggcttga t 21
<210> 32
   <211> 21
   <212> DNA
   <213> Homo sapiens
<400> 32 ttgtgattgg agtagtggcc c 21
<210> 33
   <211> 21
   <212> DNA
   <213> Homo sapiens
<400> 33 gtcattggag aggtcctgag t 21